# EUROPEAN PATENT SPECIFICATION

(11) **EP 2 029 541 B1**
(45) Date of publication and mention of the grant of the patent: **30.12.2009**
(21) Application number: 07727328.2
(22) Date of filing: 26.03.2007
(51) Int. Cl.: C07D 211/60, C07D 207/16

(54) **PROCESS FOR PREPARATION OF ENANTIOMERICALLY ENRICHED CYCLIC BETA-ARYL OR HETEROARYL CARBOXYLIC ACIDS**
VERFAHREN ZUR HERSTELLUNG VON ENANTIOMEREN ANGEREICHERTEN CYCLISCHEN BETA-ARYL- ODER HETEROARYLCARBONSÄUREN
PROCEDE DE PREPARATION D'ACIDES BETA-ARYLCARBOXYLIQUES OU HETEROARYLCARBOXYLIQUES CYCLIQUES ENRICHIS SUR LE PLAN ENANTIOMERIQUE

(30) Priority: 03.04.2006 EP 06112171
(43) Date of publication of application: 04.03.2009
(73) Proprietor: F. Hoffmann-La Roche AG, 4070 Basel (CH)
(72) Inventor: BACHMANN, Stephan, CH-4123 Allschwil (CH); SCALONE, Michelangelo, CH-4127 Birsfelden (CH); SCHNIDER, Patrick, CH-4103 Bottmingen (CH)
(74) Representative: Poppe, Regina
(86) International application number: PCT/EP2007/052855
(87) International publication number: WO 2007/113155

(56) References cited:
- PATANE M A ET AL: "Selective alpha-1A adrenergic receptor antagonists. Effects of pharmacophore regio- and stereochemistry on potency and selectivity" BIOORGANIC & MEDICINAL CHEMISTRY LETTERS, OXFORD, GB, vol. 8, no. 18, 22 September 1998 (1998-09-22), pages 2495-2500, XP004138258 ISSN: 0960-894X cited in the application
- J. BROWN ET AL.: "Enantioselective catalytic transfer hydrogenation of alpha,beta-unsaturated carboxylic acids with formates catalyzed by novel Ruthenium phospine complexes" TETRAHEDRON ASYMMETRY, vol. 2, no. 5, 1991, pages 331-334, XP002404759
- RATOVELOMANANA-VIDAL V ET AL: "Enantioselectivity hydrogenation of beta-keto esters using chiral diphosphine-Ruthenium complexes: optimisation for academic and industrial purposes and synthetic applications" ADVANCED SYNTHESIS AND CATALYSIS, WILEY-VCH, WEINHEIM, DE, vol. 345, no. 1-2, 21 January 2003 (2003-01-21), pages 261-274, XP002290486 ISSN: 1615-4150
- UJJAINWALLA F ET AL: "Design and Syntheses of Melanocortin Subtype-4 Receptor Agonists: Evolution of the Pyridazinone Archetype" BIOORGANIC & MEDICINAL CHEMISTRY LETTERS, OXFORD, GB, vol. 13, 2003, pages 4431-4435, XP002372910 ISSN: 0960-894X
- UJJAINWALLA ET AL: "Design and syntheses of melanocortin subtype-4 receptor agonists. Part 2: Discovery of the dihydropyridazinone motif" BIOORGANIC & MEDICINAL CHEMISTRY LETTERS, OXFORD, GB, vol. 15, no. 18, 15 September 2005 (2005-09-15), pages 4023-4028, XP005021091 ISSN: 0960-894X

## Description

The present invention relates to a process for the preparation of cis substituted cyclic β-aryl or heteroaryl carboxylic acid derivatives in high diastereo- and enantioselectivity by enantioselective hydrogenation in accordance with the following scheme 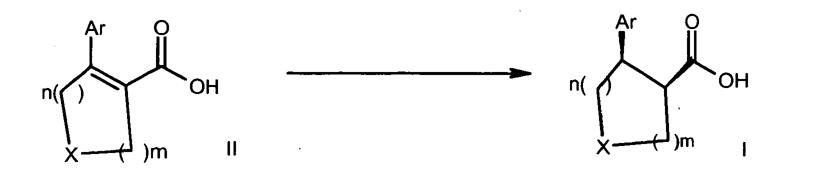 wherein
- X: is -C(R)(R')-, -N(R")-, -O-, -S(O)ₒ-, C(O)N(R"), -N(R")C(O)- or -C(O)-;
- R and R': are independently from each other hydrogen, C₁₋₇-alkyl, C₁₋₇-alkyl substituted by halogen, C₁₋₇-alkoxy, hydroxy or -(CH₂)ₚ-Ar;
- R": is hydrogen, C₁₋₇-alkyl, C₁₋₇-alkyl substituted by halogen, -S(O)ₒ-C₁₋₇-alkyl, -S(O)ₒ-Ar, -S(O)ₒ-NRR', -(CH₂)ₚ-Ar, -C(O)-C₁₋₇-alkyl, -C(O)-Ar, -C(O)-NRR' or -C(O)O-C₁₋₇-alkyl;
- Ar: is aryl¹ or heteroaryl¹;

- n: is 0, 1,2 or 3;
- m: is 0, 1, 2 or 3;
- o: is 0, 1 or 2;
- p: is 0, 1, or 2;
and corresponding salts thereof.

A further object of the present invention are new compounds of formula I, which have been prepared by the above-mentioned process.
(+)-(3*R*,4*R*)-4-(4-fluoro-phenyl)-piperidine-1,3-dicarboxylic acid-1-*tert*-butyl ester and
(-)-(3*S*,4*S*)-4-(4-fluoro-phenyl)-piperidine-1,3-dicarboxylic acid 1-tert-butyl ester,
(-)-4-(1*H*-indol-3-yl)-piperidine-1,3-dicarboxylic acid-1-*tert*-butyl ester and
(+)-4-(1*H*-indol-3-yl)-piperidine-1,3-dicarboxylic acid-1-*tert*-butyl ester,
(-)-4-o-tolyl-piperidine-1,3-dicarboxylic acid 1-*tert*-butyl ester and
(+)-4-o-tolyl-piperidine-1,3-dicarboxylic acid 1-*tert*-butyl ester,
(+)-4-(3-methoxy-phenyl)-piperidine-1,3-dicarboxylic acid-1-*tert*-butyl ester,
(+)-4-phenyl-piperidine-1,3-dicarboxylic acid-1-tert-butyl ester and
(-)-4-phenyl-piperidine-1,3-dicarboxylic acid-1-tert-butyl ester
(+)-3-phenyl-piperidine-1,4-dicarboxylic acid 1-*tert*-butyl ester and
(-)-3-phenyl-piperidine-1,4-dicarboxylic acid *1*-*tert*-butyl ester,
(-)-2-phenyl-cyclopentanecarboxylic acid and
(+)-2-phenyl-cyclopentanecarboxylic acid,
(+)-(3*R*,4*R*)-4-(phenyl)-pyrrolidine-1,3-di carboxylic acid-1-*tert*-butyl ester and
(-)-(3*S*,4*S*)-4-(phenyl)-pyrrolidine-1,3-di carboxylic acid-1-*tert*-butyl ester,
(-)-4-(4-chloro-phenyl)-pyrrolidine-1,3-di carboxylic acid-1-*tert*-butyl ester and
(+)-4-(4-chloro-phenyl)-pyrrolidine-1,3-di carboxylic acid-1-*tert*-butyl ester,
(+)-4-(3-fluoro-phenyl)-pyrrolidine-1,3-di carboxylic acid-1-*tert*-butyl ester and
(-)-4-(3-fluoro-phenyl)-pyrrolidine-1,3-di carboxylic acid-1-*tert*-butyl ester,
(3*R*,4*R*)-1-benzyl-4-phenyl-pyrrolidine-3-carboxylic acid and
(3*RS*,4*RS*)-1-benzyl-4-phenyl-pyrrolidine-3-carboxylic acid,
(-)-2-phenyl-cyclooctanecarboxylic acid,
(+)-4-phenyl-tetrahydro-thiophene-3-carboxylic acid and
(-)-4-phenyl-tetrahydro-thiophene-3-carboxylic acid

The compounds of formula I may be used as starting materials or intermediates for the preparation of pharmaceutically active compounds, especially for compounds, which may be used for the treatment of central nervous system disorders.

The term "aryl¹" refers to an aromatic monovalent mono- or polycarbocyclic radical, such as phenyl or naphthyl, preferably phenyl, which may optionally be substituted by one or more substituents, independently by C₁₋₇-alkyl, hydroxy, C₁₋₇-alkoxy, -O-C₁₋₇-alkyl substituted by halogen, -O-benzyl, -OC(O)-C₁₋₇-alkyl, -OC(O)-phenyl, halogen, C₁₋₇-alkyl substituted by halogen, cyano, amino, mono-or di-C₁₋₇-alkyl amino, -NHC(O)-C₁₋₇-alkyl, -NHC(O)-phenyl, -S(O)ₒ-amino, -S(O)ₒ-mono-or di-C₁₋₇-alkyl amino, -S(O)ₒ-C₁₋₇-alkyl, -S(O)ₒ-C₁₋₇-alkyl substituted by halogen, nitro, -C(O)OH,-C(O)-O-C₁₋₇-alkyl, -C(O)-O-C₁₋₇-alkyl substituted by halogen, -C(O)-O-phenyl, -C(O)-C₁₋₇-alkyl, -C(O)-C₁₋₇-alkyl substituted by halogen, -C(O)-amino, -C(O)-mono-or di-C₁₋₇-alkyl amino, -C(O)-NH-phenyl or the like.

The term "heteroaryl" denotes a monovalent heterocyclic 5 or 6-membered aromatic radical, wherein the heteroatoms are selected from N, O or S, for example the groups thiophenyl, indolyl, pyridinyl, pyrimidinyl, imidazolyl, piperidinyl, furanyl, pyrrolyl, isoxazolyl, pyrazolyl, pyrazinyl, benzo[1.3]dioxolyl, benzo{b}thiophenyl or benzotriazolyl, which may optionally be substituted by one or more substituents, independently by C₁₋₇-alkyl, hydroxy, C₁₋₇-alkoxy, -O-C₁₋₇-alkyl substituted by halogen, -O-benzyl, -OC(O)-C₁₋₇-alkyl, -OC(O)-phenyl, halogen, C₁₋₇-alkyl substituted by halogen, cyano, amino, mono-or di-C₁₋₇-alkyl amino, -NHC(O)-C₁₋₇-alkyl, -NHC(O)-phenyl, -S(O)ₒ-amino, -S(O)ₒ-mono-or di-C₁₋₇-alkyl amino, -S(O)ₒ-C₁₋₇-alkyl, -S(O)ₒ-C₁₋₇-alkyl substituted by halogen, nitro, -C(O)OH, -C(O)-O-C₁₋₇-alkyl, -C(O)-O-C₁₋₇-alkyl substituted by halogen, -C(O)-O-phenyl, -C(O)-C₁₋₇-alkyl, -C(O)-C₁₋₇-alkyl substituted by halogen, -C(O)-amino, -C(O)-mono-or di-C₁₋₇-alkyl amino, -C(O)-NH-phenyl or the like.

The synthesis of cis-substituted cyclic β-aryl or heteroaryl carboxylic acid derivates of general formula I is very poorly described in the literature. The reason seems to be the reluctance of the tetrasubstituted double bond of compounds of formula II to undergo the catalytic hydrogenation.

The process for homogeneous enantioselective hydrogenation as described in the present invention offers a viable method for the reduction of compounds of formula II to compounds of formula I. The reduction can be carried out much more economically, with less process steps under moderate conditions with high yields. Further, crude intermediate products can mostly be used in subsequent reaction steps without the need of any additional purification steps.

No example is reported in the literature for this type of conversion (J.M. Brown in E.N. Jacobsen, A. Pfaltz, H. Yamamoto, Comprehensive Asymmetric Catalysis, Vol I, p. 163 ff., Springer 1999). Homogeneous catalysts for this conversion should be active under relatively mild conditions, in order to allow the achievement of high diastereo-(i.e. cis/trans ratio) and enantio- (i.e. *R*,*R*/*S*,*S* ratio) selectivities. From highly enantiomerically enriched cis-configurated acids of formula I also the corresponding acids with trans-structure are easily accessible by epimerization of the center α to the carboxylic function.

The direct enantioselective reduction of cyclic β-aryl or heteroaryl substituted α, β-unsaturated carboxylic acids II to cis-substituted cyclic β-aryl or heteroaryl carboxylic acid derivates I has never been described in the literature before. Synthesis of an enantiomerically pure acid of type I has been described in Bioorg. Med. Chem. Lett. 1998, 8, 2495. The synthetic pathway described suffers from three major drawbacks as compared to the present invention:
1) The ester derivative of an acid of type II was hydrogenated to the RACEMIC ester of I, which was consecutively saponified under carefully controlled conditions to the RACEMIC acid I. After salt formation with a chiral amine the diastereomeric salts could be separated by crystallization. The pure enantiomer was generated by treatment with acid. In comparison with our direct enantioselective hydrogenation this procedure is tedious, requires at least three additional steps and is not atom-economical as at least 50 % of the material is lost during the separation of the enantiomers in the form of their diastereomeric salts.
2) Saponification of the RACEMIC cis-ester is problematic and may lead to partial epimerization to the trans-ester or acid, resulting in loss of material.
3) The method described in Bioorg. Med. Chem. Lett. 1998, 8, 2495 is not general: Under the hydrogenation conditions using Pd/C aromatic groups such as indole or functional groups on the aromatic ring such as, e.g., nitro, chlorine, bromine or iodine substituents, which are sensitive to reduction, are usually not tolerated. Chlorine, bromine or iodine are usually replaced by hydrogen under such conditions. The reaction conditions described in the present invention using a homogenous palladium complex are compatible with such reducible groups.

The following scheme describes the usual known procedure as described in Bioorg. Med. Chem. Lett. 1998, 8, 2495:

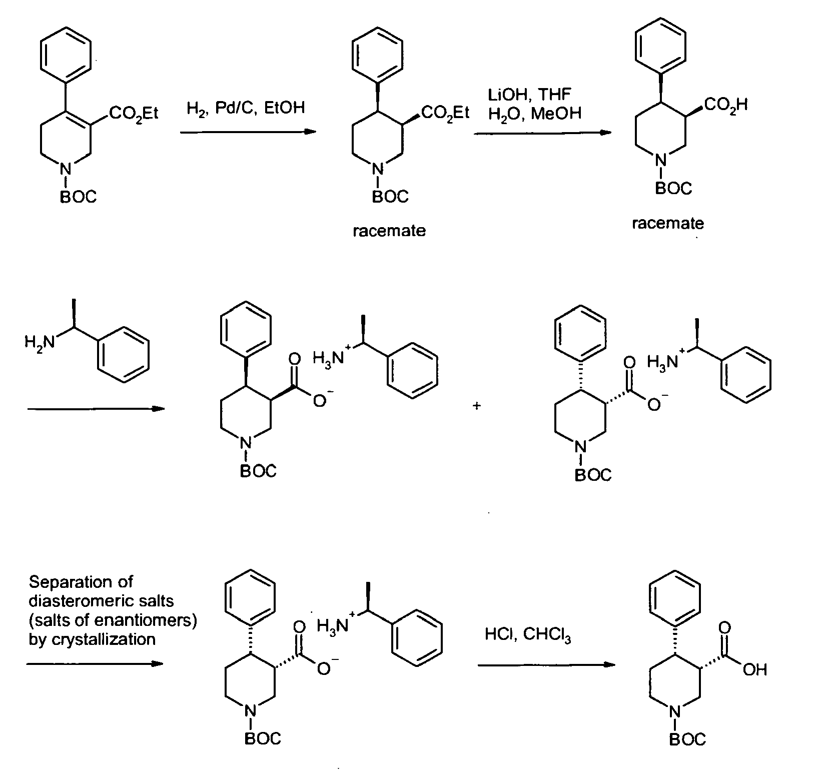

The enantioselective hydrogenation of cyclic β-aryl or heteroaryl substituted α, β-unsaturated carboxylic acids II is the only method to give direct access to enantiomerically enriched cis-substituted cyclic β-arylcarboxcylic acid derivates I.

Synthetic access to such compounds I is generally particularly difficult as the cis-substituted form is thermodynamically less stable than the trans form. Thus synthetic procedures under equilibrating conditions usually give rise to either cis/trans-mixtures or predominantly the trans form. In fact, selective epimerization of the chiral center α to the carboxyl group of enantiomerically enriched cis-substituted cyclic β-aryl or heteroaryl carboxcylic acid derivates I to the trans isomers IV is effectively done as follows: 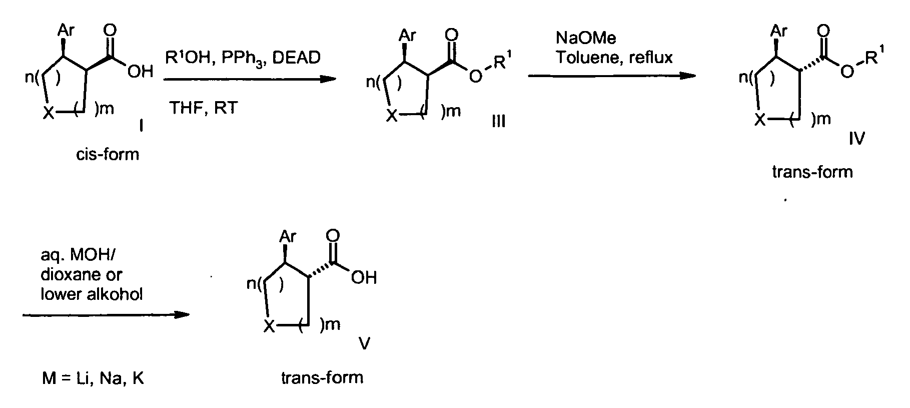 wherein R¹ is C₁₋₇-alkyl or benzyl, Ar is aryl¹ or heteroaryl¹.

Advantageously, the stereochemical integrity of the stereocenter β to the carboxylic function bearing the aryl or heteroaryl group is preserved during the epimerization. Therefore, enantioselective hydrogenation of acids II as described herein is unique in that it allows access to all possible stereoisomers of cyclic β-aryl or heteroaryl carboxylic acids and their derivatives in enantiomerically enriched or pure form, i.e. cis-substituted acids I and their derivatives as well as trans-substituted esters IV and acids V and their derivatives.

Chiral enantiomerically enriched or pure compounds of formula I or V and their derivatives are of great interest, e.g., as intermediates or starting materials for the preparation of a range of pharmaceutically active compounds.

Fiduxosin (ABT-980), α₁ₐ-adrenoreceptor antagonist, development compound at Abbot for the treatment of benign prostate hyperplasia, Organic Process Research & Development 2004, 8, 897-902 and references cited therein.

### Synthetic route:

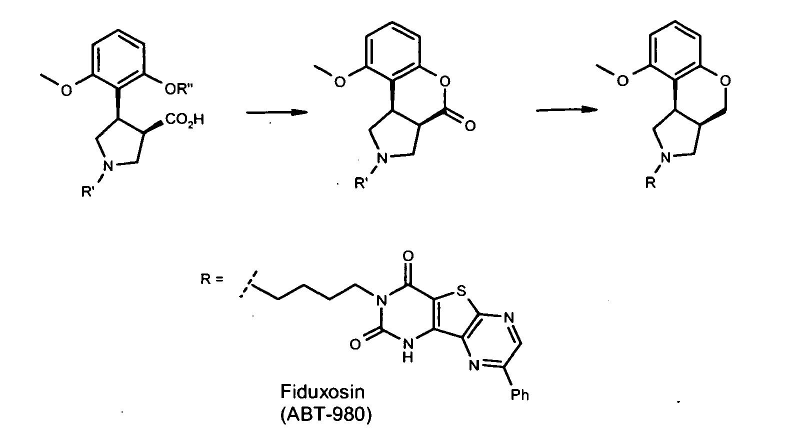

Melanocortin-4 receptor agonists for the treatment of obesity. Bioorg. Med. Chem. Lett. 2003, 13,4431 & 2005, 15, 4023 (Merck Sharp & Dohme): 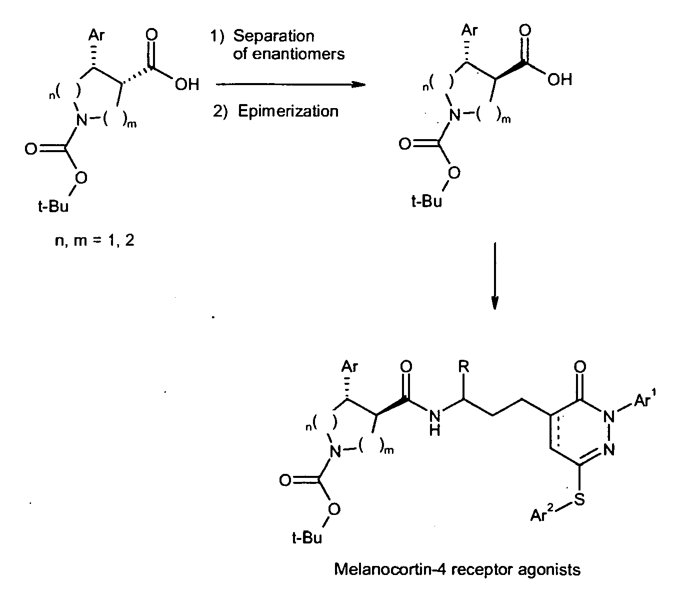 Melanocortin-4 receptor agonists for the treatment of obesity. WO02068388; J. Org. Chem. 2005, 70, 3592 (Merck Sharp & Dohme): 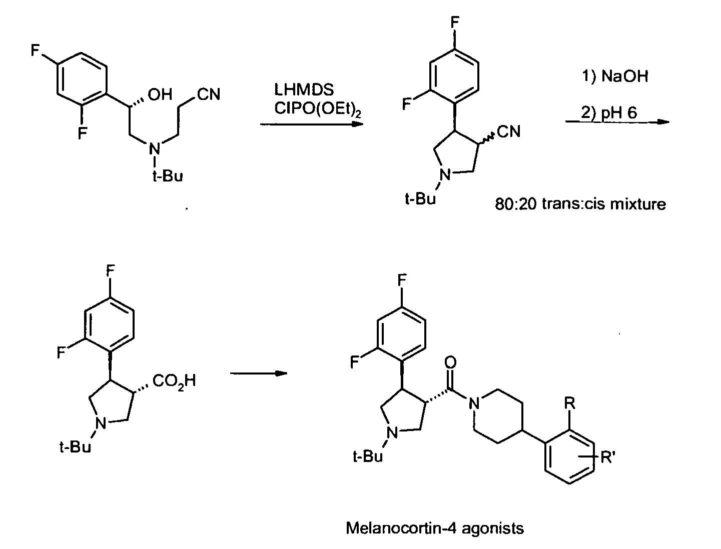 Chemokine receptor CCR5 antagonists for the treatment of viral infections. Bioorg. Med. Chem. Lett. 2004, 14,941 (Merck Sharp & Dohme): 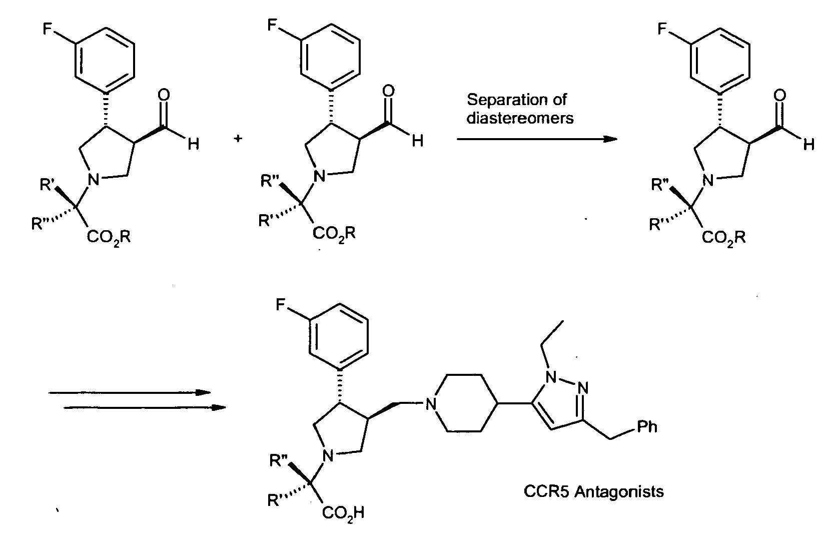 Chemokine receptor CCR5 antagonists for the treatment of viral infections. Bioorg. Med. Chem. Lett. 2001, 11, 1437 (Merck Sharp & Dohme): 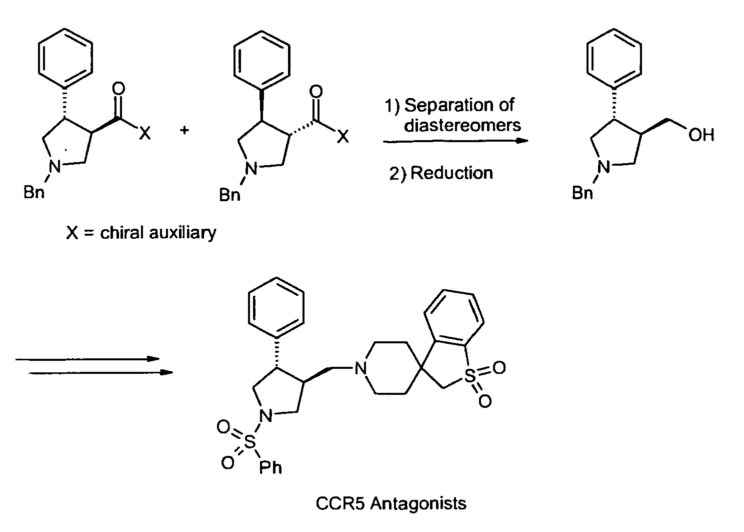 Factor Xa inhibitors as antithrombotic agents. Bioorg. Med. Chem. Lett. 1999, 9, 1195 (DuPont): 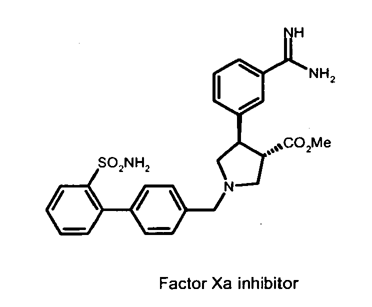 The marketed selective serotonin reuptake inhibitor paroxetine for the treatment of depression and anxiety. Process for the preparation of paroxetine: WO0129031: 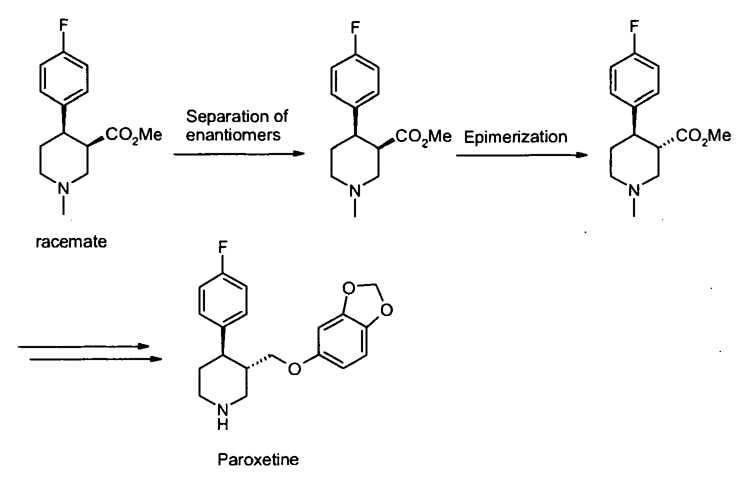 Dopamine and norepinephrine uptake inhibitor (+)-CPCA, potentially useful for the treatment of cocaine dependence and craving. The Journal of Pharmacology and Experimental Therapeutics 2003, 305, 143: 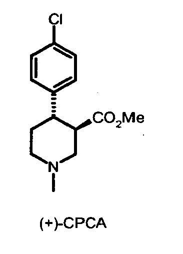 Tachykinin receptor antagonists, potentially useful preventives or remedies for lower urinary tract dysfunction, digestive organ diseases or central nervous diseases. WO2005068427: 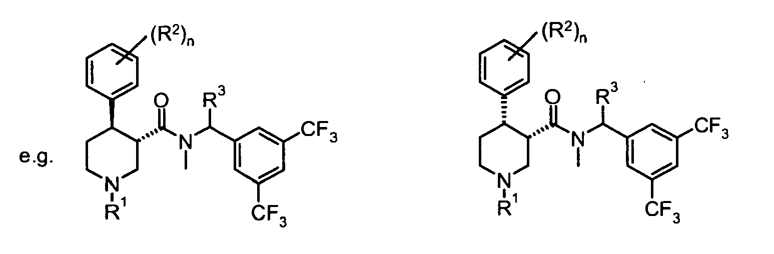 Dual Neurokinin-1 receptor antagonists and selective serotonin reuptake inhibitors, useful, e.g., for the treatment of depression and/or anxiety. US20060020011: 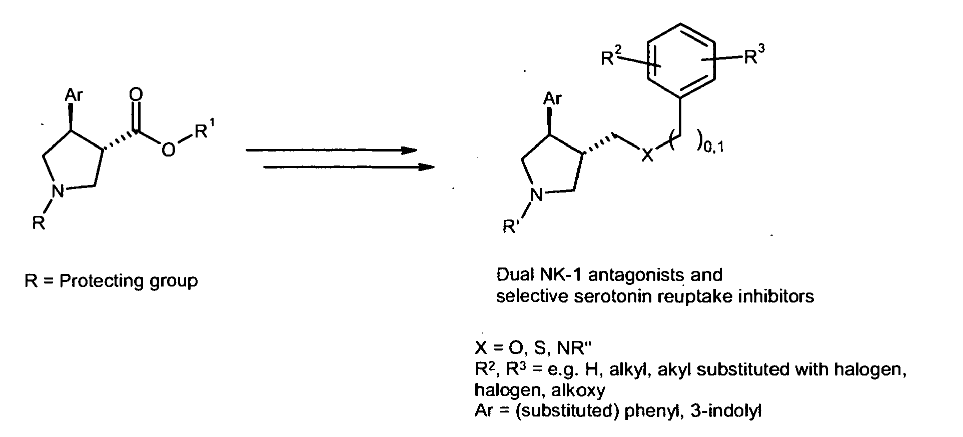 Synthetic access to the starting materials II for the enantioselective hydrogenation is straightforward from readily available β-ketoesters VII. A number of such compounds VII are commercially available. Compounds VII can also be prepared in a straightforward manner from ketones VI by consecutive treatment of a ketone VI with a suitable base, e.g. lithium diisopropylamide, lithium hexamethyldisilazide, alkyllithium with or without additives such as N,N,N',N'-tetramethylethlenediamine, lithium, sodium or potassium alkoxide.or sodium hydride in a suitable solvent such as tetrahydrofuran followed by a source of the carboxylate moiety, e.g. an alkyl or benyzl chloroformate or carbonate. β-Ketoesters VII can be transformed into triflates VIII by treatment with a bases such as sodium hydride and a triflating agent such as N-phenyltrifluoromethanesulfonimide. Coupling of a triflate VIII with an arylating agent such as, e.g., arylzinc halide or arylboronic acid or ester using a suitable palladium catalyst such as tetrakis(triphenylphosphine)palladium gives esters IX which are saponified in the usual manner to acids II.

### Synthesis of starting materials:

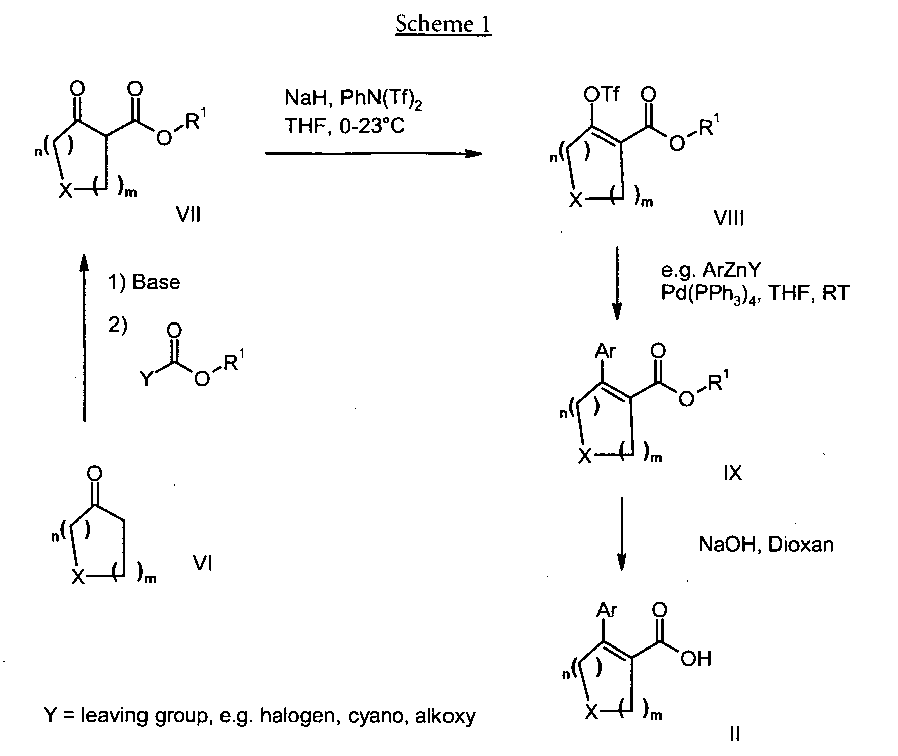

wherein R¹ is C₁₋₇-alkyl or benzyl, Ar is aryl¹ or heteroaryl¹, X is -C(R)(R')-, -N(R")-, -O-, -S(O)ₒ-, C(O)N(R"), -N(R")C(O)- or -C(O)-; R and R' are independently from each other hydrogen, C₁₋₇-alkyl, C₁₋₇-alkyl substituted by halogen, C₁₋₇-alkoxy, hydroxy or -(CH₂)ₚ-Ar; R" is hydrogen, C₁₋₇-alkyl, C₁₋₇-alkyl substituted by halogen, S(O)ₒ-C₁₋₇-alkyl, S(O)ₒ-Ar, S(O)ₒ-NRR', -(CH₂)ₚ-Ar, -C(O)-C₁₋₇-alkyl, -C(O)-Ar, -C(O)-NRR' or -C(O)O-C₁₋₇-alkyl; n and m are independently from each other 0, 1, 2 or 3; o is 0, 1 or 2; p is 0, 1, or 2;

As illustrated by the examples above, acids I and V, wherein X = NR", are of particular interest as precursors for the synthesis of, e.g., pharmaceutically active ingredients. 1-Benzyl-3-oxo-piperidine-4-carboxylic acid ethyl ester and 1-benzyl-4-oxo-piperidine-3-carboxylic acid methyl and ethyl ester are commercially available and are thus the most convenient starting materials VII-1 for the synthesis of acids II, wherein X = NR" and n = 1 and m = 2 or n = 2 and m = 1. For practical reasons it may be advantageous to change the *N*-protecting group from benzyl to *tert*-butoxycarbonyl (BOC), e.g. as described in scheme 2. 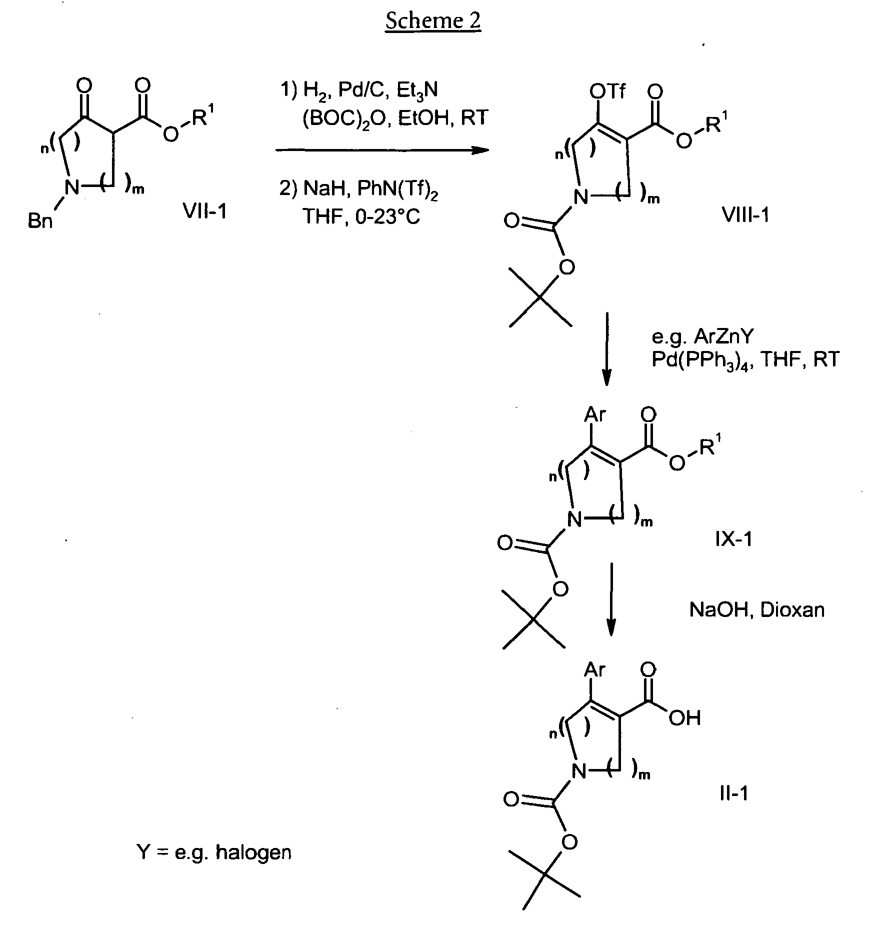 wherein R¹ is C₁₋₇-alkyl or benzyl, Ar is aryl¹ or heteroaryl¹, m, n are independently from each other 1, 2 or 3;

Acids II-2 or II-3, wherein n and m = 1, may alternatively be prepared via the route described in scheme 3: Dipolar 2+3 cycloaddition of aryl-propynoic acid ester XI with an azomethine ylide formed in situ under the reaction conditions from X leads to IX-2, which can either be saponified directly in the usual manner to acid II-2 or transformed into II-3 after change of the protecting group and saponification 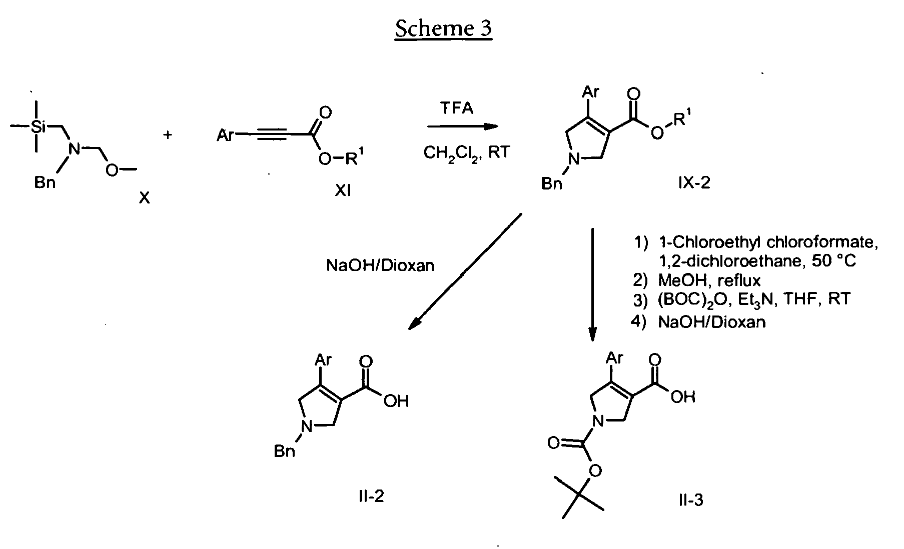 wherein R¹ is C₁₋₇-alkyl or benzyl and Ar is as defined above.

Acid II-4 is prepared via the route described in scheme 4: Dipolar 3+2 cycloaddition of thiole XIV with the phosphonic ester XVI to give thiophene XVII, which is saponified directly in the usual manner to acid II-4.

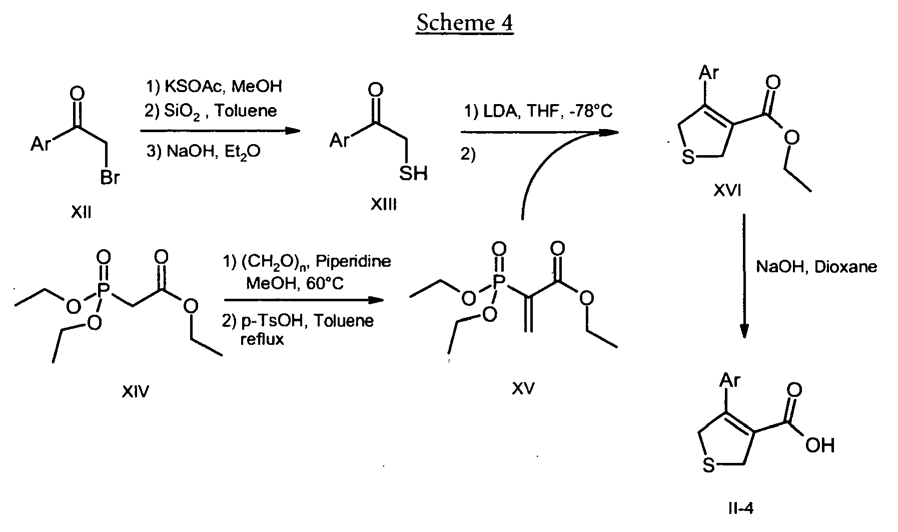

The invention may be described in detail as follow:

### Starting materials:

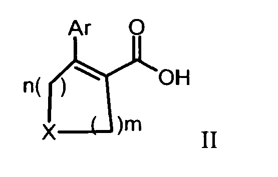

Tetrasubstituted acids II may, e.g., be the following:
2-aryl/heteroaryl-cyclopent-1-ene carboxylic acids,
4-aryl/heteroaryl-2,5-dihydro-1H-pyrrole-3-carboxylic acids,
4-aryl/heteroaryl-2,5-dihydro-furan-3-carboxylic acids,
4-aryl/heteroaryl-2,5-dihydro-thiophene-3-carboxylic acids,
1,1-dioxo-4-aryl-2,5-dihydro-1H-1λ⁶-thiophene-3-carboxylic acids,
2-aryl/heteroaryl-cyclohexyl-1-ene carboxylic acid,
4-aryl/heteroary -1,2,5,6-tetrahydro-pyridine-3-carboxylic acids,
5-aryl/heteroaryl-1,2,3,6-tetrahydro-pyridine-4-carboxylic acids,
4-aryl/heteroaryl-5,6-dihydro-2H-pyran-3-carboxylic acids,
5-aryl/heteroaryl-3,6-dihydro-2H-pyran-4-carboxylic acids,
4-aryl/heteroaryl-5,6-dihydro-2H-thiopyran-3-carboxylic acids,
5-aryl/heteroaryl -3,6-dihydro-2H-thiopyran-4-carboxylic acids,
1,1 -dioxo-4-aryl/heteroaryl-1,2,5,6-tetrahydro-1λ⁶-thiopyran-3-carboxylic acids,
1,1-dioxo-5-aryl/heteroaryl -1,2,3,6-tetrahydro-1λ⁶-thiopyran-4-carboxylic acids,
1-oxo-4-aryl/heteroaryl-1,2,5,6-tetrahydro-1λ⁴-thiopyran-3-carboxylic acids,
1-oxo-4-aryl/heteroaryl -2,5-dihydro-1H-1λ⁴-thiophene-3-carboxylic acids,
2-phenyl-cyclohept-1-enecarboxylic acid
2-phenyl-cyclooct-1-enecarboxylic acid and corresponding salts thereof.

### Products:

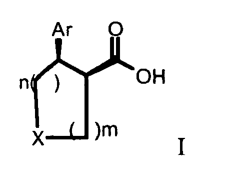

Acids I may be the following:
2-aryl/heteroaryl-cyclopentane carboxylic acids,
4-aryl/heteroaryl-2,5-dihydro-1H-pyrrolidine-3-carboxylic acids,
4-aryl/heteroaryl-tetrahydrofuran-3-carboxylic acids,
4-aryl/heteroaryl-tetrahydro-thiophene-3-carboxylic acids,
1,1-dioxo-4-aryl/heteroaryl-tetrahydro-1λ⁶-thiophene-3-carboxylic acids,
1-oxo-4-aryl/heteroaryl-tetrahydro-1λ⁴-thiophene-3-carboxylic acids,
2-aryl/heteroaryl-cyclohexane carboxylic acid,
4-aryl/heteroaryl-piperidine-3-carboxylic acids,
5-aryl/heteroaryl-piperidine-4-carboxylic acids,
4-aryl/heteroaryl-tetrahydro-pyran-3-carboxylic acids,
5-aryl/heteroaryl-tetrahydro-pyran-4-carboxylic acids,
4-aryl/heteroaryl-tetrahydro-thiopyran-3-carboxylic acids,
5-aryl/heteroaryl-tetrahydro-thiopyran-4-carboxylic acids,
1,1-dioxo-4-aryl/heteroaryl-hexahydro-1λ⁶-thiopyran-3-carboxylic acids,
1,1-dioxo-5-aryl/heteroaryl-hexahydro-1λ⁶-thiopyran-4-carboxylic acids,
1-oxo-4-aryl/heteroaryl-hexahydro-1λ⁴-thiopyran-3-carboxylic acids,
2-phenyl-cycloheptane carboxylic acid,
2-phenyl-cyclooctane carboxylic acid and corresponding salts thereof.

### Catalysts:

### Ruthenium complex catalysts:

In the ruthenium complex catalysts ruthenium is characterised by the oxidation number II. Such ruthenium complexes can optionally comprise further ligands, either neutral or anionic. Examples of such neutral ligands are e.g. olefins, e.g. ethylene, propylene, cyclooctene, 1,3-hexadiene, norbornadiene, 1,5-cyclooctadiene, benzene, hexamethylbenzene, 1,3,5-trimethylbenzene, p-cymene, or also solvents such as e.g. tetrahydrofuran, dimethylformamide, acetonitrile, benzonitrile, acetone, toluene and methanol. Examples of such anionic ligands are CH₃COO⁻, CF₃COO⁻ or halides. If the ruthenium complex is charged, non coordinating anions such as halides, BF₄⁻, ClO₄⁻, SbF₆⁻, PF₆⁻, B(phenyl)₄⁻, B(3,5-di-trifluoromethyl-phenyl)₄⁻, CF₃SO₃⁻, C₆H₅SO₃⁻ are present.

Suitable ruthenium complexes in question can be represented e.g. by the following formula

Ru(Z)₂D XVII

[Ru(Z)₂₋ₚ(D)(L¹)ₘ](B)ₚ XVIII

wherein Z represents halogen or the group A-COO, A represents lower alkyl, aryl², halogenated lower alkyl or halogenated aryl², D represents a chiral diphosphine ligand, B represents a non coordinating anion as defined above and L¹ represents a neutral ligand as defined above, p represents the numbers 1 and 2, the ligands can be the same or different, m represents the number 1, 2 or 3.

These complexes can in principle be manufactured in a manner known per se, e.g. according to B. Heiser et al., Tetrahedron: Asymmetry 1991,2,51 or N. Feiken et al., Organometallics 1997,16,537 or J.-P. Genet, Acc. Chem. Res. 2003, 36, 908 or K. Mashima et al., J. Org. Chem. 1994, 53, 3064 and references cited therein.

Conveniently and preferably, ruthenium complexes are manufactured, for example, by reacting a complex of the formula

[Ru(Z¹)₂(L¹)ₘ]ₚ(H₂O)_{q} XIX

wherein Z¹ represents halogen or a group A¹-COO, A¹ represents lower alkyl or halogenated lower alkyl, L¹ represents a neutral ligand as defined above, m represents the number 1, 2 or 3, p represents the number 1 or 2 and q represents the number 0 or 1, with a chiral diphosphine ligand. Where m represents the number 2 or 3, the ligands can be the same or different.

Typically, ruthenium catalysts exemplified within the present invention can be prepared according to the method described by M.P. Fleming et al., US 6,545,165 B1, for the preparation of chiral ruthenium dicarboxylate diphosphines.

### Rhodium complex catalysts:

In the rhodium complex catalysts rhodium is characterised by the oxidation number I, and contains a chiral phosphine ligand. Such rhodium complexes can optionally comprise further ligands, either neutral or anionic.

Examples of such neutral ligands are e.g. olefins, e.g. ethylene, propylene, cyclooctene, 1,3-hexadiene, 1,5-hexadiene, norbornadiene (nbd = bicyclo-[2.2.1]hepta-2,5-diene), (Z,Z)-1,5-cyclooctadiene (cod) or other dienes which form readily soluble complexes with rhodium or ruthenium, benzene, hexamethylbenzene, 1,3,5-trimethylbenzene, p-cymene, or also solvents such as e.g. tetrahydrofuran, dimethylformamide, acetonitrile, benzonitrile, acetone, methanol and pyridine.

Examples of such anionic ligands are halides or the group A-COO⁻, wherein A represents lower alkyl, aryl², halogenated lower alkyl or halogenated aryl². Preferably, A-COO is CH₃COO⁻ or CF₃COO⁻. If the rhodium complex is charged, non coordinating anions such as a halide, BF₄⁻, ClO₄⁻, SbF₆⁻, PF₆⁻, B(phenyl)₄⁻, B(3,5-di-trifluoromethylphenyl)₄⁻, CF₃SO₃⁻, C₆H₅SO₃⁻ are present.

Preferred catalysts comprising rhodium and a chiral diphosphine are of the formula

[Rh(chiral diphosphine)LX] or [Rh(chiral diphosphine)L]⁺A⁻

wherein X is a halide such as Cl⁻, Br⁻ or I⁻, L is a neutral ligand as defined above and A is an anion of an oxyacid or a complex acid such as ClO₄, PF₆, BR₄; wherein R is halogen or aryl², SbF₆ or AsF₆. If L is a ligand comprising two double bonds, e.g. 1,5-cyclooctadiene, only one such L is present. If L is a ligand comprising only one double bond, e.g. ethylene, two such L are present.

A rhodium complex catalyst can be prepared, for example, by reaction of rhodium precursors such as e.g. di-η⁴-chloro-bis[η⁴-(Z,Z)-1,5-cyclo- octadiene]dirhodium(I) ([Rh(cod)Cl]₂), di-µ-chloro-bis[η⁴-norbornadiene]- dirhodium(I) ([Rh(nbd)Cl]₂), bis[η⁴-(Z,Z)-1,5-cyclooctadiene]rhodium tetra- fluoroborate ([Rh(cod)₂]BF₄) or bis[η⁴-(Z,Z)-cyclooctadiene]rhodium perchlorate ([Rh(cod)₂]ClO₄) with a chiral diphosphine ligand in a suitable inert organic or aqueous solvent (e.g. according to the methods described in Experimental Chemistry, 4th edition, Vol. 18, Organometallic complexes, pp. 339-344, Ed. Chemical Society of Japan, 1991, Maruzen or J.Am. Chem. Soc. 1971, 93, 2397 or E. Jacobsen, A. Pfaltz, H. Yamamoto (Eds.), Comprehensive Aymmetric Catalysis I-III, Springer Verlag Berlin (1999) and references cited therein).

Rhodium or ruthenium complex catalysts as described above can also be prepared in situ, i.e. just before use and without isolation. The solution in which such a catalyst is prepared can already contain the substrate for the enantioselective hydrogenation or the solution can be mixed with the substrate just before the hydrogenation reaction is initiated.

The chiral diphosphine ligand is characterised by formula (3), (4), (5), (6), (7), (8), (9), (10), (11), (12), (13), (14), (15) or (16). 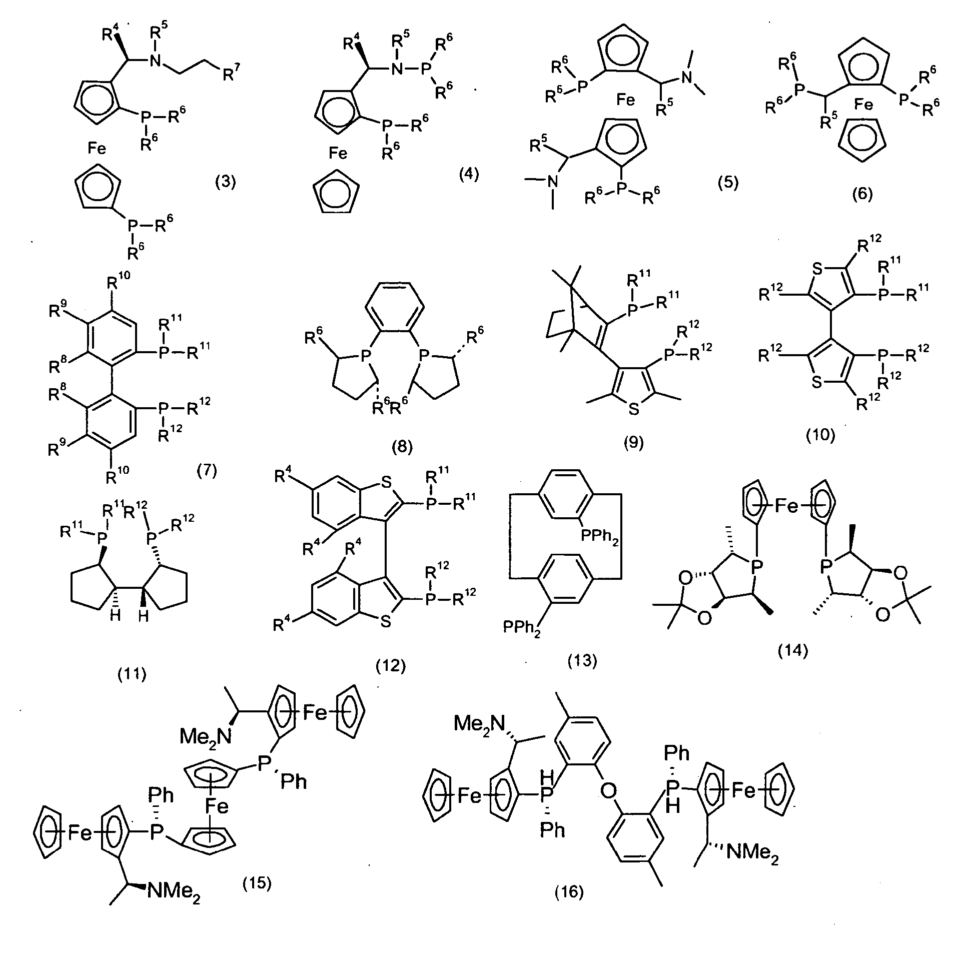 wherein
- R⁴: is lower-alkyl;
- R⁵: is lower-alkyl;
- R⁶: independently is aryl², heteroaryl², cycloalkyl or lower-alkyl;
- R⁷: is N(lower-alkyl)₂ or piperidinyl;
- R⁸: is lower-alkyl, lower-alkoxy, hydroxy or lower-alkyl-C(O)O-;
- R⁹ and R¹⁰: independently are hydrogen, lower-alkyl, lower-alkoxy or di(lower-alkyl)amino; or
- R⁸ and R⁹: which are attached to the same phenyl group, or R⁹ and R¹⁰ which are attached to the same phenyl group, or both R⁸, taken together, are -X-(CH₂)ₙ-Y-, wherein X is -O- or -C(O)O-, Y is -O- or -N(lower-alkyl)- and n is an integer from 1 to 6, or a CF₂ group; or
- R⁸ and R⁹, or R⁹ and R¹⁰,: together with the carbon atoms to which they are attached, form a naphthyl, tetrahydronaphthyl or dibenzofuran ring;
- R¹¹ and R¹²: independently are lower alkyl, cycloalkyl, phenyl, napthyl or heteroaryl, substituted with 0 to 7 substituents independently selected from the group consisting of lower-alkyl, lower-alkoxy, di(lower-alkyl)amino, morpholino, phenyl and tri(lower-alkyl)silyl;

If R¹¹ is phenyl, it is substituted with 0 to 5, preferably 0 to 3 substituents as described above.

Unless otherwise indicated, the following definitions are set forth to illustrate and define the meaning and scope of the various terms used to describe the invention herein.

The term "halogen" refers to fluorine, chlorine, bromine and iodine, with fluorine, bromine and chlorine being preferred.

The term "lower alkyl", alone or in combination with other groups, refers to a branched or straight-chain monovalent alkyl radical of one to seven carbon atoms, preferably one to four carbon atoms. This term is further exemplified by radicals such as methyl, ethyl, n-propyl, isopropyl, n-butyl, s-butyl, isobutyl, t-butyl, n-pentyl, 3-methylbutyl, n-hexyl, 2-ethylbutyl and the like. Preferable lower alkyl residues are methyl and ethyl, with methyl being especially preferred.

The term "halogenated lower alkyl" refers to a lower alkyl group as defined above wherein at least one of the hydrogens of the lower alkyl group is replaced by a halogen atom, preferably fluoro or chloro. Among the preferred halogenated lower alkyl groups are trifluoromethyl, difluoromethyl, fluoromethyl and chloromethyl.

The term "alkoxy" refers to the group R'-O-, wherein R' is alkyl. The term "lower-alkoxy" refers to the group R'-O-, wherein R' is a lower alkyl group as defined above. Examples of lower alkoxy groups are e.g. methoxy, ethoxy, propoxy, isopropoxy, butoxy, isobutoxy and hexyloxy, with methoxy being especially preferred.

The term "aryl²" refers to an aromatic monovalent mono- or polycarbocyclic radical, such as phenyl or naphthyl, preferably phenyl, which may optionally be substituted by one or more substituents, independently by C₁₋₇-alkyl, C₁₋₇-alkoxy, halogen, C₁₋₇-alkyl substituted by halogen, cyano, azido, amino, mono-or di-C₁₋₇-alkyl amino, SO₂H,
SO₂-lower alkyl, nitro, C(O)O-C₁₋₇-alkyl, C(O)-mono-or di-C₁₋₇-alkyl amino, hydroxy or the like.

The term "heteroaryl²" denotes a monovalent heterocyclic 5 or 6-membered aromatic radical, wherein the heteroatoms are selected from N, O or S, for example the groups thiophenyl, indolyl, pyridinyl, pyrimidinyl, imidazolyl, piperidinyl, furanyl, pyrrolyl, isoxazolyl, pyrazolyl, pyrazinyl, benzo[1.3]dioxolyl, benzo{b}thiophenyl or benzotriazolyl, which may optionally be substituted by one or more substituents, independently by C₁₋₇-alkyl, C₁₋₇-alkoxy, halogen, C₁₋₇-alkyl substituted by halogen, cyano, azido, amino, mono-or di-C₁₋₇-alkyl amino, SO₂H SO₂-lower alkyl, nitro, C(O)O-C₁₋₇-alkyl, C(O)-mono-or di-C₁₋₇-alkyl amino, hydroxy or the like.

The term "cycloalkyl" refers to a monovalent carbocyclic radical of three to eight, preferably four to six carbon atoms. This term is further exemplified by radicals such as cyclopropyl, cyclobutyl, cyclopentyl and cyclohexyl, with cyclopentyl and cyclohexyl being preferred. Such cycloalkyl residues may optionally be mono-, di- or tri-substituted, independently, by lower alkyl or by halogen.

In a more preferred embodiment, the catalyst is of the formula Ru(Z)₂D, wherein the chiral diphosphine is characterised by formula (7), (9), (10) or (12) and wherein Z is CH₃COO, CF₃COO or a halogenide.

Preferably, the chiral diphosphine is selected from the group consisting of (R) and (S)-enantiomers : MeOBIPHEP, BIPHEMP, TMBTP, 2-Naphthyl)-MeOBIPHEP, (6-MeO-2-Naphthyl)-MeOBIPHEP, 2-(Thienyl)-MeOBIPHEP, 3,5-tBu-MeOBIPHEP, PHANEPHOS, BICP, TriMeOBIPHEP, (R,R,S,S)-Mandyphos, BnOBIPHEP, BenzoylBIPHEP, pTol-BIPHEMP, tButylCOOBIPHEP, iPrOBIPHEP, p-Phenyl-MeOBIPHEP, pAn-MeOBIPHEP, pTol-MeOBIPHEP, 3,5-Xyl-MeOBIPHEP, 3,5-Xyl-BIPHEMP, BINAP and 2-Furyl-MeOBIPHEP, 3,5-Xyl-4-MeO-MeOBIPHEP, 2-Furyl-MeOBIPHEP, BITIANP, DuanPHos, C2-Tunaphos, f-BINAPHANE, Stylacat 4/1, TOLFER Stylacat 4/2 or Stylacat 3/1/1. More preferably, the chiral diphosphine is ((*S*)-(6-MeO-2-Naphthyl)-MeOBIPHEP, 3,5-Xyl-4-MeO-MeOBIPHEP, (*S*)-2-Furyl-MeOBIPHEP or BITIANP. Each of these chiral diphosphines individually constitutes a preferred embodiment of the present invention.

### Solvents for ruthenium complexes:

Alcohols, hydrocarbons, chlorinated hydrocarbons, supercritical or liquid carbon dioxide, THF or water. Preferred solvents are alcohols.

### Solvents for rhodium complexes:

alkanols or aromatic hydrocarbons, such as benzene, toluene, trifluoro toluene, or halogenated hydrocarbons, such as dichloromethane, dichlororethane, etc., or polyalcohols such as ethylene glycole, or amides such as DMF, DMA, N-methylpyrrolidinone, or supercritical or liquid carbon dioxide, acetonitrile or DMSO.

The solvents may be used alone or as mixture of solvents mentioned above.

The concentration of solvents is 1-50 W%, preferentially 5-20%.

Additives:
Bases: tertiary amines, such as NEt₃, i-Pr₂NEt,
secondary amines, such as iPr₂NH,
primary amines, such as C₆H₅CH₂NH₂, 1-phenyl-benzylamine, (*R*) or (*S*)), diamines, such as ethylene diamine, tetramethylethylene diamine,
salts of carboxylic acids, such as NaOAc, of alcoholates, such as NaOEt, or of NaOH. tetrasubstituted ammonium salts, such as Bu₄NX (X= F, Cl, Br, I)
Preferred additives are tertiary amines as described above.
The amounts of base is in the range of 0.1-100 equivalents, preferrentially 0.1-1.2 molar equivalents. Most preferred range is 0.15-1 molar equivalent.

Reaction conditions:
Pressure: 1-150 bar, preferentially 10-100 bar.
Temperature: 10-100°C, preferentially 20-80°C.
Substrate/catalyst ratio (s/c): 5-30000, preferentially 100-10000

### General description

With regard to the invention, the process for the preparation of enantiomerically enriched cyclic β-arylcarboxylic acid derivatives of formula 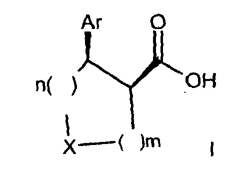 comprises catalytic homogeneous enantioselective hydrogenation of a compound of formula (II) 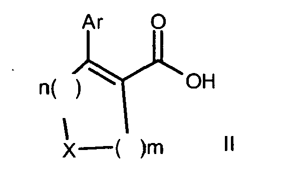 wherein
- X: is -C(R)(R')-, -N(R")-, -O-, -S(O)ₒ-, C(O)N(R"), -N(R")C(O)- or -C(O)-;
- R and R': are independently from each other hydrogen, C₁₋₇-alkyl, C₁-₇-alkyl substituted by
halogen, C₁₋₇-alkoxy, hydroxy or -(CH₂)ₚ-Ar;
- R": is hydrogen, C₁₋₇-alkyl, C₁₋₇-alkyl substituted by halogen, -S(O)ₒ-C₁₋₇-alkyl, -S(O)ₒ-Ar, -S(O)ₒ-NRR', -(CH₂)ₚ-Ar, -C(O)-C₁₋₇-alkyl, -C(O)-Ar, -C(O)-NRR' or -C(O)O-C₁₋₇-alkyl;
- Ar: is aryl¹ or heteroaryl¹;

- n: is 0, 1,2 or 3;
- m: is 0, 1,2 or 3;
- o: is 0, 1 or 2;
- p: is 0, 1, or 2;
and corresponding salts thereof
in the presence of a catalyst comprising

Ru(Z)₂D XII

wherein Z represents halogen or the group A-COO, A represents lower alkyl, aryl², halogenated lower alkyl or halogenated aryl² and D represents a chiral diphosphine ligand, or comprises

[Rh(chiral diphosphine)LX] or [Rh(chiral diphosphine)L]⁺A⁻

wherein X is Cl⁻, Br⁻ or I⁻, L is a neutral ligand, selected from the group consisting of ethylene, propylene, cyclooctene, 1,3-hexadiene, norbornadiene, 1,5-cyclooctadiene, benzene, hexamethylbenzene, 1,3,5-trimethylbenzene, p-cymene, tetrahydrofuran, dimethylformamide, acetonitrile, benzonitrile, acetone or methanol,
A is an anion of an oxyacid or a complex acid selected from the group consisting of ClO₄, PF₆, BR₄, wherein R is halogen or aryl, SbF₆ or AsF₆,
to yield said compound of formula (I).

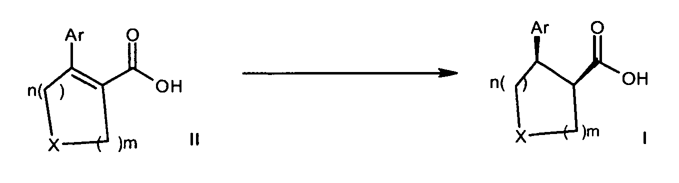

In a glove box an autoclave equipped with a glass insert and a magnetic stirring bar is charged with a compound of formula II, for example with 2-phenyl-cyclohex-1-ene-carboxylic acid, with a ruthenium catalyst, such as [Ru(OAc)₂((*R*)-2-furyl)-MeOBIPHEP], with an additive, for example triethylamine and a solvent, such as methanol. The asymmetric hydrogenation is run for about 42 h at 20 - 80 °C under 40 bar of hydrogen. After cooling to room temperature the pressure is released from the autoclave, the solvent is diluted with *tert*-butyl methyl ether, extracted, dried and concentrated in vacuo to give a compound of formula I, for example (-)-2-phenylcyclohexane carboxylic acid.

Enantiomeric excess (ee) values were determined by chiral GC or HPLC.

### Experimental:

List of abbreviations for the used ligands:

| | |
|---|---|
| BIPHEMP¹ | (6,6'-Dimethylbiphenyl-2,2'-diyl)bis(diphenylphosphine) |
| pTol-BIPHEMP¹ | (6,6'-Dimethylbiphenyl-2,2'-diyl)bis(di-p-tolylphosphine) |
| 3,5-Xyl-BIPHEMP¹ | Phosphine, [6,6'-dimethoxy[1,1'-biphenyl]-2,2'-diyl]bis[bis(3,5-dimethylphenyl)- |
| MeOBIPHEP¹ | (6,6'-Dimethoxybiphenyl-2,2'-diyl)bis(diphenylphosphine) |
| (2-Naphthyl)-MeOBIPHEP¹ | (6,6'-Dimethoxybiphenyl-2,2'-diyl)bis(di-2-naphthylphosphin) |
| 6-MeO-2-Naphthyl-MeOBIPHEP¹ | (6,6'-Dimethoxybiphenyl-2,2'-diyl)bis(di-2-(6-methoxy)-naphthylphosphine) |
| 3,5-Xyl,4-MeO-MeOBIPHEP¹ | (6,6'-Dimethoxy[1,1'-biphenyl]-2,2'-diyl)bis[bis(3,5-di-*tert*-butyl-4-methoxyphenyl)phosphine) |
| 3,5-*t*-Bu-MeOBIPHEP¹ | (6,6'-Dimethoxy[1,1'-biphenyl]-2,2'-diyl)bis[bis(3,5-di-*tert*-butyl-phenyl)phosphine) |
| 2-Furyl-MeOBIPHEP¹ | (6,6'-Dimethoxybiphenyl-2,2'-diyl)bis(di-2-furylphosphine) |
| 2-Thienyl-MeOBIPHEP¹ | (6,6'-Dimethoxy[ 1,1'-biphenyl]-2,2'-diyl)bis(bis(2-thienyl)phosphine) |
| pPhenyl-MeOBIPHEP¹ | (6,6'-dimethoxy[1,1'-biphenyl]-2,2'-diyl)bis[bis([1,1'-biphenyl]-4-yl)- phosphine |
| pAn-MeOBIPHEP¹ | (6,6'-dimethoxy[1,1'-biphenyl]-2,2'-diyl)bis[bis(4-methoxyphenyl)- phosphine |
| pTol-MeOBIPHEP¹ | (6,6'-Dimethoxybiphenyl-2,2'-diyl)bis[di(p-tolyl)phosphine] |
| 3,5-Xyl-MeOBIPHEP¹ | [6,6'-Dimethoxy[1,1'-biphenyl]-2,2'-diyl]bis[bis(3,5-dimethylphenyl)phosphine (CAS Reg. No. 394248-45-4 *(R)*) |
| TriMeOBIPHEP¹ | Phosphine, (4,4',5,5',6,6'-hexamethoxy[1,1'-biphenyl]-2,2'-diyl)bis [diphenyl] |
| BenzoylBIPHEP⁶ | (6,6'-Dibenzoyloxybiphenyl-2,2'-diyl)bis(diphenylphosphin) |
| tButylCOOBIPHEP⁶ | Propanoic acid, 2,2-dimethyl-,6,6'-bis(diphenylphosphino) [1,1'-biphenyl]-2,2'-diyl ester |
| iPrOBIPHEP¹ | (6,6'-Di-2-propoxybiphenyl-2,2'-diyl)bis(diphenylphosphin) |
| BnOBIPHEP¹ | (6,6'-Dibenzyloxybiphenyl-2,2'-diyl)bis(diphenylphosphin) |
| BINAP | 2,2'-Bis(diphenylphosphino)-1,1'-binaphthyl (commercially available from Fluka) |
| DIOP | 1,4-Bis-(diphenylphosphino)-1,4-dideoxy-2,3-O.isopropylidene-threitol (commercially available from Fluka) |
| BITIANP² | 3,3'-bis-diphenylphosphanyl-1H,1'H-[4,4']-biisothiochromenyl |
| BICP³ | 2,2'-bis(diphenylphosphino)-(1*S*,1'*S*,2*S*,2'*S*)-1,1'-bicyclopentyl |
| DuanPhos³ | 2,2'-Di-tert-butyl-2,3,2',3'-tetrahydro- H,1'H-(1,1')-biisophosphinolyl |
| C2-Tunaphos³ | (6,6'-O-[1,2-ethylene]-oxybiphenyl-2,2'-diyl)-bis(diphenyl)phosphine |
| f-BINAPHANE³ | 1,1'-Bis-((*S*)-4,5-dihydro-3H-dinaphtho[2,1-c:1',2'-e] phosphepino)-ferrocene |
| PHANEPHOS⁴ | 4,12-Bis(diphenylphosphino)[2.2]-paracyclophane |
| TMBTP⁵ | 2,2',5,5'-Tetramethyl-4,4'-bis(diphenylphosphino)-3,3'-bithiophene |
| Mandyphos⁴ | 1,1'-Bis[(dimethylamino)phenylmethyl]-2,2'-bis(diphenyl-diphosphino)-ferrocene |
| Stylacat 4/1⁷ | 1,1'-bis-[((1-*N*,*N*-Dimethylamino)ethylferrocenyl)-(phenylphosphino)]ferrocene |
| TOLFER Stylacat 4/2⁷ | 2,2'-(bis-[((1-*N*,*N*-Dimethylamino)ethylferrocenyl)-phenylphosphino]-4-tolylether |
| Stylacat 3/1/1⁷ | 2-[1-[(*N*-Methyl-*N*-diphenylphosphino)amino]ethyl]-1-[(1-naphthyl)phenylphosphino]ferrocene |

| | |
|---|---|
| 1) These ligands are known and/or can be prepared according to the examples or methods as described in patent application documents EP 0 398 132, WO 92/16535, EP 0 104 375 or EP 0 580 331. ² Synthesis and characterization described in: Benincori, T.; Brenna, E.; Sannicolo, F.; Trimarco, L.; Antognazza, P.; Cesarotti, E.; Demartin, F.; Pilati, T.J. Org. Chem. 1996, 61, 6244. ³ Commercially available from Chiral Quest Inc., Princeton Corporate Plaza, Monmouth Jct., NJ08852, USA ⁴ Commercially available from Strem Chemicals Inc. D-77672 Kehl ⁵ Commercially available from Chemi S.p. A., Via dei Lavoratori, Cinasello Balsamo, Milano 20092, Italy. ⁶Synthesis according to : Bulliard, Michel; Laboue, Blandine; Roussiasse, Sonia. Use of optically active acyloxy-substituted diphosphinobiphenyls as ligands for catalyzed asym. hydrogenation or isomerization, WO 2002012253 A1. 7 Commercially available from Phoenix Chemicals, 34 Thursby Road, Croft Business Park, Bromborough, Wirral, Merseyside CH62 3PW, UK. | |

### Enantioselective hydrogenations:

### Example 1 of I

### (+)-(3R,4R)-4-(4-Fluoro-phenyl)-piperidine-1,3-dicarboxylic acid-1-tert-butyl ester and (-)-(3S,4S)-4-(4-Fluoro-phenyl)-piperidine-1,3-dicarboxylic acid 1-tert-butyl ester

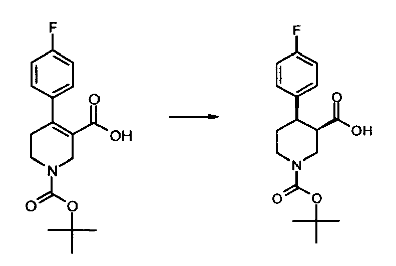

In a glove box (O₂ content ≤ 2 ppm) a 35 ml autoclave equipped with a 15 ml glass insert and a magnetic stirring bar was charged with 0.300 g (0.934 mmol) of 4-(4-fluorophenyl)-5,6-dihydro-2*H*-pyridine-1,3-dicarboxylic acid-1-*tert*-butyl ester, 9.67 mg (0.00936 mmol) of [Ru(OAc)₂((*S*)-3,5-Xyl-4-MeO)-MeOBIPHEP], 15 mg (0.16 mmol, 0.16 eq.) of triethylamine and 5 ml of methanol. The asymmetric hydrogenation was run for 42 h at 80°C under 40 bar of hydrogen. After cooling to room temperature the pressure was released from the autoclave, the methanol solution was diluted with 50 ml of *tert*-butyl methyl ether and extracted with two 50-ml portions of a 1 M aqueous sodium hydroxide solution. The aqueous layer was poured on ice, acidified with ice-cold 2 M aqueous hydrochloric acid solution to pH 1 and extracted with two 100-ml portions of ethyl acetate. The combined organic layers were dried over sodium sulfate, filtered and concentrated in vacuo to give (+)-(3*R*,4*R*)-4-(4-fluoro-phenyl)-piperidine-1,3-dicarboxylic acid-1-*tert*-butyl ester in 89 % yield (0.27 g) and with 96.6 % ee.

MS m/e (%): 322 (M-H⁺, 100).

### GC method for ee determination:

A 2-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethyl acetate. BGB-175 column, 10 m*0.1 mm*df 0.1 µm, hydrogen 230 kPa, split ratio 1 : 300; temperature gradient 100 - 200 °C, program with 2 °C/min; injector temperature 200 °C, detector temperature 210 °C. Retention times: 46.59 min (methyl ester of (+)-acid), 46.76 min (methyl ester of (-)-acid).

The absolute configuration was assigned as described below after transformation of the title compound to its trans isomer (-)-(3*S*,4*R*)-4-(4-fluoro-phenyl)-piperidine-1,3-dicarboxylic acid 1-tert-butyl ester (reaction sequence described in examples 1 of III and I of V).

In a similar manner, but in a 6 ml, 35 ml or 185 ml autoclave, the reactions in Table 1 were performed.

**Table 1:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1^{a)} | 0.05 | 25 | Ru(OAc)₂(*(R)*-MeOBIPHEP) + 0.86 toluene | 0.6 | 42 | 90 | (-)^{d)} | 94.6 |
| 2^{a)} | 0.05 | 25 | Ru(OAc)₂(*(S)*-(6-MeO-2-Naphtyl)-MeOBIPHEP) | 0.6 | 42 | 80 | (+)^{e)} | 95.8 |
| 3^{a)} | 0.05 | 25 | Ru(OAc)₂(*(R)*-3,5-tBu-MeOBIPHEP) | 0.6 | 42 | 88 | (-) | 93.8 |
| 4^{a)} | 0.05 | 25 | Ru(OAc)₂((+)-(*S*)-TMBTP | 0.6 | 42 | 84 | (+) | 88.5 |
| 5^{a)} | 0.05 | 25 | Ru(OAc)₂(*(S)*-3,5-Xyl,4-MeO-MeOBIPHEP) | 0.6 | 42 | 88 | (+) | 94.5 |
| 6^{a)} | 0.05 | 25 | Ru (OAc)₂((all-S)-BICP) | 0.6 | 42 | 84 | (+) | 82.3 |
| 7^{b)} | 0.3 | 100 | Ru(OAc)₂((*S*)-(6-MeO-2-Naphtyl)-MeOBIPHEP) | 0.16 | 42 | 90 | (+) | 91.5 |
| 9^{b)} | 0.3 | 100 | Ru(OAc)₂((*S*)-MeOBIPHEP) + 1.072 toluene | 0.16 | 42 | 90 | (+) | 92.8 |
| 11^{b)} | 0.3 | 250 | Ru(OAc)₂((*S*)-(6-MeO-2-Naphtyl)-MeOBIPHEP) | 0.16 | 42 | 87 | (+) | 94.7 |
| 12^{b)} | 0.3 | 250 | Ru(OAc)2((*S*)-3,5-Xyl,4-MeO-MeOBIPHEP) | 0.06 | 42 | 83 | (+) | 95.7 |
| 13 ^{c)} | 9.18 | 250 | Ru(OAc)₂((*S*)-3,5-Xyl,4-MeO-MeOBIPHEP) | 0.06 | 42 | 94 | (+) | 94.6 |
| 15^{c)} | 2.2 | 250 | Ru(OAc)₂((*S*)-3,5-Xyl,4-MeO-MeOBIPHEP) | 1 | 42 | 99 | (+) | 95.3 |

| | | | | | | | | |
|---|---|---|---|---|---|---|---|---|
| ^{a)} 35 ml autoclave.*^{b)}* 6 ml autoclave.^{c)} 185 ml autoclave.*^{d)}* [α]_{D} = -54.44 (c = 0.369, CHCl₃).^{e)} [α]_{D} = + 56.26 (c = 0.446, CHCl₃). | | | | | | | | |

### Example 2 of I

### (-)-4-(1H-Indol-3-yl)-piperidine-1,3-dicarboxylic acid-1-tert-butyl ester and (+)-4-(1H-Indol-3-yl)-piperidine-1,3-dicarboxylic acid-1-tert-butyl ester

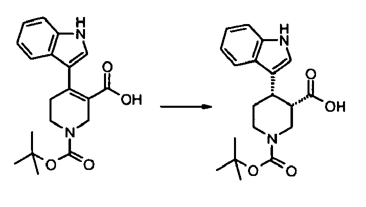

In a glove box (O₂ content ≤ 2 ppm) a 185 ml autoclave equipped with a mechanical stirrer was charged with 1.00 g (2.92 mmol) of 4-(1*H*-indol-3-yl)-5,6-dihydro-2*H-*pyridine-1,3-dicarboxylic acid-1-*tert*-butyl ester, 8.88 mg (0.0117 mmol) of [Ru(OAc)₂((*R*)-2-furyl)-MeOBIPHEP], 295 mg (2.92 mmol, 1.0 eq.) of triethylamine and 20 ml of methanol. The asymmetric hydrogenation was run for 42 h at 80°C under 40 bar of hydrogen. After cooling to room temperature the pressure was released from the autoclave, the methanol solution was diluted with 200 ml of tert-butyl methyl ether and extracted with two 100 ml portions of a 1 M aqueous sodium hydroxide solution. The aqueous layer was poured on ice, acidified with ice-cold 2 M aqueous hydrochloric acid solution to pH 1 and extracted with three 200-ml portions of ethyl acetate. The combined organic layers were dried over sodium sulfate, filtered and concentrated in vacuo to give (-)-4-(1*H*-indol-3-yl)-piperidine-1,3-dicarboxylic acid-1-*tert*-butyl ester in 89 % yield and with 98.8 % ee.

MS m/e (%): 245 (M+H⁺, 19).

### HPLC method for ee determination:

Chiralpak-OD-H column, 25 cm*4.6 mm, 90 % n-heptane and 10 % ethanol with 1 % trifluroacetic acid, flow 0.8 ml/min, 25 °C, 0.002 ml injection volume, 222 nm. Retention times: (-)-acid 13.4 min, (+)-acid 21.6 min.

In a similar manner, but in a 6 ml or 185 ml autoclave, the reactions in Table 2 were performed.

**Table 2:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1*^{a)}* | 0.05 | 25 | Ru(OAc)₂((rac)-BIPHEMP) | 1 | 42 | 60 | racemate | -- |
| 2*^{a)}* | 0.05 | 25 | Ru(OAc)₂((*R*)-(2-Furyl)-MeOBIPHEP) | 1 | 42 | 80 | (-)*^{c)}* | 99.0 |
| 3*^{a)}* | 0.05 | 25 | Ru(OAc)₂((*S*)-3,5-Xyl,4-MeO-MeOBIPHEP) | 1 | 42 | 80 | (+)*^{d)}* | 95.0 |
| 4*^{a)}* | 0.05 | 25 | Ru(OAc)₂((*S*)-(6-MeO-2-Naphtyl)-MeOBIPHEP) | 1 | 42 | 80 | (+) | 95.6 |
| 6*^{b)}* | 1.00 | 250 | Ru(OAc)₂((*S*)-(6-MeO-2-Naphtyl)-MeOBIPHEP) | 1 | 42 | 92 | (+) | 94.0 |

| | | | | | | | | |
|---|---|---|---|---|---|---|---|---|
| *^{a)}*6 ml autoclave.*^{b)}* 185 ml autoclave,*^{c)}* [α]_{D} = -94.46 (c = 0.29, MeOH). *^{d)}* [α]_{D} = +93.53 (c = 0.265 MeOH). | | | | | | | | |

### Example 3 of I

### (-)-4-o-Tolyl-piperidine-1,3-dicarboxylic acid 1-tert-butyl ester and (+)-4-o-Tolyl-piperidine-1,3-dicarboxylic acid 1-tert-butyl ester

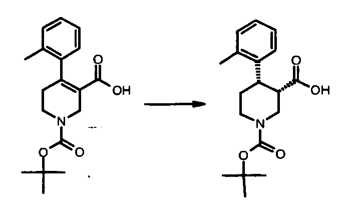

In a glove box (O₂ content ≤ 2 ppm) a 35 ml autoclave equipped with a 15 ml glass insert and a magnetic stirring bar was charged with 300 mg (0.945 mmol) of 4-o-tolyl-5,6-dihydro-2*H*-pyridine-1,3-dicarboxylic acid 1-*tert*-butyl ester, 7.2 mg (0.0094 mmol) of [Ru(OAc)₂((*R*)-(2-furyl)-MeOBIPHEP], 95.9 mg (0.945 mmol, 1.0 eq.) of triethylamine and 6 ml of methanol. The asymmetric hydrogenation was run for 42 h at 80°C under 40 bar of hydrogen. After cooling to room temperature the pressure was released from the autoclave, the methanol solution was diluted with 100 ml of *tert*-butyl methyl ether and extracted with two 100-ml portions of a 1 M aqueous sodium hydroxide solution. The aqueous layer was poured on ice, acidified with ice-cold 2 M aqueous hydrochloric acid solution to pH 1 and extracted with three 100-ml portions of ethyl acetate. The combined organic layers were dried over sodium sulfate, filtered and concentrated in vacuo to give (-)-4-o-tolyl-piperidine-1,3-dicarboxylic acid 1-*tert*-butyl ester in 75 % yield and with 99.1 % ee. Crystallization from ethyl acetate/n-heptane gave (-)-4-o-tolyl-piperidine-1,3-dicarboxylic acid 1-*tert*-butyl ester with >99.9 % ee.

MS m/e (%): 318 (M-H⁺, 100).

### [α]_{D} = -79.03 (c = 0.612, CHCl₃)

### HPLC method for ee determination:

Chiralpak-ADH column, 25 cm*4.6 mm, 85 % n-heptane + 15 % ethanol with 1 % trifluroacetic acid, flow 0.7 ml/min, 20 °C, 0.005 ml injection volume, 215 nm. Retention times: (-)-acid 8.1 min, (+)-acid 8.8 min.

In a similar manner, but in a 6 ml or 35 ml autoclave, the reactions in Table 3 were performed.

**Table 3:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1*^{a)}* | 0.1 | 25 | Ru(OAc)₂((rac)-BIPHEMP) | 0.5 | 48 | 98 | racemate | -- |
| 2*^{b)}* | 0.05 | 25 | Ru(OAc)₂(*(R)*-MeOBIPHEP) + 0.86 toluene | 0.7 | 42 | 80 | (-) | 80.5 |
| 3*^{b)}* | 0.05 | 25 | Ru(OAc)₂((*S*)-(6-MeO-2-Naphtyl)-MeOBIPHEP) | 0.7 | 42 | 80 | (+) | 82.9 |
| 4*^{b)}* | 0.05 | 25 | Ru(OAc)₂(*(R)*-3,5-tBu-MeOBIPHEP) | 0.7 | 42 | 80 | (-) | 50 |
| 5*^{b)}* | 0.05 | 25 | Ru(OAc)2(*(S)*-3,5-Xyl,4-MeO-MeOBIPHEP) | 0.7 | 42 | 80 | (+) | 76.2 |
| 6*^{b)}* | 0.05 | 25 | Ru(OAc)₂(*(R)*-MeOBIPHEP) + 0.86 toluene | 1 | 66.5 | 80 | (-) | 90.8 |
| 7*^{b)}* | 0.05 | 25 | Ru(OAc)₂((*R*)-(2-Furyl)-MeOBIPHEP) | 1 | 66.5 | 80 | (-) | 95.3 |
| 8*^{b)}* | 0.05 | 25 | Ru(OAc)2((*R*)-[2,2]-PHANEPHOS | 1 | 66.5 | 80 | (-) | 84.1 |
| 9*^{b)}* | 0.05 | 25 | Ru(OAc)₂(*(R)*-BITIANP) | 1 | 66.5 | 80 | (-) | 93.4 |
| 10*^{b)}* | 0.05 | 25 | Ru(OAc)₂((+)-*(S)*-TMBTP | 1 | 66.5 | 80 | (+) | 51.1 |
| 11*^{b)}* | 0.05 | 25 | Ru(OAc)₂(*(S)*-(2-Thienyl)-MeOBIPHEP) | 1 | 66.5 | 80 | (+) | 82.3 |
| 15*^{a)}* | 0.3 | 100 | Ru(OAC)₂((*S*)-BITIANP) | 1 | 68 | 98 | (+) | 95.5 |

| | | | | | | | | |
|---|---|---|---|---|---|---|---|---|
| *^{a)}* 35 ml autoclave.*^{b)}* 6 ml autoclave. | | | | | | | | |

### Example 4 of I

### (+)-4-(3-Methoxy-phenyl)-piperidine-1,3-dicarboxylic acid-1-tert-butyl ester

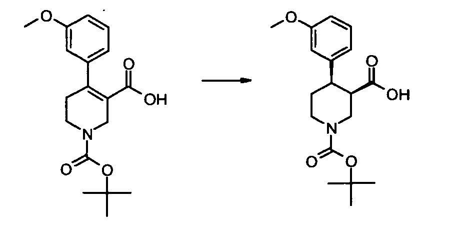

In a glove box (O₂ content ≤ 2 ppm) a 6 ml autoclave equipped with a glass insert and a magnetic stirring bar was charged with 50 mg (0.15 mmol) of 4-(3-methoxy-phenyl)-5,6-dihydro-2*H*-pyridine-1,3-dicarboxylic acid-1-*tert*-butyl ester, 7.7 mg (0.0069 mmol) of [Ru(OAc)₂(*(S)*-6-MeO-2-naphthyl)-MeOBIPHEP], 17.2 mg (0.172 mmol, 1.15 eq.) of triethylamine and 1 ml of methanol to give an orange suspension. The asymmetric hydrogenation was run for 66 h at 80°C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (+)-4-(3-methoxy-phenyl)-piperidine-1,3-dicarboxylic acid-1-*tert*-butyl ester in 80 % yield and with 96.6 % ee.

MS m/e (%): 334 (M-H⁺, 100).
[α]_{D} = +54.27 (c = 0.387, CHCl₃)

### HPLC method for ee determination:

Chiralcel-OD-H column, 25 cm*4.6 mm, 90 % n-heptane + 10 % ethanol with 1 % trifluroacetic acid, flow 1 ml/min, 30 °C, 0.002 ml injection volume, 215 nm, 266 nm. Retention times: (-)-acid 8.0 min, (+)-acid 11.0 min.

### Example 5 of I

### (+)-3-Phenyl-piperidine-1,4-dicarboxylic acid 1-tert-butyl ester and (-)-3-Phenyl-piperidine-1,4-dicarboxylic acid 1-tert-butyl ester

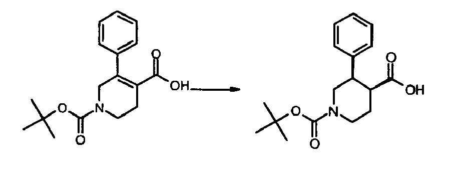

In a glove box (O₂ content ≤ 2 ppm) a 35 ml autoclave equipped with a 15 ml glass insert and a magnetic stirring bar was charged with 400 mg (1.32 mmol) of 5-phenyl-3,6-dihxdro-2*H*-pyridine-1,4-dicarboxylic acid 1-*tert*-butyl ester, 14.7 mg (0.0131 mmol) of [Ru(OAC)₂(*(S)*-6-MeO-2-naphthyl)-MeOBIPHEP], 133.1 mg (1.319 mmol, 1.0 eq.) of triethylamine and 8 ml of methanol. The asymmetric hydrogenation was run for 66 h at 80°C under 40 bar of hydrogen. After cooling to room temperature the pressure was released from the autoclave, the methanol solution was diluted with 100 ml of *tert*-butyl methyl ether and extracted with two 100-ml portions of a 1 M aqueous sodium hydroxide solution. The aqueous layer was poured on ice, acidified with ice-cold 2 M aqueous hydrochloric acid solution to pH 1 and extracted with three 150-ml portions of ethyl acetate. The combined organic layers were dried over sodium sulfate, filtered and concentrated in vacuo to give (+)-3-phenyl-piperidine-1,4-dicarboxylic acid 1-*tert*-butyl ester in 100 % yield and with 98.0 % ee.

MS m/e (%): 304 (M-H⁺, 100).
[a]_{D} = +67.17 (c = 0.636, CHCl₃)

### HPLC method for ee determination:

Chiralpak-IA column, 25 cm*4.6 mm, 50 % n-heptane + 50 % (90% n-heptane + 10 % ethanol + 0.1 % trifluroacetic acid), flow 0.8 ml/min, 25°C, 0.002 ml injection volume, 215 nm. Retention times: (+)-acid 11.8 min, (-)-acid 12.8 min.

In a similar manner, but in a 6 ml or 35 ml autoclave, the reactions in Table 5 were performed.

**Table 5:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1*^{a)}* | 0.05 | 25 | Ru(OAc)₂((rac)-BIPHEMP) | 1 | 67 | 99 | racemate | -- |
| 2*^{a)}* | 0.05 | 25 | Ru(OAc)₂(*(S)*3,5-Xyl,4-MeO-MeOBIPHEP) | 1 | 48 | 100 | (+) | 98.8 |
| 3*^{a)}* | 0.05 | 25 | Ru(OAc)₂(*(S)*-(6-MeO-2-Naphtyl)-MeOBIPHEP) | 1 | 48 | 100 | (+) | 99.1 |
| 4*^{a)}* | 0.05 | 25 | Ru(OAc)₂((*S*)-BITIANP) | 1 | 48 | 100 | (+) | 98.0 |
| 5*^{b)}* | 0.4 | 100 | Ru(OAc)₂((*R*)-BITIANP) | 1 | 66 | 100 | (-)*^{c)}* | 97.2 |

| | | | | | | | | |
|---|---|---|---|---|---|---|---|---|
| *^{a)}* 6 ml autoclave.*^{b)}* 35 ml autoclave.*^{c)}* [α]_{D} = -65.19 (c = 0.515, CHCl₃). | | | | | | | | |

### Example 6 of I

### (+)-4-Phenyl-piperidine-1,3-dicarboxylic acid 1-tert butyl ester and (-)-4-Phenyl-piperidine-1,3-dicarboxylic acid 1-tert butyl ester

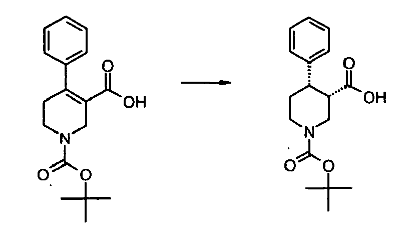

In a glove box (O₂ content ≤ 2 ppm) a 35 ml autoclave equipped with a 15 ml glass insert and a magnetic stirring bar was charged with 0.300 g (0.989 mmol) of 4-phenyl-5,6-dihydro-2*H*-pyridine-1,3-dicarboxylic acid-1-*tert*-butyl ester, 3.01 mg (0.00396 mmol) of [Ru(OAc)₂((*R*)-2-Furyl)-MeOBIPHEP], 99 mg (0.989 mmol, 1 eq.) of triethylamine and 6 ml of methanol. The asymmetric hydrogenation was run for 68 h at 80°C under 40 bar of hydrogen. After cooling to room temperature the pressure was released from the autoclave, the methanol solution was diluted with 50 ml of *tert*-butyl methyl ether and extracted with two 50-ml portions of a 1 M aqueous sodium hydroxide solution. The aqueous layer was poured on ice, acidified with ice-cold 2 M aqueous hydrochloric acid solution to pH 1 and extracted with two 100-ml portions of ethyl acetate. The combined organic layers were dried over sodium sulfate, filtered and concentrated in vacuo to give (-)-(4-phenyl)-piperidine-1,3-dicarboxylic acid-1-*tert*-butyl ester in 93 % yield (0.28 g) and with 97.3 % ee.

MS m/e (%): 306 (M+H⁺, 100%).
[a]_{D}=-59.80 (c = 0.351, CHCl₃)

### GC method for ee determination:

A 2-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethyl acetate. BGB-172 column, 30 m*0.25 mm*df 0.25 µm, hydrogen 150 kPa, split ratio 1 : 20; temperature gradient 180 - 230 °C, program with 2 °C/min; injector temperature 210 °C, detector temperature 240 °C. Retention times: 19.90 min (methyl ester of (+)-acid), 20.23 min (methyl ester of (-)-acid).

In a similar manner, but with different chiral complexes, bases or solvents, the reactions in Table 6 were performed (all in 35 ml autoclaves).

**Table 6:**

| Reaction No. | Scale (g) | S/C | Catalyst | Solvent | Base 1 equiv. | t (h) | Yield (%) | Major enantiomer | e.e. (%) |
|---|---|---|---|---|---|---|---|---|---|
| 1 | 0.1 | 25 | RU(OAc)₂((*S*)-(6-MeO-2-Naphtyl)-MeOBIPHEP) | MeOH | NEt₃ | 67 | 89 | (+) | 95.9 |
| 2 | 0.1 | 25 | RU(OAc)₂((*S*)-3,5-Xyl,4-MeO-MeOBIPHEP) | MeOH | NEt₃ | 68 | 75 | (+) | 95.9 |
| 3 | 0.1 | 25 | Ru(OAc)₂((*S*)-(BITIANP) | MeOH | NEt₃ | 68 | 88 | (+) | 96.5 |
| 4 | 0.2 | 250 | [Ru(OAc)₂((*R*)-2-Furyl)-MEOBIPHEP] | MeOH | NEt₃ | 24 | 78 | (-) | 96.7 |
| 5 | 0.05 | 25 | Ru(OAc)₂((*S*)-(6-MeO-2-Naphtyl)-MeOBIPHEP) | MeOH | None | 68 | 40 | (+) | 94.3 |
| 6 | 0.05 | 25 | Ru(OAc)₂((*S*)-(6-MeO-2-Naphtyl)-MeOBIPHEP) | MeOH | Cs₂CO₃ | 68 | 98 | (+) | 96.6 |
| 7 | 0.05 | 25 | Ru(OAc)₂((*S*)-(6-MeO-2-Naphtyl)-MeOBIPHEP) | MeOH | NHEt₂ | 68 | 91 | (+) | 96.1 |
| 8 | 0.05 | 25 | Ru(OAc)₂((*S*)-(6-MeO-2-Naphtyl)-MeOBIPHEP) | MeOH | NaOEt | 68 | 80 | (+) | 96.2 |
| 9 | 0.05 | 25 | Ru(OAc)₂((*S*)-(6-MeO-2-Naphtyl)-MeOBIPHEP) | MeOH | NaCH(= O)H | 46 | 86 | (+) | 96.1 |
| 10 | 0.05 | 25 | Ru(OAC)₂((*S*)-(6-MeO-2-Naphtyl)-MeOBIPHEP) | CH₂Cl₂ | NEt₃ | 65 | 73 | (+) | 91.6 |
| 11 | 0.05 | 25 | Ru(OAc)₂((*S*)-(6-MeO-2-Naphtyl)-MeOBIPHEP) | AcOEt | NEt₃ | 65 | 80 | (+) | 89.0 |
| 12 | 0.05 | 25 | Ru(OAc)₂((*S*)-(6-MeO-2-Naphtyl)-MeOBIPHEP) | THF | NEt₃ | 65 | 78 | (+) | 79.6 |
| 13 | 0.05 | 25 | Ru(OAc)₂((*S*)-(6-MeO-2-Naphtyl)-MeOBIPHEP) | TFE | NEt₃ | 46 | 90 | (+) | 94.8 |
| 14 | 0.05 | 25 | Ru(OAC)₂((*S*)-(6-MeO-2-Naphtyl)-MeOBIPHEP) | MeOH/ H₂O (9:1) | NEt₃ | 46 | 98 | (+) | 96.5 |

In a similar manner, but at different temperatures, different reaction times and under various pressure of hydrogen, the reactions in Table 6.1 were performed. Scale: 50 mg, S/C = 25

**Table 6.1:**

| Reaction No. | Catalyst | Solvent | Base 1 equiv. | t (h) | T (°C) | p (bar) | Yield (%) | Major enantiomer | e.e. (%) |
|---|---|---|---|---|---|---|---|---|---|
| 1^{a} | Ru(OAc)₂((*S*)-(6-MeO-2-Naphtyl)-MeOBIPHEP) | MeOH | NEt₃ | 64 | 60 | 40 | 88 | (+) | 96.9 |
| 2^{a} | Ru(OAc)₂((*S*)-(6-MeO-2-Naphtyl)-MeOBIPHEP) | MeOH | NEt₃ | 48 | 50 | 50 | >99 | (+) | 97.1 |
| 3^{a} | Ru(OAc)₂(*S*)-(6-MeO-2-Naphtyl)-MeOBIPHEP) | MeOH | NEt₃ | 44 | 40 | 40 | 82 | (+) | 96.9 |
| 4^{a} | Ru(OAc)₂((*S*)-(6-MeO-2-Naphtyl)-MeOBIPHEP) | MeOH | NEt₃ | 70 | Rt (24-26°C) | 40 | 76 | (+) | 98.4 |
| 5^{a} | Ru(OAc)₂((R) -(2-Furyl)-MeOBIPHEP) | MeOH | NEt₃ | 24 | 80 | 40 | 78 | (-) | 96.7 |
| 6^{a,b} | Ru(OAc)₂((R) -(2-Furyl)-MeOBIPHEP) | MeOH | NEt₃ | 68 | 80 | 40 | 94 | (-) | 97.5 |

| | | | | | | | | | |
|---|---|---|---|---|---|---|---|---|---|
| ^{a} 35 ml autoclave, ^{b} Technical MeOH and NEt₃, autoclave loaded under air. | | | | | | | | | |

### Example 7 of I

### (+)-4-Phenyl-piperidine-1,3-dicarboxylic acid 1-tert butyl ester and (-)-4-Phenyl-piperidine-1,3-dicarboxylic acid 1-tert butyl ester

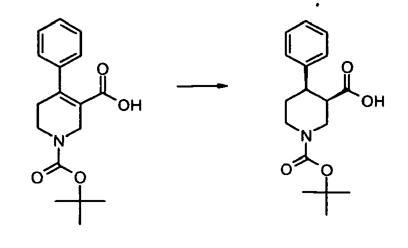

In a glove box (O₂ content ≤ 2 ppm) a 6 ml autoclave equipped with a glass insert and a magnetic stirring bar was charged with 2.16 mg (0.0066 mmol) [Ru(OAc)₂(COD)], 6.71 mg (*R*_{C},*S*_{pl,}*S*_{P})*-* TOLFER Stylacat 4/2 (0.00725 mmol) and methanol (1 ml). The corresponding catalyst solution was heated at 60°C overnight (18 h in total), cooled to ambient temperature and charged with 0.05 g (0.165 mmol) 4-phenyl-5,6-dihydro-2*H-*pyridine-1,3-dicarboxylic acid-1-*tert*-butyl ester and 16.7 mg (0.165 mmol, 1 equiv.) of triethylamine. The asymmetric hydrogenation was run for 66 h at 80°C under 40 bar of hydrogen. After cooling to room temperature the pressure was released from the autoclave, the methanol solution was diluted with 30 ml of *tert*-butyl methyl ether and extracted with two 30-ml portions of a 1M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (+)-(4-phenyl)-piperidine-1,3-dicarboxylic acid-1-*tert*-butyl ester in 91 % yield (0.046 g) and with 97.3 % ee.

MS m/e (%): 306 (M+H⁺, 100%).

### GC method for ee determination:

A 2-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethyl acetate. BGB-172 column, 30 m*0.25 mm*df 0.25 µm, hydrogen 150 kPa, split ratio 1 : 20; temperature gradient 180 - 230 °C, program with 2 °C/min; injector temperature 210 °C, detector temperature 240 °C. Retention times: 19.90 min (methyl ester of (+)-acid), 20.23 min (methyl ester of (-)-acid).

In analogy to the above described experiment, but with different chiral ligands, the reactions in Table 7 were performed.

**Table 7:**

| Reaction No. | S/C | Ruthenium Precursor | Chiral Ligand | t (h) | Yield (%) | Major enantio mer | e.e. (%) |
|---|---|---|---|---|---|---|---|
| 1 | 25 | Ru(OAc)₂(COD) | (1*R*,1'*R*,2*S*,2'*S*)-DuanPhos | 68 | 99 | (-) | 82.6 |
| 2 | 25 | Ru(OAc)₂(COD) | (*S*_{C},*R*ₚₗ,*R*_{P})-Stylacat 4/1 | 68 | 92 | (+) | 92.4 |
| 3 | 25 | Ru(OAc)₂(COD) | (*S*_{C},*R*pₗ,*R*_{P})-Stylacat 3/1/1 | 68 | 91 | (-) | 35.9 |
| 4 | 25 | Ru(OAc)₂(COD) | (*S*)_f BINAPHANE | 68 | 99 | (+) | 41.4 |
| 5 | 25 | Ru(OAc)₂(COD) | (*S*,*S*)-DIOP | 68 | 99 | (+) | 26.2 |
| 6 | 25 | Ru(OAc)₂(COD) | (R)-C2-Tunaphos | 68 | 99 | (-) | 93.1 |

### Example 8 of I

### (-)-2-Phenyl-cyclohexane carboxylic acid and (+)-2-Phenyl-cyclohexane carboxylic acid

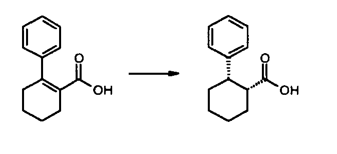

In a glove box (O₂ content < 2 ppm) a 6 ml autoclave equipped with a glass insert and a magnetic stirring bar was charged with 50 mg (0.25 mmol) of 2-phenyl-cyclohex-1-ene-carboxylic acid, 11.1 mg (0.00989 mmol) of [Ru(OAc)₂((R)-2-furyl)-MeOBIPHEP], 24.9 mg (0.247 mmol, 1.0 eq.) of triethylamine and 1 ml of methanol. The asymmetric hydrogenation was run for 42 h at 80 °C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (-)-2-phenyl-cyclohexane carboxylic acid in 100 % yield and with 95.1 % ee.

MS m/e (%): 203 (M-H⁺, 100).
[α]_{D} = -76.42 (c = 0.254, CHCl₃)

### HPLC method for ee determination:

Chiralpak-IA column, 25 cm*4.6 mm, 95 % n-heptane + 5 % isopropanol with 1 % trifluroacetic acid, flow 0.8 ml/min, 20 °C, 0.002 ml injection volume, 215 nm. Retention times: (+)-acid 7.6 min, (-)-acid 8.2 min.

The reactions in Table 8 were performed according to the procedure above.

**Table 8:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1 | 0.05 | 25 | Ru(OAc)₂((rac)-BIPHEMP) | 1 | 42 | 79 | racemate | -- |
| 2 | 0.05 | 25 | Ru(OAc)₂((*S*)-3,5-Xyl,4-MeO-MeOBIPHEP) | 1 | 42 | 100 | (+) | 90.8 |
| 3 | 0.05 | 25 | Ru(OAc)₂((*S*)-(6-MeO-2-Naphtyl)-MeOBIPHEP) | 1 | 42 | 100 | (+) | 90.4 |

### Example 9 of I

### (-)-2-Phenyl-cyclopentanecarboxylic acid and (+)-2-Phenyl-cyclopentanecarboxylic acid

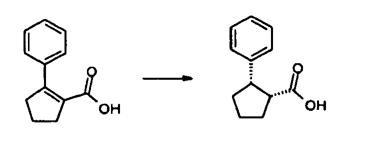

In a glove box (O₂ content ≤ 2 ppm) a 6 ml autoclave equipped with a glass insert and a magnetic stirring bar was charged with 50 mg (0.27 mmol) of 2-phenyl-cyclopent-1-enecarboxylic acid, 8.1 mg (0.011 mmol) of [Ru(OAc)₂((*R*)-(2-furyl)-MeOBIPHEP], 26.8 mg (0.266 mmol, 1.0 eq.) of triethylamine and 1 ml of methanol. The asymmetric hydrogenation was run for 68 h at 80 °C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two
50-ml portions of ethyl acetate. The combined organic layers were dried over sodium sulfate, filtered and concentrated in vacuo to give (-)-2-phenyl-cyclopentanecarboxylic acid in 98 % yield and with 97.1 % ee.

MS m/e (%): 189 (M-H⁺, 100).
[α]_{D} = -85.22 (c = 0.277, CHCl₃)

### HPLC method for ee determination:

Chiralpak-IA column, 25 cm*4.6 mm, 93 % n-heptane + 7 % isopropanol with 1 % trifluroacetic acid, flow 0.8 ml/min, 20 °C, 0.002 ml injection volume, 215 nm. Retention times: (+)-acid 7.2 min, (-)-acid 7.8 min.

The reactions in Table 9 were performed according to the procedure above.

**Table 9:**

| Reaction No. | Scale (g) | S/C | Catalyst | Net₃ (equiv.) | t (h) | Yield (%) | Major enantiomer | e.e. (%) |
|---|---|---|---|---|---|---|---|---|
| 1 | 0.05 | 25 | Ru(OAc)₂((rac)-BIPHEMP) | 1 | 66 | 89 | racemate | -- |
| 2 | 0.05 | 25 | Ru(OAc)₂((*S*)3,5-Xyl,4-MeO-MeOBIPHEP) | 1 | 68 | 96 | (+) | 78.6 |
| 3 | 0.05 | 25 | Ru(OAc)₂((*S*)-(6-MeO-2-Naphtyl)-MeOBIPHEP) | 1 | 68 | 98 | (+) | 79.3 |

### Example 10 of I

### (+)-(3R,4R)-4-(Phenyl)-pyrrolidine-1,3-di carboxylic acid-1-tert-butyl ester and (-)-(3S,4S)-4-(Phenyl)-pyrrolidine-1,3-di carboxylic acid-1-tert-butyl ester

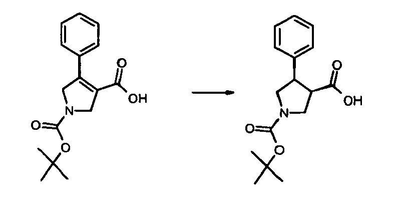

In a glove box (O₂ content ≤ 2 ppm) a 185 ml autoclave equipped with a mechanical stirrer was charged with 4.46 g (5.4 mmol) of 4-(phenyl)-2,5-dihydro-pyrrole-1,3-dicarboxylic acid-1-*tert*-butyl ester, 173 mg (0.154 mmol) of [Ru(OAc)₂((*S*)-6-MeO-2-naphthyl)-MeOBIPHEP], 771 mg (7.62 mmol, 0.5 eq.) of triethylamine and 50 ml of methanol. The asymmetric hydrogenation was run for 48 h at 80 °C under 40 bar of hydrogen. After cooling to room temperature the pressure was released from the autoclave, the methanol solution was diluted with 200 ml of *tert*-butyl methyl ether and extracted with two 200-ml portions of a 1M aqueous sodium hydroxide solution. The aqueous layer was poured on ice, acidified with ice-cold 2 M aqueous hydrochloric acid solution to pH 1 and extracted with three 300-ml portions of ethyl acetate. The combined organic layers were dried over sodium sulfate, filtered and concentrated in vacuo to give 3.95 g (88 %) (+)-(3R,4R)-4-(phenyl)-pyrrolidine-1,3-di carboxylic acid-1-*tert*-butyl ester with 90.5 % ee. Crystallization from cyclohexane/ethyl acetate 9:1 gave 2.80 g (+)-(3R,4R)-4-(phenyl)-pyrrolidine-1,3-di carboxylic acid-1-*tert*-butyl ester with 98.4 % ee.

MS m/e (%): 290 (M-H⁺, 100).
[α]_{D}=+51.71 (c = 0.700, CHCl₃)

### HPLC method for ee determination:

A 1-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethanol. Chiralpak-ADH column, 25 cm*4.6 mm, 93 % n-heptane + 7 % ethanol, flow 0.7 ml/min, 25 °C, 0.005 ml injection volume, 210 nm. Retention times: 11.3 min (methyl ester of (-)-acid), 14.6 min (methyl ester of (+)-acid).

### Assignment of the absolute configuration

To a solution of (+)-(3R,4R)-4-(phenyl)-pyrrolidine-1,3-di carboxylic acid-1-*tert*-butyl ester (300 mg, 1.03 mmol, 98.4 % ee) and triethylamine (167 mg, 1.65 mmol) in 10 ml tetrahydrofuran was added isobutyl chloroformate (211 mg, 1.54 mmol) at -10°C. After 30 minutes a solution of 2-mercaptopyridine N-oxide (275 mg, 2.16 mmol) and triethylamine (223 mg, 2.20 mmol) in 6 ml tetrahydrofuran was added. After completed addition the reaction mixture was warmed to room temperature and stirred for 3 h in the dark. After filtration and washing with 15 ml tetrahydrofuran 2-methyl-1-propanethiol (1.02 g, 11.3 mmol) the mixture was stirred under irradiation with a high-pressure mercury lamp for 20 h. After quenching with 2 M aqueous sodium hydroxide solution the mixture was extracted with three portions of *tert*-butyl methyl ether. The combined organic extracts were washed with brine, dried over sodium sulfate and concentrated in vacuo. The residue was purified by Kugelrohr distillation in high vacuo to give 206 mg (81 %) (R)-3-phenyl-pyrrolidine-1-carboxylic acid *tert*-butyl ester.

MS m/e (%): 248 (M+H⁺, 10).
[α]_{D} = +13.52 (c = 0.192, dichloromethane)
Lit.: A. I. Meyers, L. Snyder, J. Org. Chem. 1993, 58, 36. [α]_{D} = +10.3 (c = 1.03, dichloromethane)

A solution of (R)-3-phenyl-pyrrolidine-1-carboxylic acid *tert*-butyl ester (140 mg, 0.566 mmol) in 4.5 ml of a 1.25 M solution of hydrochloric acid in methanol was stirred at 40 °C for 2h. After evaporation of the solvent the residue was dissolved in a mixture of *tert-*butyl methyl ether and 2 M aqueous sodium hydroxide solution. The mixture was extracted with three portions of *tert*-butyl methyl ether. The combined organic extracts were dried over sodium sulfate and concentrated in vacuo. The residue was purified by Kugelrohr distillation in high vacuo to give 51 mg (61 %) of (R)-3-phenyl-pyrrolidine.

MS m/e (%): 148 (M+H⁺, 100).
[α]_{D} = -22.32 (c = 0.408, EtOH)
Lit.: C.C. Tseng et al. Chem. Pharm. Bull. 1977, 25, 166. For the (S) enantiomer
[α]_{D} = +22.7 (c = 2.36, EtOH)
In a similar manner, but in a 6 ml or 35 ml autoclave, the reactions in Table 10 were performed.

**Table 10:**

| Reaction No. | *S*cale (g) | S/C | Catalyst | Et₃N (equiv.) | t (h) | Yield (%) | Major enantiomer | e.e. (%) |
|---|---|---|---|---|---|---|---|---|
| 1^{a)} | 0.2 | 25 | Ru(OAc)₂((*R*)-MeOBIPHEP) + 0.86 toluene | 0.5 | 42 | 41 | (-) | 68 |
| 2^{b)} | 0.05 | 25 | Ru(OAc)₂((*R*)-MeOBIPHEP) + 0.86 toluene | 0.6 | 42 | 91 | (-) | 84.3 |
| 3^{b)} | 0.05 | 25 | Ru(OAc)₂((*S*)-(6-MeO-2-Naphtyl)-MeOBIPHEP) | 0.6 | 42 | 99 | (+) | 94.9 |
| 4^{b)} | 0.05 | 25 | Ru(OAc)₂((*R*)-3,5-tBu-MeOBIPHEP) | 0.6 | 42 | 95 | (-) | 41.8 |
| 5 ^{b)} | 0.05 | 25 | Ru(OAc)₂((+)-(*S*)-TMBTP | 0.6 | 42 | > 90 | (+) | 76.8 |
| 6 ^{b)} | 0.05 | 25 | Ru(OAc)₂((*S*)-3,5-Xyl,4-MeO-MeOBIPHEP) | 0.6 | 42 | > 90 | (+) | 94.0 |
| 7^{b)} | 0.05 | 25 | Ru(OAc)₂((all-*S*)-BICP) | 0.6 | 42 | 89 | (+) | 71.5 |

| | | | | | | | | |
|---|---|---|---|---|---|---|---|---|
| ^{a)} 35 ml autoclave. ^{b)} 6 ml autoclave. | | | | | | | | |

### Example 11 of I

### (-)-4-(4-Chloro-phenyl)-pyrrolidine-1,3-di carboxylic acid-1-tert-butyl ester and (+)-4-(4-Chloro-phenyl)-pyrrolidine-1,3-di carboxylic acid-1-tert-butyl ester

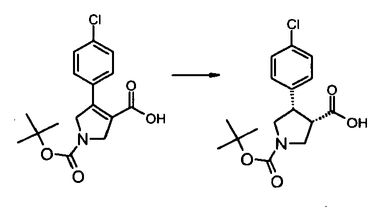

In a glove box (O₂ content ≤ 2 ppm) a 6 ml autoclave equipped with a glass insert and a magnetic stirring bar was charged with 50 mg (0.154 mmol) of 4-(4-chloro-phenyl)-2,5-dihydro-pyrrole-1,3-dicarboxylic acid-1-*tert*-butyl ester, 4.7 mg (0.0062 mmol) of [Ru(OAc)₂((*R*)-2-furyl)-MeOBIPHEP], 15.4 mg (0.154 mmol, 1.0 eq.) of triethylamine and 1 ml of methanol. The asymmetric hydrogenation was run for 42 h at 80 °C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (-)-4-(4-chloro-phenyl)-pyrrolidine-1,3-di carboxylic acid-1-*tert*-butyl ester in 80 % yield and with 98.3 % ee.

MS m/e (%): 324 (M-H⁺, 100).
[α]_{D} = -50.37 (c = 0.326, CHCl₃)

### HPLC method for ee determination:

Chiralpak-ADH column, 25 cm*4.6 mm, 85 % n-heptane + 15 % ethanol with 0.5 % trifluoroacetic acid, flow 0.7 ml/min, 20 °C, 0.002 ml injection volume, 215 nm.
Retention times: (+)-acid 10.6 min, (-)-acid 11.8 min.

The reactions in Table 11 were performed according to the procedure above.

**Table 11:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1 | 0.05 | 25 | Ru(OAc)₂(*S*)-(6-MeO-2-Naphtyl)-MeOBIPHEP) | 1 | 42 | 100 | (+) | 88.3 |
| 2 | 0.05 | 25 | Ru(OAc)₂,((*S*)3,5-Xyl,4-MeO-MeOBIPHEP) | 1 | 42 | 80 | (+) | 85.7 |

### Example 12 of I

### (+)-4-(3-Fluoro-phenyl)-pyrrolidine-1,3-dicarboxylic acid-1-tert-butyl ester and (-)-4-(3-Fluoro-phenyl)-pyrrolidine-1,3-di carboxylic acid-1-tert-butyl ester

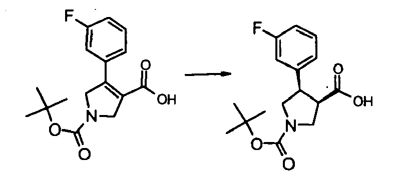

In a glove box (O₂ content ≤ 2 ppm) a 6 ml autoclave equipped with a glass insert and a magnetic stirring bar was charged with 50 mg (0.16 mmol) of 4-(3-fluoro-phenyl)-2,5-dihydro-pyrrole-1,3-dicarboxylic acid-1-*tert*-butyl ester, 7.4 mg (0.0065 mmol) of [Ru(Oac)₂((*S*)-6-MeO-2-naphthyl)-MeOBIPHEP], 16.4 mg (0.163 mmol, 1.0 eq.) of triethylamine and 1 ml of methanol. The asymmetric hydrogenation was run for 42 h at 80°C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium_sulphate, filtered and concentrated in vacuo to give (+)-4-(3-fluoro-phenyl)-pyrrolidine-1,3-di carboxylic acid-1-*tert*-butyl ester in 77 % yield and with 87.1 % ee.

MS m/e (%): 308 (M-H⁺, 100).

### HPLC method for ee determination:

Chiralpak-ADH column, 25 cm*4.6 mm, 85 % n-heptane + 15 % ethanol with 0.5 % trifluoroacetic acid, flow 0.7 ml/min, 20 °C, 0.002 ml injection volume, 215 nm.
Retention times: (-)-acid 9.3 min, (+)-acid 11.2 min.

The reaction in Table 12 was performed according to the procedure above.

**Table 12:**

| Reaction No. | Scale (g) | S/C | Catalyst | NEt₃ (equiv.) | t (h) | Yield (%) | Major enantiomer | e.e. (%) |
|---|---|---|---|---|---|---|---|---|
| 1 | 0.025 | 25 | Ru(OAc)₂((*R*)-(2-Furyl)-MeOBIPHEP | 1 | 42 | 100 | (-)^{a)} | 98.1 |

| | | | | | | | | |
|---|---|---|---|---|---|---|---|---|
| ^{a)} [α]_{D} = -46.03 (c = 0.341, CHCl₃). | | | | | | | | |

### Example 13 of I

### (3R,4R)-1-Benzyl-4-phenyl-pyrrolidine-3-carboxylic acid and (3RS,4RS)-1-benzyl-4-phenyl-pyrrolidine-3-carboxylic acid

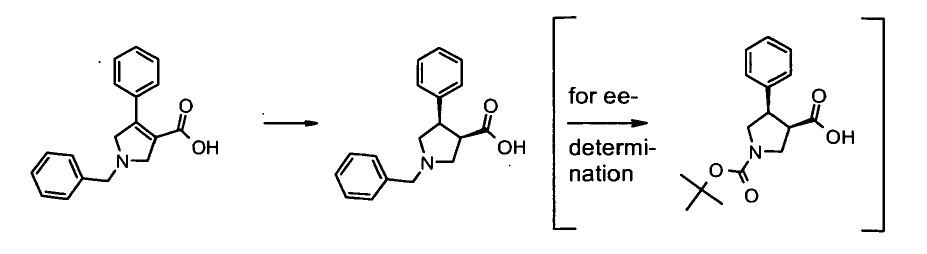

### Preparation of the racemate:

In a glove box (O₂ content ≤ 2 ppm) a 6 ml autoclave equipped with a glass insert and a magnetic stirring bar was charged with 50 mg (0.18 mmol) of 1-benzyl-4-phenyl-2,5-dihydro-1*H*-pyrrole-3-carboxylic acid, 5.5 mg (0.0072 mmol) of Ru(OAc)2((rac)-BIPHEMP), 17.9 mg (0.179 mmol, 1.0 eq.) of triethylamine and 1 ml of methanol. The racemic hydrogenation was run for 42 h at 80 °C under 40 bar of hydrogen. After cooling to room temperature the pressure was released from the autoclave. The reaction mixture was diluted with 30 ml of *tert*-butyl methyl ether and extracted with two portions of a 1 M aqueous sodium hydroxide solution. The layers were separated and the aqueous phase was poured on ice. The pH was adjusted to pH 6 using 2 M aqueous hydrochloric acid solution. After extraction with three portions of dichloromethane (3 x 50 ml) the combined organic layers were dried over sodium_sulphate, filtered and concentrated in vacuo to give (3R*S*,4R*S*)-1-benzyl-4-phenyl-pyrrolidine-3-carboxylic acid in 40 % yield (20 mg).
MS m/e (%): 280 (M-H⁺, 100).

### Enantioselective hydrogenation:

In a glove box (O₂ content ≤2 ppm) a 6 ml autoclave equipped with a glass insert and a magnetic stirring bar was charged with 50 mg (0.18 mmol) of 1-benzyl-4-phenyl-2,5-dihydro-1*H*-pyrrole-3-carboxylic acid, 8.0 mg (0.0072 mmol) of [Ru(OAc)₂((*S*)-6-MeO-2-naphthyl)-MeOBIPHEP], 17.9 mg (0.179 mmol, 1.0 eq.) of triethylamine and 1 ml of methanol. The asymmetric hydrogenation was run for 68 h at 80 °C under 40 bar of hydrogen. After cooling to room temperature the pressure was released from the autoclave and the solvent was evaporated in vacuo. The residue was redissolved in 2 ml ethanol and 0.050 ml triethylamine (0.355 mmol) and 43 mg (0.20 mmol) di-*tert*-butyl dicarbonate were added. The reaction mixture was purged with argon prior the addition of Pd / C (10 %) and then filled with hydrogen. The reaction mixture was stirred for 16 h at room temperature under hydrogen atmosphere and then filtered through Decalite. The filtrate was diluted with 30 ml of *tert*-butyl methyl ether and extracted with two portions of a 1 M aqueous sodium hydroxide solution. The layers were separated and the aqueous phase was poured on ice. The pH was adjusted to pH 1 using 2 M aqueous hydrochloric acid solution. After extraction with three portions of dichloromethane (3 x 50 ml) the combined organic layers were dried over sodium_sulphate, filtered and concentrated in vacuo to give (+)-(3R,4R)-4-(phenyl)-pyrrolidine-1,3-di carboxylic acid-1-*tert*-butyl ester in 6 % yield and with 97.4 % ee.

MS m/e (%): 290 (M-H⁺, 100).

### HPLC method for ee determination:

Chiralpak-ADH column, 25 cm*4.6 mm, 93 % n-heptane + 7 % ethanol, flow 0.7 ml/min, 25 °C, 0.003 ml injection volume, 210 nm. Retention times: 11.3 min (methyl ester of
(-)-acid), 14.6 min (methyl ester of (+)-acid).

The reactions in Table 13 were performed according to the procedure above.

**Table 13:**

| Reaction No. | Scale (g) | S/C | Catalyst | NEt₃ (equiv.) | t (h) | Yield (%) | Major enantiomer *a)* | e.e. (%)^{a)} |
|---|---|---|---|---|---|---|---|---|
| 1 | 0.05 | 25 | Ru(OAc)₂(rac)-BIPHEMP) | 1 | 42 | 40 | racemate | -- |
| 2 | 0.05 | 25 | Ru(OAc)₂(*S*)3,5-Xyl,4-MeO-MeOBIPHEP) | 1 | 68 | 23 | (+) | 97.7 |
| 3 | 0.05 | 25 | Ru(OAc)₂((*S*)-BITIANP) | 1 | 68 | 25 | (+) | 92.7 |

| | | | | | | | | |
|---|---|---|---|---|---|---|---|---|
| ') Optical rotation and ee of (+)-(3R,4R)- or (-)-(3*S*,4*S*)-4-(phenyl)-pyrrolidine-1,3-di carboxylic acid-1-*tert*-butyl ester obtained after debenzylation and *N*-tert-butoxycarbonyt protection of the primary hydrogenation product (3R,4R)- or (3*S*,4*S*)-1-benzyl-4-phenylpyrrolidine-3-carboxylic acid. | | | | | | | | |

### Example 14 of 1

### (+)-4-Phenyl-tetrahydro-thiophene-3-carboxylic acid and (-)-4-Phenyl-tetrahydro-thiophene-3-carbbacylic acid

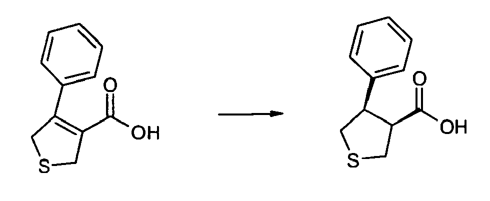

In a glove box (O₂ content ≤ 2 ppm) a 6 ml autoclave equipped with a glass insert and a magnetic stirring bar was charged with 0.050 g (0.242 mmol) of 4-phenyl-2,5-dihydrothiophene-3-carboxylic acid, 36.92 mg (0.0485 mmol) of [Ru(OAc)₂((-R)-2-Furyl)-MeOBIPHEP], 24.5 mg (0.242 mmol, 1 eq.) of triethylamine and 1 ml of methanol. The asymmetric hydrogenation was run for 70 h at 80°C under 40 bar of hydrogen. After cooling to room temperature the pressure was released from the autoclave, the methanol solution was diluted with 30 ml of *tert*-butyl methyl ether and extracted with two 30-ml portions of a 1M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (+)-4-phenyl-tetrahydro-thiophene-3-carboxylic acid in 60 % yield (0.03 g) and with 98.1% ee.
MS m/e (%): 207 (M⁺-H, 100).
[α]_{D} = +33.93 (c = 0.342, CHCl₃)

### GC method for ee determination:

A 2-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethyl acetate. BGB-172 column, 60 m*0.25 mm*df 0.25 µm, hydrogen 150 kPa, split ratio 1 : 50; temperature gradient 160 - 230 °C, program with 2 °C/min; injector temperature 210 °C, detector temperature 230 °C. Retention times: 33.11 min (methyl ester of (+)-acid), 33.57 min (methyl ester of (-)-acid).

The reactions in Table 14 were performed according to the procedure above.

**Table 14:**

| Reaction No. | Scale (g) | S/C | Catalyst | Solvent | Base (1 equiv.) | t (h) | Yield (%) | Major enantio mer | e.e. (%) |
|---|---|---|---|---|---|---|---|---|---|
| 1 | 0.05 | 5 | Ru(OAc)₂(*S*)-(6-MeO-2-Naphtyl)- MeOBIPHEP) | MeOH | NEt₃ | 68 | 99 | (-) | 73.0 |
| 2 | 0.05 | 5 | Ru(OAc)₂((*S*) (BITIANP) | MeOH | NEt₃ | 68 | 58 | (-) | 74.5 |

### Example 15 of I

### (-)-2-Phenyl-cyclooctanecarboxylic acid

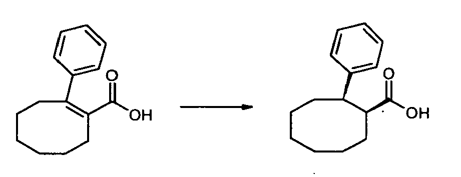

In a glove box (O₂ content ≤ 2 ppm) a 35 ml autoclave equipped with a 15 ml glass insert and a magnetic stirring bar was charged with 0.050 g (0.217 mmol) of 2-phenyl-cyclooct-1-ene-carboxylic acid, 9.31 mg (0.00868 mmol) of [Ru((*S*)-MeOBIPHEP)(pCym)I]I,2.2 mg (0.0217 mmol, 0.1 eq.) of triethylamine and 1 ml of methanol. The asymmetric hydrogenation was run for 42 h at 80°C under 40 bar of hydrogen. After cooling to room temperature the pressure was released from the autoclave, the methanol solution was diluted with 30 ml of *tert*-butyl methyl ether and extracted with two 30-ml portions of a 1 M aqueous sodium hydroxide solution. The aqueous layer was poured on ice, acidified with ice-cold 2 M aqueous hydrochloric acid solution to pH 1 and extracted with two 50-ml portions of ethyl acetate. The combined organic layers were dried over sodium sulfate, filtered and concentrated in vacuo to give (-)-(2-phenyl)-cyclooctanecarboxylic acid in 76 % yield (0.036 g) and with 45.9 % ee.
MS m/e (%): 231 (M-H⁺, 100).
[α]_{D} = -3.97 (c= 0.504, CHCl₃)

### GC method for ee determination:

A 2-mg sample of the title compound was converted to the methyl ester by treatment with 0.5 ml of an approximately 0.5 M solution of diazomethane in diethyl ether at room temperature. After evaporation of excess diazomethane and diethyl ether under a gentle stream of argon the residue was dissolved in 1 ml of ethyl acetate. BGB-172 column, 60 m*0.25 mm*df 0.25 µm, hydrogen 150 kPa, split ratio 1 : 50; temperature gradient 160 - 230 °C, program with 2 °C/min; injector temperature 210 °C, detector temperature 230 °C. Retention times: 32.66 min (methyl ester of (+)-acid), 32.85 min (methyl ester of (-)-acid).

In a similar manner, the reactions in Table 15 were performed.

**Table 15:**

| Reactio n No. | Scale (g) | S/C | Catalyst | Solvent | Base (1 equiv.) | t (h) | Conv. (%) (isol. Yield (%)) | Major enantio mer | e.e. (%) |
|---|---|---|---|---|---|---|---|---|---|
| 1 | 0.05 | 25 | Ru(OAc)₂((*S*)-(6-MeO-2-Naphtyl)-MeOBIPHEP) | MeOH | NEt₃ | 67 | >99 (80) | (-) | 34.6 |
| 2 | 0.05 | 25 | Ru(OAc)₂((*S*)-pTol-MeOBIPHEP | MeOH | NEt₃ | 67 | > 99 (92) | (-) | 38.6 |
| 3 | 0.05 | 25 | [Ru((*S*)-MeOBIPHEP)(pC ym)I]I | MeOH | none | 67 | 55 (n.d.)^{a} | (-) | 81.4 |
| 4 | 0.05 | 6 | [Ru((*S*)-3,5-tBu-MeOBIPHEP)(C6 H6)Cl] BF4 | MeOH | none | 20 | 17 (n.d.)^{a} | (-) | 95.8 |

| | | | | | | | | | |
|---|---|---|---|---|---|---|---|---|---|
| *^{a}*Yield not determined | | | | | | | | | |

### Synthesis of cyclic β-aryl substituted α,β-unsaturated carboxylic acids II as starting materials for the enantioselective hydrogenations:

### Example 1 of II

### 4-(4-Fluoro-phenyl)-5,6-dihydro-2H-pyridine-1,3-dicarboxylic acid 1-tert-butyl ester

### a) 4-Trifluoromethanesulfonyloxy-5,6-dihydro-2H-pyridine-1,3-dicarboxylic acid 1-tert-butyl ester 3-methyl ester

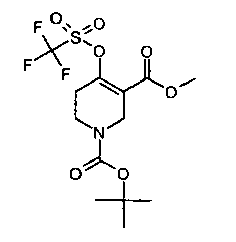

To a solution of 4-oxo-piperidine-1,3-dicarboxylic acid 1-*tert*-butyl ester 3-methyl ester (8.64 g, 33.5 mmol) in 230 ml THF was added sodium hydride (suspension in oil, 55 %, 3.26 g, 74.6 mmol) at 0 °C. After stirring for 30 min. at 0 °C N-phenyltrifluoromethanesulfonimide (20.4 g , 56.0 mmol) was added. The ice-water bath was removed and the reaction mixture was stirred for 2 days. Quenching with ice was followed by concentration in vacuo to remove THF. The residue was diluted with *tert*-butyl methyl ether and washed with three portions of 1 M aqueous sodium hydroxide solution. The organic layer was washed with brine and dried over sodium sulfate. Concentration in vacuo gave the crude title compound with a purity of 90 % (11.4 g, 26.4 mmol, 71 %).
MS m/e (%): 334 (M+H⁺-C₄H₈, 100).

### b) 4-(4-Fluoro-phenyl)-5,6-dihydro-2H-pyridine-1,3-dicarboxylic acid 1-tert-butyl ester 3-methyl ester

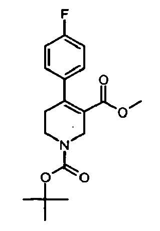

To a mixture of 4-trifluoromethanesulfonyloxy-5,6-dihydro-2*H*-pyridine-1,3-dicarboxylic acid 1-*tert*-butyl ester 3-methyl ester (10.1 g, 25.9 mmol), 4-fluorophenylzinc bromide solution (0.5 M in THF, 86.3 ml, 43.1 mmol) and 290 ml THF was added tetrakis(triphenylphosphine)palladium(0) (0.83 g, 0.72 mmol) at RT. After stirring for 6 h the reaction was quenched with ice. The mixture was diluted with *tert-*butyl methyl ether and washed with 2 M aqueous sodium carbonate solution. The aqueous layer was extracted with two portions of *tert*-butyl methyl ether. The combined organic layers were washed with brine, dried over sodium sulfate and concentrated in vacuo. Purification of the residue by flash chromatography (heptane / ethyl) gave the title compound as a lightly yellow amorphous residue (6.8 g, 71 %).
MS m/e (%): 336 (M+H⁺, 10).

### c) 4-(4-Fluoro-phenyl)-5,6-dihydro-2H-pyridine-1,3-dicarboxylic acid 1-tert-butyl ester

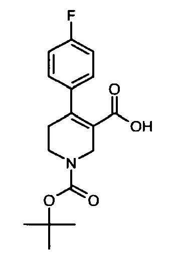

A mixture of 4-(4-fluoro-phenyl)-5,6-dihydro-2*H*-pyridine-1,3-dicarboxylic acid 1*-tert-*butyl ester 3-methyl ester (6.8 g, 20 mmol), 100 ml 1,4-dioxane and 100 ml 2 M NaOH was stirred at RT for 20 h. After extraction of the reaction mixture with two portions of *tert*-butyl methyl ether, the combined organic layers were extracted with 1 M aqueous sodium hydroxide solution (100 ml). The combined aqueous layers were cooled to 0 °C by addition of ice (150 g) and acidified to pH 1 with ice-cold 4 M aqueous hydrochloric acid solution (70 ml). The aqueous layer was extracted with three 150 ml-portions of ethyl acetate. The combined organic layers were washed with brine (50 ml), dried over sodium sulfate and concentrated in vacuo. Crystallization of the crude acid (6.4 g) from a mixture of n-heptane and ethyl acetate (19: 1, 120 ml) gave the title compound as white crystals (5.1 g, 78 %).
MS m/e (%): 320 (M-H⁺, 100).

### Example 2 of II

### 4-(1H-Indol-3-yl)-5,6-dihydro-2H-pyridine-1,3-dicarboxylic acid 1-tert-butyl ester

### a) 4-[1-(tert-Butyl-dimethyl-silanyl)-1H-indol-3-yl]-5,6-dihydro-2H-pyridine-1,3-dicarboxylic acid 1-tert-butyl ester 3-methyl ester

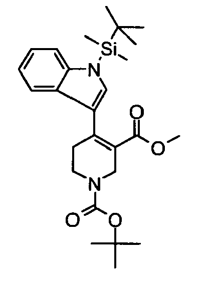

To a solution of 3-bromo-1-(*tert*-butyl-dimethyl-silanyl)-1*H*-indole (23.0 g, 74.1 mmol) in dry THF (280 ml) was added dropwise at -78 °C a solution of *tert*-butyllithium in pentane (1.7 M, 87.2 ml, 148 mmol). To the resulting orange solution was added dropwise a freshly prepare solution of dried zink chloride (11.1 g, 81.5 mmol) in dry THF (110 ml) at -78 °C. After completed addition the reaction mixture was allowed to slowly warm to room temperature over a period of 1.5 h. To this mixture were added a solution of 4-trifluoromethanesulfonyloxy-5,6-dihydro-2*H*-pyridine-1,3-dicarboxylic acid 1*-tert-*butyl ester 3-methyl ester (19.6 g, 50.3 mmol) in THF (130 ml) and tetrakis(triphenylphosphine)palladium(0) (1.75 g, 1.51 mmol). After stirring for 64 h at room temperature the reaction was quenched with ice. The mixture was diluted with *tert-*butyl methyl ether and washed with 2 M aqueous sodium carbonate solution. The aqueous layer was extracted with two portions of *tert*-butyl methyl ether. The combined organic layers were washed with water and brine, dried over sodium sulfate and concentrated in vacuo. Purification of the residue by flash chromatography (heptane / ethyl) gave the title compound as an amorphous residue (18.0 g, 76 %).
MS m/e (%): 471 (M+H⁺, 85).

### b) 4-(1H-Indol-3-yl)-5,6-dihydro-2H-pyridine-1,3-dicarboxylic acid 1-tert-butyl ester

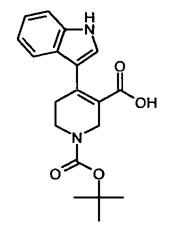

The title compound was obtained as a light brown solid after trituration from THF in comparable yield according to the procedure described above for the preparation of 4-(4-fluoro-phenyl)-5,6-dihydro-2*H*-pyridine-1,3-dicarboxylic acid 1-*tert*-butyl ester using 4-[1-(*tert*-butyl-dimethyl-silanyl)-1*H*-indol-3-yl]-5,6-dihydro-2*H*-pyridine-1,3-dicarboxylic acid 1-*tert*-butyl ester 3-methyl ester instead of 4-(4-fluoro-phenyl)-5,6-dihydro-2*H*-pyridine-1,3-dicarboxylic acid 1-*tert*-butyl ester 3-methyl ester in step c). MS m/e (%): 341 (M-H⁺, 100)

### Example 3 of II

### 4-o-Tolyl-5,6-dihydro-2H-pyridine-1,3-dicarboxylic acid 1-tert-butyl ester

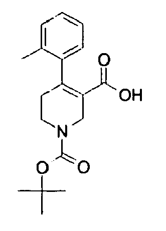

The title compound was obtained as white crystals in comparable yields according to the procedures described above for the preparation of 4-(1H-indol-3-yl)-5,6-dihydro-2H-pyridine-1,3-dicarboxylic acid 1-*tert*-butyl ester using o-tolylmagnesium chloride instead of 3-lithio-1-(*tert*-butyl-dimethyl-silanyl)-1*H*-indole freshly prepared from 3-bromo-1-(*tert*-butyl-dimethyl-silanyl)-1*H*-indole and *tert*-butyllithium in step a).
MS m/e (%): 316 (M-H⁺, 100)

### Example 4 of II

### 4-(3-Methoxy-phenyl)-5,6-dihydro-2H-pyridine-1,3-dicarboxylic acid 1-tert-butyl ester

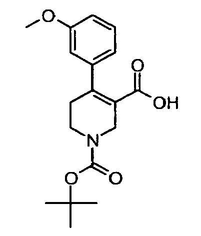

The title compound was obtained as off-white crystals in comparable yields according to the procedures described above for the preparation of 4-(4-fluoro-phenyl)-5,6-dihydro-2*H*-pyridine-1,3-dicarboxylic acid 1-*tert*-butyl ester using 3-methoxyphenylzinc iodide instead of 4-fluorophenylzinc bromide in step b).
MS m/e (%): 332 (M-H⁺, 100)

### Example 5 of II

### 4-Phenyl-5,6-dihydro-2H-pyridine-1,3-dicarboxylic acid 1-tert-butyl ester

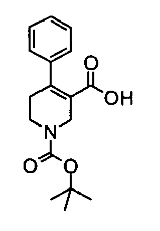

The title compound was obtained as off-white crystals in comparable yields according to the procedures described above for the preparation of 4-(4-fluoro-phenyl)-5,6-dihydro-2*H*-pyridine-1,3-dicarboxylic acid 1-*tert*-butyl ester using phenylzinc iodide instead of 4-fluorophenylzinc bromide in step b).
MS m/e (%): 302 (M-H⁺, 100)

### Example 6 of II

### 5-Phenyl-3,6-dihydro-2H-pyridine-1,4-dicarboxylic acid 1-tert-butyl ester

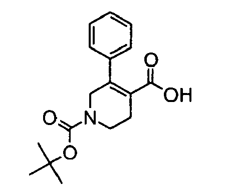

The title compound was obtained as a colorless viscous oil after flash column chromatography in comparable yields according to the procedures described above for the preparation of 4-(4-fluoro-phenyl)-5,6-dihydro-2*H*-pyridine-1,3-dicarboxylic acid 1-*tert*-butyl ester using 3-oxo-piperidine-1,4-dicarboxylic acid 1-tert-butyl ester 4-ethyl ester instead of 4-oxo-piperidine-1,3-dicarboxylic acid 1-*tert*-butyl ester 3-methyl ester in step a) and phenylzinc iodide instead of 4-fluorophenylzinc bromide in step b).
MS m/e (%): 302 (M-H⁺, 100)

### Example 7 of II

### 2-Phenyl-cyclohex-1-enecarboxylic acid

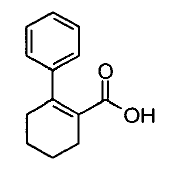

The title compound was obtained as white crystals in comparable yields according to the procedures described above for the preparation of 4-(4-fluoro-phenyl)-5,6-dihydro-2*H-*pyridine-1,3-dicarboxylic acid 1-*tert*-butyl ester using cyclohexanone-2-carboxylic acid ethylester instead of 4-oxo-piperidine-1,3-dicarboxylic acid 1-*tert*-butyl ester 3-methyl ester in step a) and phenylzinc iodide instead of 4-fluorophenylzinc bromide in step b). MS m/e (%): 201 (M-H⁺, 100)

### Example 8 of II

### 2-Phenyl-cyclopent-1-enecarboxylic acid

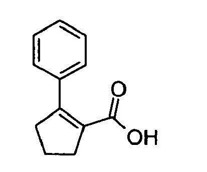

The title compound was obtained as off-white crystals in comparable yields according to the procedures described above for the preparation of 4-(4-fluoro-phenyl)-5,6-dihydro-2*H*-pyridine-1,3-dicarboxylic acid 1-*tert*-butyl ester using cyclopentanone-2-carboxylic acid methylester instead of 4-oxo-piperidine-1,3-dicarboxylic acid *1-tert-butyl* ester 3-methyl ester in step a) and phenylzinc iodide instead of 4-fluorophenylzinc bromide in step b).
MS m/e (%): 187 (M-H⁺, 100)

### Example 9 of II

### 4-Phenyl-2,5-dihydro-pyrrole-1,3-dicarboxylic acid 1-tert-butyl ester

### a) 1-Benzyl-4-phenyl-2,5-dihydro-1H-pyrrole-3-carboxylic acid ethyl eater

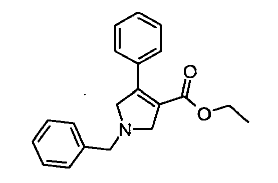

A solution of ethyl phenylpropiolate (12.0 g, 68.9 mmol) and *N*-(methoxymethyl)-*N-*(trimethylsilylmethyl) benzylamine (26.2 g, 110 mmol) in 180 ml dichloromethane was cooled to 0 °C with an ice-water bath. Trifluoroacetic acid (0.53 ml, 6.9 mmol) was added slowly, keeping the temperature of the reaction mixture below 20 °C. After completed addition the mixture was stirred at room temperature for 16 h. The solvent was removed under reduced pressure. The residue was dissolved in 2 M aqueous hydrochloric acid solution (150 ml) and extracted with three portions of n-heptane (3 x 100 ml). The aqueous layer was basified with 32% aqueous sodium hydroxide solution (30 ml) and extracted with three portions of ethyl acetate (3 x 150 ml). The combined ethyl acetate extracts were washed with brine, dried over sodium sulfate and concentrated under reduced pressure. Flash chromatography (n-heptane / ethyl acetate) gave the title compound (17.0 g, 80%) as a slightly yellow oil.
MS m/e (%): 308.5 (M+H⁺, 100).

### b) 4-Phenyl-2,5-dihydro-pyrrole-1,3-dicarboxylic acid 1-tert-butyl ester

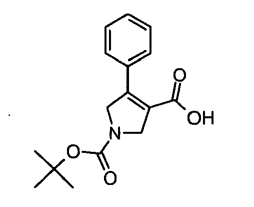

A mixture of 1-benzyl-4-phenyl-2,5-dihydro-1*H*-pyrrole-3-carboxylic acid ethyl ester (25.0 g, 81.3 mmol) and 1-chloroethyl chloroformate (10.7 ml, 97.6 mmol) in 450 ml 1,2-dichloroethane was heated at 50 °C for 24 h. After evaporation of the solvent the residue was dissolved in methanol and heated at reflux for 1 h. The reaction mixture was concentrated in vacuo and the residual hydrochloride was redissolved in a mixture of 450 ml THF and triethylamine (34.0 ml, 244 mmol). Di*-tert*-butyl dicarbonate (26.6 g, 122 mmol) was added at 0 °C, and the reaction mixture was stirred for 1h. The reaction mixture was diluted with saturated aqueous ammonium chloride solution and extracted with three portions of *tert*-butyl methyl ether (3 x 200ml). The combined organic layers were dried over sodium sulfate and concentrated in vacuo to give 40 g of crude 4-phenyl-2,5-dihydro-pyrrole-1,3-dicarboxylic acid 1-*tert*-butyl ester 3-ethyl ester, which was contaminated mainly with di-tert-butyl dicarbonate and benzyl chloride, as a yellow oil. A mixture of this material, 400 ml 1,4-dioxane and 400 ml 2 M aqeuous sodium hydroxide solution was stirred at room temperature over night. The reaction mixture was washed with two portions of hepane. The aqueous layer was acidified with ice-cold 4 M aqueous hydrochloric acid solution (270 ml). Filtration and washing with cold water gave the title compound as white crystals (19.7 g, 83 %).
MS m/e (%): 288 (M-H⁺, 100).

### Example 10 of II

### 4-(4-Chloro-phenyl)-2,5-dihydro-pyrrole-1,3-dicarboxylic acid 1-tert-butyl ester

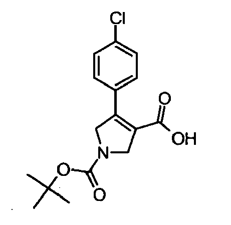

The title compound was obtained as white crystals according to the procedures described above for the preparation of 4-phenyl-2,5-dihydro-pyrrole-1,3-dicarboxylic acid *1-tert-*butyl ester using methyl (4-chlorophenyl)propiolate (prepared as described by T. Eckert, J. Ipaktschi, Synthetic Communications 1998, 28, 327.) instead of ethyl phenylpropiolate in step a).
MS m/e (%): 268 (M+H⁺ -C₄H₉, 100).

### Example 11 of II

### 4-(3-Fluoro-phenyl)-2,5-dihydro-pyrrole-1,3-dicarboxylic acid 1-tert-butyl ester

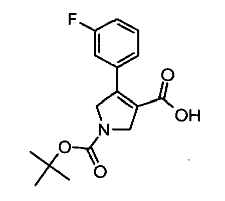

The title compound was obtained as off-white crystals according to the procedures described above for the preparation of 4-phenyl-2,5-dihydro-pyrrole-1,3-dicarboxylic acid 1-*tert*-butyl ester using methyl (3-fluorophenyl)propiolate (prepared as described by T. Eckert, J. Ipaktschi, Synthetic Communications 1998, 28, 327.) instead of ethyl phenylpropiolate in step a).
MS m/e (%): 306 (M-H⁺, 69).

### Example 12 of II

### 1-Benzyl-4-phenyl-2,5-dihydro-1H-pyrrole-3-carboxylic acid

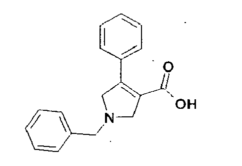

A mixture of 1-benzyl-4-phenyl-2,5-dihydro-1*H*-pyrrole-3-carboxylic acid ethyl ester (1.88 g , 6.12 mmol), 33 ml 1,4-dioxane and 33 ml 2M aqeuous sodium hydroxide solution was stirred at room temperature over night. The mixture was acidified to pH 4 with ice-cold 4 M aqueous hydrochloric acid solution and extracted with three portions of dichloromethane. The combined organic layers were dried over sodium sulfate and concentrated in vacuo to give 1.2 g of a white solid. Trituration from warm ethanol and filtration gave the title compound (0.54 g, 32 %) as a white solid.
MS m/e (%): 278 (M-H⁺, 100).

### Example 13 of II

### 4-Phenyl-2,5-dihydro-thiophene-3-carboxylic acid

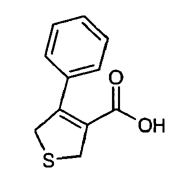

The title compound has been synthesized in comparable yields according to the following literature procedures using toluene instead of benzene for the formation of thio benzoic acid :
a) T. Aoyama, T. Takido, M. Kodomari, Synth.Comm. 2003, 33 (21), 3817;
b) D. H. Martyres , J. E. Baldwin, R. M. Adlington, V. Lee, M. R. Probert, D. J. Watkin, Tetrahedron 2001, 57, 4999;
c) G: M. Coppola, R. E. Damon, H. Xu, Synlett 1995, 11, 1143.
MS m/e (%): 205 (M-H⁺, 100).

### Example 14 of II

### 2-Phenyl-cyclooct-1-enecarboxylic acid

### a) 2-Trifluoromethanesulfonyloxy-1,2-dihydro-1-carboxylic acid ethyl ester

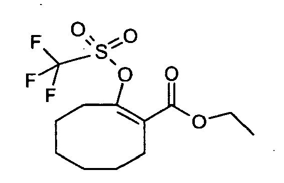

To a solution of 2-Oxo-cyclooctanecarboxylic acid ethyl ester (9.65 g, 47.2 mmol) in 33 ml THF was added sodium hydride (suspension in oil, 55 %, 4.57 g, 104.8 mmol) at 0 °C. After stirring for 30 min. at 0 °C N-phenyltrifluoromethanesulfonimide (28.17 g, 78.8 mmol) was added. The ice-water bath was removed and the reaction mixture was stirred for 2 days. Quenching with ice was followed by concentration in vacuo to remove THF. The residue was diluted with *tert*-butyl methyl ether and washed with three portions of 1 M aqueous sodium hydroxide solution. The organic layer was washed with brine and dried over sodium sulfate. Concentration in vacuo gave the crude title compound with a purity of 94 % (15.41 g, 93 %).
MS m/e (%): 285 ( [M-OCH₂CH₃]⁺, 100).

### b) 2-Phenyl-cyclooct-1-enecarboxylic acid ethyl ester

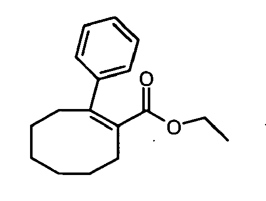

To a mixture of 2-Trifluoromethanesulfonyloxy-1,2-dihydro-1-carboxylic acid ethyl ester (10.35 g, 29.8 mmol), phenylzinc iodide solution (0.5 M in THF, 98.8 ml, 49.4 mmol) and 330 ml THF was added tetrakis(triphenylphosphine)palladium(0) (2.08 g, 1.78 mmol) and lithium chloride (1.27 g, 29.8 mmol) at RT. After stirring for 27 h the reaction was quenched with ice. The mixture was diluted with *tert*-butyl methyl ether and washed with 2 M aqueous sodium carbonate solution. The aqueous layer was extracted with two portions of *tert*-butyl methyl ether. The combined organic layers were washed with brine, dried over sodium sulfate and concentrated in vacuo. Purification of the residue by flash chromatography (heptane / ethyl acetate 50:1) gave the title compound as a colourless oil in 90 % purity (4.57 g, 54 %).
MS m/e (%): 259 (M+H⁺, 100%).

### c) 2-Phenyl-cyclooct-1-enecarboxylic acid

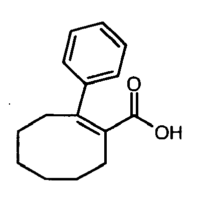

A mixture of 2-Phenyl-cyclooct-1-enecarboxylic acid ethyl ester (3.44 g, 9.02 mmol), 172 ml 1,4-dioxane and 172 ml 1 M LiOH was refluxed for 20 h. After cooling to ambient temperature and extraction of the reaction mixture with two portions of *tert*-butyl methyl ether (440 ml in total), the combined organic layers were extracted with 1 M aqueous sodium hydroxide solution (220 ml). The combined aqueous layers were cooled to 0 °C by addition of ice (150 g) and acidified to pH 1 with ice-cold 4 M aqueous hydrochloric acid solution (100 ml). The aqueous layer was extracted with two 250 ml-portions of ethyl acetate. The combined organic layers were washed with brine (50 ml), dried over sodium sulfate and concentrated in vacuo. Crystallization of the crude acid from a mixture of n-heptane and ethyl acetate (13 : 1, 210 ml) gave the title compound as off-white crystals (2.3 g, 75 %).

MS m/e (%): 229 (M-H⁺, 100).

### Representative procedure for the epimerization of enantiomerically enriched cis-substituted cyclic β-arylcarboxcylic acid derivates

### Example 1 of III

### (+)-(3R,4R)-4-(4-Fluoro-phenyl)-piperidine-1,3-dicarboxylic acid 1-tert-butyl ester 3-methyl ester

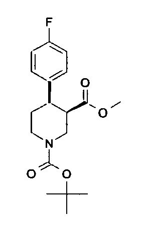

To a solution of triphenylphosphine (3.82 g, 14.6 mmol) in 70 ml tetrahydrofuran was added diethyl azodicarboxylate (2.53 g, 14.6 mmol) at 0 °C. After 30' methanol (4.55 ml, 112.0 mmol) and a solution of (3R,4R)-4-(4-fluoro-phenyl)-piperidine-1,3-dicarboxylic acid 1-*tert*-butyl ester (3.62 g, 11.2 mmol, 93.6% ee) in 30 ml tetrahydrofuran were added subsequently at 0-5 °C. The reaction mixture was stirred for 20 h at room temperature. Quenching with water was followed by extraction with *tert*-butyl methyl ether (3 x 100ml). The combined organic layers were dried over sodium sulfate, concentrated under reduced pressure and purified by flash chromatography (n-heptane / ethyl acetate) to give the title compound (3.55 g, 94 %) as a colorless oil.
MS m/e (%): 338 (M+H⁺, 28).
[α]_{D} = +68.69 (c = 0.310, CHCl₃)
[α]₅₇₈ = +71.27 (c = 0.310, CHCl₃)
[α]₃₆₅ = +221.60 (c = 0.310, CHCl₃)

### Example 1 of V

### (-)-(3S,4R)-4-(4-Fluoro-phenyl)-piperidine-1,3-dicarboxylic acid 1-tert-butyl ester

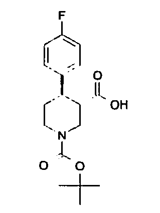

A mixture of (+)-(3R,4R)-4-(4-fluoro-phenyl)-piperidine-1,3-dicarboxylic acid 1*-tert-*butyl ester 3-methyl ester (3.55 g, 10.5 mmol) and sodium methoxide (1.14 g, 21.1 mmol) in 100 ml anhydrous toluene was heated at reflux over night. After cooling to room temperature the reaction mixture was quenched with water and concentrated in vacuo. The residue was dissolved in a mixture of 100 ml 1,4-dioxane and 50 ml 2M aqueous sodium hydroxide solution. After stirring at RT for 5 h the mixture was diluted with water and washed with two portions of *tert*-butyl methyl ether. The aqueous layer was cooled to 0 °C, acidified to pH 1-2 with ice-cold 1M aqueous hydrochloric acid solution and extracted with three portions of *tert*-butyl methyl ether. The combined organic layers were dried over sodium sulfate and concentrated in vacuo. Flash column chromatography and crystallization from heptane/ethyl acetate 9:1 (30 ml) gave the title compound as white crystals (1.76 g, 52 %, 97.5 % ee).
MS m/e (%): 322 (M-H⁺, 100).
[α]_{D} = 0.650 (c = 0.154, CHCl₃)

### HPLC method for ee determination:

Chiralpak-OD-H column, 25 cm*4.6 mm, 95 % n-heptane + 5 % 2-propanol with 0.1 % trifluroacetic acid, flow 0.7 ml/min, 30 °C, 0.001 ml injection volume, 210 nm. Retention times: (-)-acid 9.5 min, (+)-acid 11.5 min.

### Assignment of the absolute configuration

The absolute configuration of the title compound was assigned as (3*S*,4R) by comparison of the optical rotation and the retention time by HPLC analysis on a Chiralpak-OD-H column with the values of a sample of (-)-(3*S*,4R)-4-(4-fluoro-phenyl)-piperidine-1,3-dicarboxylic acid 1-*tert*-butyl ester which was derived from (-)-(3*S*,4R)-4-(4-fluorophenyl)-1-methyl-piperidine-3-carboxylic acid methyl ester (prepared as described in WO0129031) as follows:
A solution of (-)-(3*S*,4R)-4-(4-fluoro-phenyl)-1-methyl-piperidine-3-carboxylic acid methyl ester (575 mg, 2.29 mmol) and 1-chloroethyl chloroformate (393 mg, 2.75 mmol) in 5 ml 1,2-dichloroethane was heated at reflux for 4 h. After cooling to room temperature and evaporation of the solvent in vacuo the residue was dissolved in 5 ml methanol. The solution was heated at reflux for 1 h, followed by cooling to room temperature and concentration in vacuo. The residue was dissolved in 11.5 ml of a 2 M aqueous solution of hydrochloric acid and heated at reflux over night. After cooling the reaction mixture to 0 °C on an ice-water bath were added consecutively 2.8 ml of a 32 % aqueous solution of sodium hydroxide and a solution of di-*tert*-butyl dicarbonate (1.00 g, 4.58 mmol) in 15 ml 1,4-dioxane. The ice-water bath was removed after completed addition and stirring was continued at room temperatue for 4 h. The pH of the reaction mixture was adjusted to 8 by the addition of 1 M aqueous sodium hydroxide solution. Washing with two portions of *tert*-butyl methyl ether was followed by back-extraction of the combined organic layers with 1 M aqueous sodium hydroxide solution. The combined aqueous layers were cooled to 0 °C, acidified to pH 1 with ice-cold 4 M aqueous hydrochloric acid solution and extractred with three portions of ethyl acetate. The combined organic layers were washed with brine, dried over sodium sulfate and concentrated in vacuo to give (-)-(3*S*,4R)-4-(4-fluoro-phenyl)-piperidine-1,3-dicarboxylic acid 1-tert-butyl ester (590 mg, 80 %) with 93.8 % ee.
MS m/e (%): 322 (M-H⁺, 100).
[α]_{D} = -0.867 (c = 0.462, CHCl₃)

## Claims

1. A process for the preparation of enantiomerically enriched cyclic β-arylcarboxylic acid derivatives of formula 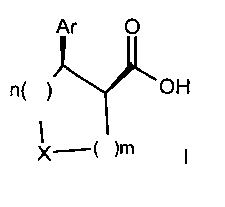 wherein
X is -C(R)(R')-, -N(R")-, -O-, -S(O)ₒ-, C(O)N(R"), -N(R")C(O)- or -C(O)-;
R and R' are independently from each other hydrogen, C₁₋₇-alkyl, C₁₋₇-alkyl substituted by
halogen, C₁₋₇-alkoxy, hydroxy or -(CH₂)_{P}-Ar;
R" is hydrogen, C₁₋₇-alkyl, C₁₋₇-alkyl substituted by halogen, -S(O)ₒ-C₁₋₇-alkyl, -S(O)ₒ-Ar, -S(O)ₒ-NRR', -(CH₂)ₚ-Ar, -C(O)-C₁₋₇-alkyl, -C(O)-Ar, -C(O)-NRR' or -C(O)O-C₁₋₇-alkyl;
Ar is aryl¹ or heteroaryl¹;
n is 0, 1, 2 or 3;
m is 0, 1, 2 or 3;
o is 0, 1 or 2;
p is 0, 1, or 2;
and corresponding salts thereof.
comprising catalytic homogeneous enantioselective hydrogenation of a compound of formula (II) 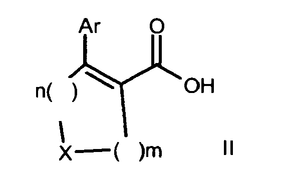 wherein
X is -C(R)(R')-, -N(R")-, -O-, -S(O)ₒ-, C(O)N(R"), -N(R")C(O)- or -C(O)-;
R and R' are independently from each other hydrogen, C₁₋₇-alkyl, C₁₋₇-alkyl substituted by
halogen, C₁₋₇-alkoxy, hydroxy or -(CH₂)ₚ-Ar;
R" is hydrogen, C₁₋₇-alkyl, C₁₋₇-alkyl substituted by halogen, -S(O)ₒ-C₁₋₇-alkyl, -S(O)ₒ-Ar, -S(O)ₒ-NRR', -(CH₂)ₚ-Ar, -C(O)-C₁₋₇-alkyl, -C(O)-Ar, -C(O)-NRR' or -C(O)O-C₁₋₇-alkyl;
Ar is aryl¹ or heteroaryl¹;
n is 0, 1, 2 or 3;
m is 0, 1, 2 or 3;
o is 0, 1 or 2;
p is 0, 1, or 2;
and corresponding salts thereof
in the presence of a catalyst comprising
Ru(Z)₂D XVII
[Ru(Z)₂₋ₚ(D)(L¹)ₘ](B)ₚ XVIII
wherein Z represents halogen or the group A-COO, A represents lower alkyl, aryl², halogenated lower alkyl or halogenated aryl², D represents a chiral diphosphine ligand, B represents a non coordinating anion as defined above and L¹ represents a neutral ligand as defined above, p represents the numbers 1 and 2, the ligands can be the same or different, m represents the numbers 1, 2 or 3,
or comprising
[Rh(chiral diphosphine)LX] or [Rh(chiral diphosphine)L]⁺A⁻
wherein X is Cl-, Br or I⁻, L is a neutral ligand, selected from the group consisting of ethylene, propylene, cyclooctene, 1,3-hexadiene, norbornadiene, 1,5-cyclooctadiene, benzene, hexamethylbenzene, 1,3,5-trimethylbenzene, p-cymene, tetrahydrofuran, dimethylformamide, acetonitrile, benzonitrile, acetone or methanol, A is an anion of an oxyacid or a complex acid selected from the group consisting of ClO₄ PF₆, BR₄, wherein R is halogen or aryl, SbF₆ or AsF₆, to yield said compound of formula (I).

2. A process according to claim 1, wherein the the chiral diphosphine ligand is **characterised by** formula (3), (4), (5), (6), (7), (8), (9), (10), (11), (12), (13), (14), (15) or (16). 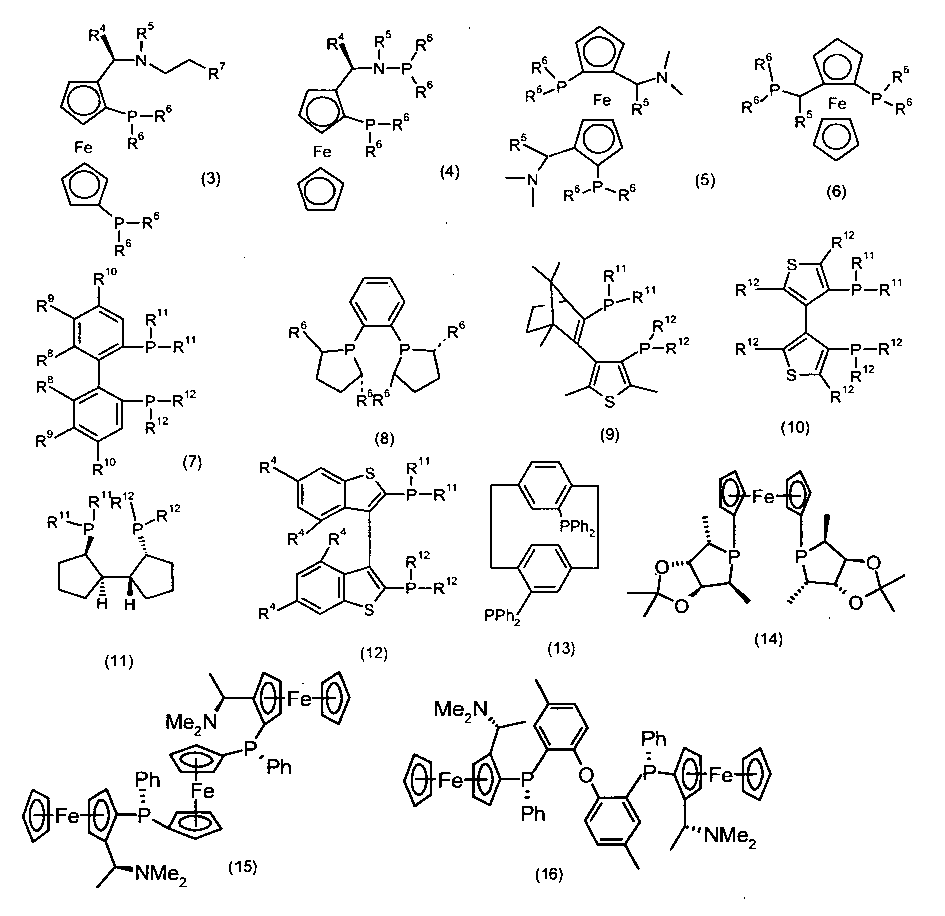 wherein
R⁴ is lower-alkyl;
R⁵ is lower-alkyl;
R⁶ independently is aryl², heteroaryl², cycloalkyl or lower-alkyl;
R⁷ is N(lower-alkyl)₂ or piperidinyl;
R⁸ is lower-alkyl, lower-alkoxy, hydroxy or lower-alkyl-C(O)O-;
R⁹ and R¹⁰ independently are hydrogen, lower-alkyl, lower-alkoxy or di(lower-alkyl)amino; or
R⁸ and R⁹ which are attached to the same phenyl group, or R⁹ and R¹⁰ which are attached to the same phenyl group, or both R⁸, taken together, are -X-(CH₂)ₙ-Y-, wherein X is -O- or -C(O)O-, Y is -O- or -N(lower-alkyl)- and n is an integer from 1 to 6, or a CF₂ group; or
R⁸ and R⁹, or R⁹ and R¹⁰, together with the carbon atoms to which they are attached, form a naphthyl, tetrahydronaphthyl or dibenzofuran ring;
R¹¹ and R¹² independently are lower alkyl, cycloalkyl, phenyl, napthyl or heteroaryl, substituted with 0 to 7 substituents independently selected from the group consisting of lower-alkyl, lower-alkoxy, di(lower-alkyl)amino, morpholino, phenyl and tri(lower-alkyl)silyl;

3. A process according to any of claims 1 or 2, wherein the catalyst is of the formula
Ru(Z)₂D XVII
(Ru(Z)₂₋ₚ(D)(L¹)m](B)ₚ XVIII
wherein Z represents halogen or the group A-COO, A represents lower alkyl, aryl², halogenated lower alkyl or halogenated aryl₂, B represents a non coordinating anion as defined above and L¹ represents a neutral ligand as defined above, p represents the numbers 1 and 2, m represents the numbers 1, 2 or 3, the ligands can be the same or different and D represents a chiral diphosphine ligand, wherein the chiral diphosphine is **characterised by** formula (7), (9), (10) or (12) as defined in claim 2.

4. A process according to claim 3, wherein Z is CH₃COO, CF₃COO or a halogenide.

5. A process according to claim 3, wherein the chiral diphosphine is selected from the group consisting of (R) and (S)-enantiomers of MeOBIPHEP, BIPHEMP, TMBTP, 2-Naphthyl)-MeOBIPHEP, (6-MeO-2-Naphthyl)-MeOBIPHEP, 2-(Thienyl)-MeOBIPHEP, 3,5-tBu-MeOBIPHEP, PHANEPHOS, BICP, TriMeOBIPHEP, (R,R,S,S)-Mandyphos, BnOBIPHEP, BenzoylBIPHEP, pTol-BIPHEMP, tButylCOOBIPHEP, iPrOBIPHEP, p-Phenyl-MeOBIPHEP, pAn-MeOBIPHEP, pTol-MeOBIPHEP, 3,5-Xyl-MeOBIPHEP, 3,5-Xyl-BIPHEMP, BINAP and 2-Furyl-MeOBIPHEP, 3,5-Xyl-4-MeO-MeOBIPHEP, 2-Furyl-MeOBIPHEP, or BITIANP.

6. A process according to claim 5, wherein the chiral diphosphine is *(S)*-(6-MeO-2-Naphthyl)-MeOBIPHEP, 3,5-Xyl-4-MeO-MeOBIPHEP, *(S)*-2-Furyl-MeOBIPHEP or BITIANP.

7. A process according to any of claims 1 to 6, wherein the catalyst is selected from the group consisting of (R) and (S) enantiomers of [Ru(CH₃COO)₂(TMBTP)], [Ru(CF₃COO⁻)₂(TMBTP)], [Ru(CH₃COO⁻)₂(2-naphthyl)-MeOBIPHEP)], [Ru(CF₃COO⁻)(2-naphthyl)-MeOBIPHEP)], [Ru(CH₃COO⁻)(6-MeO-2-naphthyl)-MeOBIPHEP)] and [Ru(CF₃COO⁻)₂(6-MeO-2-naphthyl)-MeOBIPHEP)].

8. A process according to any of claims 1 to 7, wherein the catalytic hydrogenation is carried out at a pressure of 1 to 150 bar.

9. A process according to claim 8, wherein the catalytic hydrogenation is carried out at a pressure of 10 to 100 bar.

10. A process according to any of claims 1 to 9, wherein the catalytic hydrogenation is carried out at a temperature of 10 to 100°C.

11. A process according to claim 10, wherein the catalytic hydrogenation is carried out at a temperature of 20 to 80°C.

12. A process according to any of claims 1 to 11, wherein the catalytic hydrogenation is carried out in the presence of a base.

13. A process according to claim 12, wherein the base is selected from the group consisting of NEt₃, i-Pr₂NEt, i-Pr₂NH, C₆H₅CH₂NH₂, 1-phenyl-benzylamine, *(R*) or (*S*)), ethylene diamine, tetramethylethylene diamine, NaOAc, NaOEt, NaOH or of Bu₄NX, wherein X is F, Cl, Br or I.

14. A process according to claim 13, wherein the catalytic hydrogenation is carried out in the presence of NEt₃ or i-Pr₂Net.

15. A process according to any of claims 1 to 14, wherein the catalytic hydrogenation is carried out in a solvent.

16. A process according to claim 15, wherein the catalytic hydrogenation with a Ru-catalyst is carried out with alcohols, hydrocarbons, chlorinated hydrocarbons, THF or water, or with a mixture thereof.

17. A process according to claim 15, wherein the catalytic hydrogenation with a Rh-catalyst is carried out with alkanols, benzene, toluene, trifluoro toluene, dichloromethane, dichlororethane, ethylene glycole, DMF, DMA, N-methylpyrrolidinone, acetonitrile or DMSO or with a mixture thereof.

18. A process according to claim 16, wherein the catalytic hydrogenation with a Ru-catalyst is carried out in a solvent which is methanol or ethanol.

19. A process according to claim 15, wherein the concentration of solvents is 1-50 W%.

20. A process according to any one of claims 1 to 19, wherein the ratio of substrate/catalyst (s/C) is 5 : 30000.

21. A process according to any of claims 1 to 20, wherein the compounds of formula (I) are
2-aryl/heteroaryl-cyclopentane carboxylic acids,
4-aryl/heteroaryl-2,5-dihydro-1H-pyrrolidine-3-carboxylic acids,
4-aryl/heteroaryl-tetrahydrofuran-3-carboxylic acids,
4-aryl/heteroaryl-tetrahydro-thiophene-3-carboxylic acids,
1,1-dioxo-4-aryl/heteroaryl-tetrahydro-1λ⁶-thiophene-3-carboxylic acids,
1-oxo-4-aryl/heteroaryl-tetrahydro-1λ⁴-thiophene-3-carboxylic acids,
2-aryl/heteroaryl-cyclohexane carboxylic acid,
4-aryl/heteroaryl-piperidine-3-carboxylic acids,
5-aryl/heteroaryl-piperidine-4-carboxylic acids,
4-aryl/heteroaryl-tetrahydro-pyran-3-carboxylic acids,
5-aryl/heteroaryl-tetrahydro-pyran-4-carboxylic acids,
4-aryl/heteroaryl-tetrahydro-thiopyran-3-carboxylic acids,
5-aryl/heteroaryl-tetrahydro-thiopyran-4-carboxylic acids,
1,1 -dioxo-4-aryl/heteroaryl-hexahydro-1λ⁶-thiopyran-3-carboxylic acids,
1,1-dioxo-5-aryl/heteroaryl-hexahydro-1λ⁶-thiopyran-4-carboxylic acids,
1-oxo-4-aryl/heteroaryl-hexahydro-1λ⁴-thiopyran-3-carboxylic acids,
2-phenyl-cycloheptane carboxylic acid,
2-phenyl-cyclooctane carboxylic acid and corresponding salts thereof.

22. Compounds of formula I according to claim 1, which compounds are
(+)-(3*R*,4*R*)-4-(4-fluoro-phenyl)-piperidine-1,3-dicarboxylic acid-1-*tert*-butyl ester and
(-)-(3*S*,4*S*)-4-(4-fluoro-phenyl)-piperidine-1,3-dicarboxylic acid 1-*tert*-butyl ester,
(-)-4-(1H-indol-3-yl)-piperidine-1,3-dicarboxylic acid-1-*tert*-butyl ester and
(+)-4-(1H-indol-3-yl)-piperidine-1,3-dicarboxylic acid-1-*tert*-butyl ester,
(-)-4-o-tolyl-piperidine-1,3-dicarboxylic acid 1-*tert*-butyl ester and
(+)-4-o-tolyl-piperidine-1,3-dicarboxylic acid 1-*tert*-butyl ester,
(+)-4-(3-methoxy-phenyl)-piperidine-1,3-dicarboxylic acid-1-*tert*-butyl ester,
(+)-4-phenyl-piperidine-1,3-dicarboxylic acid-1-tert-butyl ester and
(-)-4-phenyl-piperidine-1,3-dicarboxylic acid-1-tert-butyl ester
(+)-3-phenyl-piperidine-1,4-dicarboxylic acid 1-*tert*-butyl ester and
(-)-3-phenyl-piperidine-1,4-dicarboxylic acid 1-*tert*-butyl ester,
(-)-2-phenyl-cyclopentanecarboxylic acid and
(+)-2-phenyl-cyclopentanecarboxylic acid,
(+)-(3*R*,4*R*)-4-(phenyl)-pyrrolidine-1,3-di carboxylic acid-1-*tert*-butyl ester and
(-)-(3*S*,4*S*)-4-(phenyl)-pyrrolidine-1,3-di carboxylic acid-1-*tert*-butyl ester,
(-)-4-(4-chloro-phenyl)-pyrrolidine-1,3-di carboxylic acid-1-*tert*-butyl ester and
(+)-4-(4-chloro-phenyl)-pyrrolidine-1,3-di carboxylic acid-1-*tert*-butyl ester,
(+)-4-(3-fluoro-phenyl)-pyrrolidine-1,3-di carboxylic acid-1-*tert*-butyl ester and
(-)-4-(3-fluoro-phenyl)-pyrrolidine-1,3-di carboxylic acid-1-*tert*-butyl ester,
(3*R*,4*R*)-1-benzyl-4-phenyl-pyrrolidine-3-carboxylic acid and
(3*RS*,4*RS*)-1-benzyl-4-phenyl-pyrrolidine-3-carboxylic acid,
(-)-2-phenyl-cyclooctanecarboxylic acid,
(+)-4-phenyl-tetrahydro-thiophene-3-carboxylic acid and
(-)-4-phenyl-tetrahydro-thiophene-3-carboxylic acid.

23. A process according to any of claims 1 to 20, wherein the starting compounds of formula (II) are 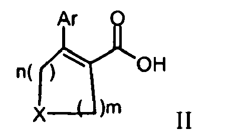 2-aryl/heteroaryl-cyclopent-1-ene carboxylic acids,
4-aryl/heteroaryl-2,5-dihydro-1H-pyrrole-3-carboxylic acids,
4-aryl/heteroaryl-2,5-dihydro-furan-3-carboxylic acids,
4-aryl/heteroaryl-2,5-dihydro-thiophene-3-carboxylic acids,
1,1-dioxo-4-aryl-2,5-dihydro-1H-1λ⁶-thiophene-3-carboxylic acids,
2-aryl/heteroaryl-cyclohexyl-1-ene carboxylic acid,
4-aryl/heteroary -1,2,5,6-tetrahydro-pyridine-3-carboxylic acids,
5-aryl/heteroaryl-1,2,3,6-tetrahydro-pyridine-4-carboxylic acids,
4-aryl/heteroaryl-5,6-dihydro-2H-pyran-3-carboxylic acids,
5-aryl/heteroaryl-3,6-dihydro-2H-pyran-4-carboxylic acids,
4-aryl/heteroaryl-5,6-dihydro-2H-thiopyran-3-carboxylic acids,
5-aryl/heteroaryl-3,6-dihydro-2H-thiopyran-4-carboxylic acids,
1,1-dioxo-4-aryl/heteroaryl-1,2,5,6-tetrahydro-1λ⁶-thiopyran-3-carboxylic acids,
1,1-dioxo-5-aryl/heteroaryl-1,2,3,6-tetrahydro-1λ⁶-thiopyran-4-carboxylic acids,
1-oxo-4-aryl/heteroaryl-1,2,5,6-tetrahydro-1λ⁴-thiopyran-3-carboxylic acids,
1-oxo-4-aryl/heteroaryl-2,5-dihydro-1H-1λ⁴-thiophene-3-carboxylic acids,
2-phenyl-cyclohept-1-enecarboxylic acid
2-phenyl-cyclooct-1-enecarboxylic acid and corresponding salts thereof.

## Patentansprüche

1. Verfahren zur Herstellung enantiomerenangereicherter cyclischer β-Arylcarbonsäurederivate der Formel 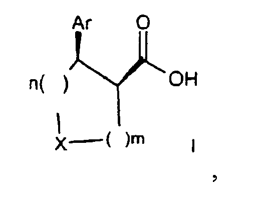 worin
X -C(R)(R')-, -N(R")-, -O-, -S(O)ₒ-, C(O)N(R"), -N(R")C(O)- oder -C(O)- ist;
R und R' unabhängig voneinander Wasserstoff, C₁₋₇-Alkyl, C₁₋₇Alkyl, substituiert durch Halogen, C₁₋₇-Alkoxy, Hydroxy oder-(CH₂)ₚ-Ar sind;
R" Wasserstoff, C₁₋₇-Alkyl, C₁₋₇-Alkyl, substituiert durch Halogen, -S(O)ₒ-C₁₋₇-Alkyl, -S(O)ₒ-Ar, -S(O)ₒ-NRR', -(CH₂)ₚ-Ar, -C(O)-C₁₋₇-Alkyl, -C(O)-Ar, -C(O)-NRR' oder -C(O)O-C₁₋₇-Alkyl ist;
Ar Aryl¹ oder Heteroaryl¹ ist;
n 0, 1, 2 oder 3 ist;
m 0, 1, 2 oder 3 ist;
o 0, 1 oder 2 ist;
p 0, 1 oder 2 ist;
und entsprechender Salze davon,
umfassend die katalytische homogene enantioselektive Hydrierung einer Verbindung der Formel (II) 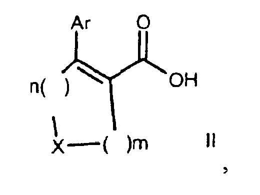 worin
X -C(R)(R')-, -N(R")-, -O-, -S(O)ₒ-, C(O)N(R"), -N(R")C(O)- oder -C(O)- ist;
R und R' unabhängig voneinander Wasserstoff, C₁₋₇-Alkyl, C₁₋₇-Alkyl, substituiert durch Halogen, C₁₋₇-Alkoxy, Hydroxy oder -(CH₂)ₚ-Ar sind;
R" Wasserstoff, C₁₋₇-Alkyl, C₁₋₇-Alkyl, substituiert durch Halogen, -S(O)ₒ-C₁₋₇Alkyl, -S(O)ₒ-Ar, -S(O)ₒ-NRR', -(CH₂)ₚ-Ar, -C(O)-C₁₋₇-Alkyl, -C(O)-Ar, -C(O)-NRR' oder -C(O)O-C₁₋₇-Alkyl ist;
Ar Aryl¹ oder Heteroaryl¹ ist;
n 0, 1, 2 oder 3 ist;
m 0, 1, 2 oder 3 ist;
o 0, 1 oder 2 ist;
p 0, 1 oder 2 ist;
und entsprechender Salze davon,
in Gegenwart eines Katalysators, umfassend
Ru(Z)₂D XVII
[Ru(Z)₂₋ₚ(D)(L¹)m](B)ₚ XVIII,
worin Z Halogen oder die Gruppe A-COO darstellt, A Niederalkyl, Aryl², halogeniertes Niederalkyl oder halogeniertes Aryl² darstellt, D einen chiralen Diphosphinliganden darstellt, B ein nicht-koordinierendes Anion wie oben definiert darstellt und L¹ einen neutralen Liganden wie oben definiert darstellt, p die Zahlen 1 und 2 darstellt, die Liganden gleich oder verschieden sein können, m die Zahlen 1, 2 oder 3 darstellt,
oder umfassend
[Rh(chirales Diphosphin)LX] oder [Rh(chirales Diphosphin)L]⁺A⁻,
worin X Cl⁻, Br⁻ oder I⁻ ist, L ein neutraler Ligand, ausgewählt aus der Gruppe, bestehend aus Ethylen, Propylen, Cycloocten, 1,3-Hexadien, Norbomadien, 1,5-Cyclooctadien, Benzol, Hexamethylbenzol, 1,3,5-Trimethylbenzol, p-Cymen, Tetrahydrofuran, Dimethylformamid, Acetonitril, Benzonitril, Aceton oder Methanol, ist, A ein Anion einer Oxysäure oder einer Komplexsäure ist, ausgewählt aus der Gruppe, bestehend aus ClO₄, PF₆, BR₄, worin R Halogen oder Aryl ist, SbF₆ oder AsF₆,
unter Erhalt der Verbindung der Formel (I).

2. Verfahren nach Anspruch 1, wobei der chirale Diphosphinligand durch die Formel (3), (4), (5), (6), (7), (8), (9), (10), (11), (12), (13), (14), (15) oder (16) **gekennzeichnet** ist, 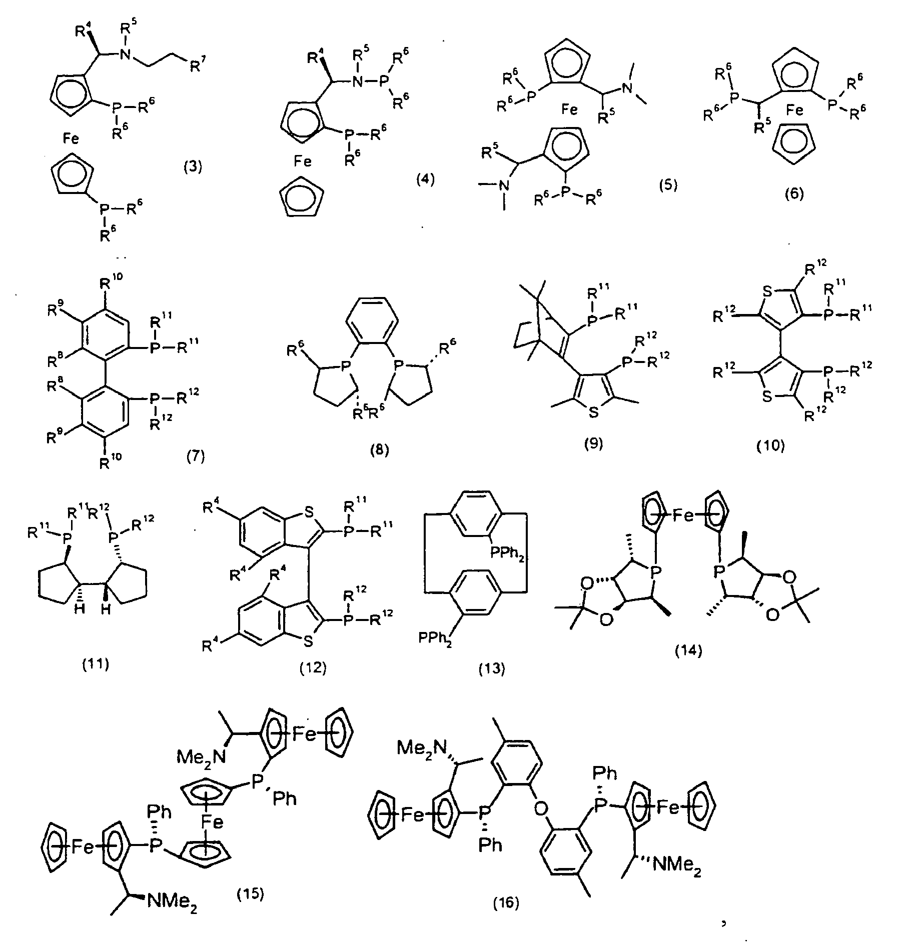 worin
R⁴ Niederalkyl ist;
R⁵ Niederalkyl ist;
R⁶ unabhängig Aryl², Heteroaryl², Cycloalkyl oder Niederalkyl ist;
R⁷ N(Niederalkyl)₂ oder Piperidinyl ist;
R⁸ Niederalkyl, Niederalkoxy, Hydroxy oder Niederalkyl-C(O)O- ist;
R⁹ und R¹⁰ unabhängig voneinander Wasserstoff, Niederalkyl, Niederalkoxy oder Di(niederalkyl)amino sind; oder
R⁸ und R⁹, die an dieselbe Phenylgruppe gebunden sind, oder R⁹ und R¹⁰, die an dieselbe Phenylgruppe gebunden sind, oder beide R⁸ zusammen -X-(CH₂)ₙ-Y-, worin X -O- oder -C(O)O- ist, Y -O- oder -N(Niederalkyl)- ist und n eine ganze Zahl von 1 bis 6 ist, oder eine CF₂-Gruppe sind; oder
R⁸ und R⁹ oder R⁹ und R¹⁰ zusammen mit den Kohlenstoffatomen, an die sie gebunden sind, einen Naphthyl-, Tetrahydronaphthyl- oder Dibenzofuranring bilden;
R¹¹ und R¹² unabhängig voneinander Niederalkyl, Cycloalkyl, Phenyl, Naphthyl oder Heteroaryl, substituiert mit 0 bis 7 Substituenten, unabhängig voneinander ausgewählt aus der Gruppe, bestehend aus Niederalkyl, Niederalkoxy, Di(niederalkyl)amino, Morpholino, Phenyl und Tri(niederalkyl)silyl, sind.

3. Verfahren nach einem der Ansprüche 1 oder 2, wobei der Katalysator die Formel
Ru(Z)₂D XVII
[Ru(Z)₂₋ₚ(D)(L¹)ₘ](B)ₚ XVIII
aufweist, worin Z Halogen oder die Gruppe A-COO darstellt, A Niederalkyl, Aryl², halogeniertes Niederalkyl oder halogeniertes Aryl² darstellt, B ein nicht-koordinierendes Anion wie oben definiert darstellt und L¹ einen neutralen Liganden wie oben definiert darstellt, p die Zahlen 1 und 2 darstellt, m die Zahlen 1, 2 oder 3 darstellt, die Liganden gleich oder verschieden sein können und D einen chiralen Diphosphinliganden darstellt, wobei das chirale Diphosphin durch die Formel (7), (9), (10) oder (12) wie in Anspruch 2 definiert **gekennzeichnet** ist.

4. Verfahren nach Anspruch 3, wobei Z CH₃COO, CF₃COO oder ein Halogenid ist.

5. Verfahren nach Anspruch 3, wobei das chirale Diphosphin ausgewählt ist aus der Gruppe, bestehend aus (R)- und (S)-Enantiomeren von MeOBIPHEP, BIPHEMP, TMBTP, (2-Naphthyl)-MeOBIPHEP, (6-MeO-2-Naphthyl)-MeOBIPHEP, 2-(Thienyl)-MeOBIPHEP, 3,5-tBu-MeOBIPHEP, PHANEPHOS, BICP, TriMeOBIPHEP, (R,R,S,S)-Mandyphos, BnOBIPHEP, BenzoylBIPHEP, pTol-BIPHEMP, tButylCOOBIPHEP, iPrOBIPHEP, p-Phenyl-MeOBIPHEP, pAn-MeOBIPHEP, pTol-MeOBIPHEP, 3,5-Xyl-MeOBIPHEP, 3,5-Xyl-BIPHEMP, BINAP und 2-Furyl-MeOBIPHEP, 3,5-Xyl-4-MeO-MeOBIPHEP, 2-Furyl-MeOBIPHEP oder BITIANP.

6. Verfahren nach Anspruch 5, wobei das chirale Diphosphin (*S*)-(6-MeO-2-Naphthyl)-MeOBIPHEP, 3,5-Xyl-4-MeO-MeOBIPHEP, *(S)*-2-Furyl-MeOBIPHEP oder BITIANP ist.

7. Verfahren nach einem der Ansprüche 1 bis 6, wobei der Katalysator ausgewählt ist aus der Gruppe, bestehend aus (R)- und (S)-Enantiomeren von [Ru(CH₃COO⁻)₂(TMBTP)], [Ru(CF₃COO⁻)₂(TMBTP)], [Ru(CH₃COO⁻)₂(2-Naphthyl)-MeOBIPHEP)], [Ru(CF₃COO⁻)₂(2-Naphthyl)-MeOBIPHEP)], [Ru(CH₃COO⁻)₂(6-MeO-2-Naphthyl)-MeOBIPHEP)] und [Ru(CF₃COO⁻)₂(6-MeO-2-Naphthyl)-MeOBIPHEP)].

8. Verfahren nach einem der Ansprüche 1 bis 7, wobei die katalytische Hydrierung bei einem Druck von 1 bis 150 bar durchgeführt wird.

9. Verfahren nach Anspruch 8, wobei die katalytische Hydrierung bei einem Druck von 10 bis 100 bar durchgeführt wird.

10. Verfahren nach einem der Ansprüche 1 bis 9, wobei die katalytische Hydrierung bei einer Temperatur von 10 bis 100 °C durchgeführt wird.

11. Verfahren nach Anspruch 10, wobei die katalytische Hydrierung bei einer Temperatur von 20 bis 80 °C durchgeführt wird.

12. Verfahren nach einem der Ansprüche 1 bis 11, wobei die katalytische Hydrierung in Gegenwart einer Base durchgeführt wird.

13. Verfahren nach Anspruch 12, wobei die Base ausgewählt ist aus der Gruppe, bestehend aus NEt₃, i-Pr₂NEt, i-Pr₂NH, C₆H₅CH₂NH₂, 1-Phenyl-benzylamin, (R) oder (S)), Ethylendiamin, Tetramethylethylendiamin, NaOAc, NaOEt, NaOH oder Bu₄NX, worin X F, Cl, Br oder I ist.

14. Verfahren nach Anspruch 13, wobei die katalytische Hydrierung in Gegenwart von NEt₃ oder i-Pr₂NEt durchgeführt wird.

15. Verfahren nach einem der Ansprüche 1 bis 14, wobei die katalytische Hydrierung in einem Lösungsmittel durchgeführt wird.

16. Verfahren nach Anspruch 15, wobei die katalytische Hydrierung mit einem Ru-Katalysator mit Alkoholen, Kohlenwasserstoffen, chlorierten Kohlenwasserstoffen, THF oder Wasser oder mit einem Gemisch davon durchgeführt wird.

17. Verfahren nach Anspruch 15, wobei die katalytische Hydrierung mit einem Rh-Katalysator mit Alkanolen, Benzol, Toluol, Trifluortoluol, Dichlormethan, Dichlorethan, Ethylenglycol, DMF, DMA, N-Methylpyrrolidinon, Acetonitril oder DMSO oder mit einem Gemisch davon durchgeführt wird.

18. Verfahren nach Anspruch 16, wobei die katalytische Hydrierung mit einem Ru-Katalysator in einem Lösungsmittel, das Methanol oder Ethanol ist, durchgeführt wird.

19. Verfahren nach Anspruch 15, wobei die Konzentration an Lösungsmitteln 1 - 50 Gew.-% beträgt.

20. Verfahren nach einem der Ansprüche 1 bis 19, wobei das Verhältnis von Substrat/Katalysator (s/K) 5 : 30.000 beträgt.

21. Verfahren nach einem der Ansprüche 1 bis 20, wobei die Verbindungen der Formel (I)
2-Aryl/Heteroaryl-cyclopentan-carbonsäuren,
4-Aryl/Heteroaryl-2,5-dihydro-1H-pyrrolidin-3-carbonsäuren,
4-Aryl/Heteroaryl-tetrahydrofuran-3-carbonsäuren,
4-Aryl/Heteroaryl-tetrahydro-thiophen-3-carbonsäuren,
1,1-Dioxo-4-aryl/heteroaryl-tetrahydro-1λ⁶-thiophen-3-carbonsäuren,
1-Oxo-4-aryl/heteroaryl-tetrahydro-1λ⁴-thiophen-3-carbonsäuren,
2-Aryl/Heteroaryl-cyclohexan-carbonsäure,
4-Aryl/Heteroaryl-piperidin-3-carbonsäuren,
5-Aryl/Heteroaryl-piperidin-4-carbonsäuren,
4-Aryl/Heteroaryl-tetrahydro-pyran-3-carbonsäuren,
5-Aryl/Heteroaryl-tetrahydro-pyran-4-carbonsäuren,
4-Aryl/Heteroaryl-tetrahydro-thiopyran-3-carbonsäuren,
5-Aryl/Heteroaryl-tetrahydro-thiopyran-4-carbonsäuren,
1,1-Dioxo-4-aryl/heteroaryl-hexahydro-1λ⁶-thiopyran-3-carbonsäuren,
1,1-Dioxo-5-aryl/heteroaryl-hexahydro-1λ⁶-thiopyran-4-carbonsäuren,
1-Oxo-4-aryl/heteroaryl-hexahydro-1λ⁴-thiopyran-3-carbonsäuren,
2-Phenyl-cycloheptan-carbonsäure,
2-Phenyl-cyclooctan-carbonsäure und die entsprechenden Salze davon sind.

22. Verbindungen der Formel I nach Anspruch 1, wobei die Verbindungen
(+)-(3*R*,4*R*)-4-(4-Fluor-phenyl)-piperidin-1,3-dicarbonsäure-1-*tert*-butylester und
(-)-(3*S*,4*S*)-4-(4-Fluor-phenyl)-piperidin-1,3-dicarbonsäure-1-*tert*-butylester,
(-)-4-(1*H*-Indol-3-yl)-piperidin-1,3-dicarbonsäure-1-*tert*-butylester und
(+)-4-(1*H*-Indol-3-yl)-piperidin-1,3-dicarbonsäure-1-*tert*-butylester,
(-)-4-o-Tolyl-piperidin-1,3-dicarbonsäure-1-*tert*-butylester und
(+)-4-o-Tolyl-piperidin-1,3-dicarbonsäure-1-*tert*-butylester,
(+)-4-(3-Methoxy-phenyl)-piperidin-1,3-dicarbonsäure-1-*tert*-butylester,
(+)-4-Phenyl-piperidin-1,3-dicarbonsäure-1-*tert*-butylester und
(-)-4-Phenyl-piperidin-1,3-dicarbonsäure-1-*tert*-butylester,
(+)-3-Phenyl-piperidin-1,4-dicarbonsäure-1-*tert*-butylester und
(-)-3-Phenyl-piperidin-1,4-dicarbonsäure-1-*tert*-butylester,
(-)-2-Phenyl-cyclopentan-carbonsäure und
(+)-2-Phenyl-cyclopentan-carbonsäure,
(+)-(3*R*,4*R*)-4-(Phenyl)-pyrrolidin-1,3-dicarbonsäure-1-*tert*-butylester und
(-)-(3*S*,4*S*)-4-(Phenyl)-pyrrolidin-1,3-dicarbonsäure-1-*tert*-butylester,
(-)-4-(4-Chlor-phenyl)-pyrrolidin-1,3-dicarbonsäure-1-*tert*-butylester und
(+)-4-(4-Chlor-phenyl)-pyrrolidin-1,3-dicarbonsäure-1-*tert*-butylester,
(+)-4-(3-Fluor-phenyl)-pyrrolidin-1,3-dicarbonsäure-1-*tert*-butylester und
(-)-4-(3-Fluor-phenyl)-pyrrolidin-1,3-dicarbonsäwe-1-*tert*-butylester,
(3*R*,4*R*)-1-Benzyl-4-phenyl-pyrrolidin-3-carbonsäure und
(3*RS*,4*RS*)-1-Benzyl-4-phenyl-pyrrolidin-3-carbonsäure,
(-)-2-Phenyl-cyclooctan-carbonsäure,
(+)-4-phenyl-tetrahydro-thiophen-3-carbonsäure und
(-)-4-Phenyl-tetrahydro-thiophen-3-carbonsäure
sind.

23. Verfahren nach einem der Ansprüche 1 bis 20, wobei die Ausgangsverbindungen der Formel (II) 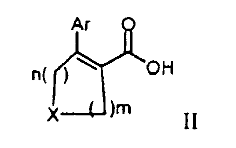 2-Aryl/Heteroaryl-cyclopent-1-en-carbonsäuren,
4-Aryl/Heteroaryl-2,5-dihydro-1H-pyrrol-carbonsäuren,
4-Aryl/Heteroaryl-2,5-dihydro-furan-3-carbonsäuren,
4-Aryl/Heteroaryl-2,5-dihydro-thiophen-3-carbonsäuren,
1,1-Dioxo-4-aryl-2, 5-dihydro-1H-1λ⁶-thiophen-3-carbonsäuren,
2-Aryl/Heteroaryl-cyclohexyl-1-en-carbonsäure,
4-Aryl/Heteroaryl-1,2,5,6-tetrahydro-pyridin-3-carbonsäuren,
5-Aryl/Heteroaryl-1,2,3,6-tetrahydro-pyridin-4-carbonsäuren,
4-Aryl/Heteroaryl-5,6-dihydro-2H-pyran-3-carbonsäuren,
5-Aryl/Heteroaryl-3,6-dihydro-2H-pyran-4-carbonsäuren,
4-Aryl/Heteroaryl-5,6-dihydro-2H-thiopyran-3-carbonsäuren,
5-Aryl/Heteroaryl-3,6-dihydro-2H-thiopyran-4-carbonsäuren,
1,1-Dioxo-4-aryl/heteroaryl-1,2,5,6-tetrahydro-1λ⁶-thiopyran-3-carbonsäuren,
1,1-Dioxo-5-aryl/heteroaryl-1,2, 3, 6-tetrahydro-1λ⁶-thiopyran-4-carbonsäuren,
1-Oxo-4-aryl/heteroaryl-1,2,5,6-tetrahydro-1λ⁴-thiopyran-3-carbonsäuren,
1-Oxo-4-aryl/heteroaryl-2,5-dihydro-1H-1λ⁴-thiophen-3-carbonsäuren,
2-Phenyl-cyclohept-1-en-carbonsäure;
2-Phenyl-cyclooct-1-en-carbonsäure und die entsprechenden Salze davon
sind.

## Revendications

1. Procédé pour la préparation de dérivés d'acides β-arylcarboxyliques cycliques énantiomériquement enrichis de formule 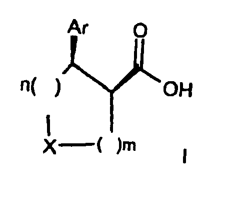 où
X est -C(R)(R')-, -N(R")-, -O-, -S(O)ₒ-, C(O)N(R"), -N(R")C(O)- ou -C(O)- ;
R et R' sont indépendamment l'un de l'autre l'hydrogène, C₁₋₇-alkyle, C₁₋₇-alkyle substitué par
halogène, C₁₋₇-alcoxy, hydroxy ou -(CH₂)ₚ-Ar;
R" est l'hydrogène, C₁₋₇-alkyle, C₁₋₇-alkyle substitué par halogène, -S(O)ₒ-C₁₋₇-alkyle, -S(O)ₒ-Ar, -S(O)ₒ-NRR', -(CH₂)ₚ-Ar, -C(O)-C₁₋₇-alkyle, -C(O)-Ar, -C(O)-NRR' ou -C(O)O-C₁₋₇-alkyle ;
Ar est aryle¹ ou hétéroaryle¹;
n est 0, 1, 2 ou 3 ;
m est 0, 1, 2 ou 3 ;
o est 0, 1 ou 2 ;
p est 0, 1 ou 2 ;
et de leurs sels correspondants,
comprenant l'hydrogénation énantiosélective homogène catalytique d'un composé de formule (II) 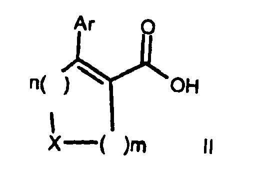 où
X est -C(R)(R')-, -N(R")-, -O-, -S(O)ₒ-, C(O)N(R"), -N(R")C(O)- ou -C(O)- ;
R et R' sont indépendamment l'un de l'autre l'hydrogène, C₁₋₇-alkyle, C₁₋₇-alkyle substitué par
halogène, C₁₋₇-alcoxy, hydroxy ou -(CH₂)ₚ-Ar ;
R" est l'hydrogène, C₁₋₇-alkyle, C₁₋₇-alkyle substitué par halogène, -S(O)ₒ-C₁₋₇-alkyle, -S(O)ₒ-Ar, -S(O)ₒ-NRR', -(CH₂)ₚ-Ar, -C(O)-C₁₋₇-alkyle, -C(O)-Ar, -C(O)-NRR' ou -C(O)O-C₁₋₇-alkyle ;
Ar est aryle¹ ou hétéroaryle¹ ;
n est 0, 1, 2 ou 3 ;
m est 0, 1, 2 ou 3 ;
o est 0, 1 ou 2 ;
p est 0, 1 ou 2 ;
et de ses sels correspondants,
en présence d'un catalyseur comprenant
Ru(Z)₂D x XVIII
[RU(Z)₂₋ₚ(D)(L¹)ₘ](B)ₚ XVIII
où Z représente un halogène ou le groupe A-COO, A représente alkyle inférieur, aryle² alkyle inférieur halogéné ou aryle² halogéné, D représente un ligand diphosphine chirale, B représente un anion non coordonnant comme défini ci-dessus et L¹ représente un ligand neutre comme défini ci-dessus, p représente les nombres 1 et 2, les ligands peuvent être identiques ou différents, m représente les nombres 1, 2 ou 3,
ou comprenant
[Rh(diphosphine chirale)LX] ou [Rh(diphosphine chirale)L]⁺A⁻
où X est Cl⁻, Br⁻ ou I⁻, L est un ligand neutre, choisi dans le groupe consistant en éthylène, propylène, cyclooctène, 1,3-hexadiène, norbomadiène, 1,5-cyclo-octadiène, benzène, hexaméthylbenzène, 1,3,5-triméthylbenzène, p-cymène, tétrahydrofurane, diméthylformamide, acétonitrile, benzonitrile, acétone ou méthanol,
A est un anion d'un oxyacide ou d'un acide complexe choisi dans le groupe consistant en ClO₄, PF₆, BR₄ où R est un halogène ou aryle, SbF₆ ou AsF₆,
pour donner ledit composé de formule (I).

2. Procédé selon la revendication 1 où le ligand diphosphine chirale est **caractérisé par** la formule (3), (4), (5), (6), (7), (8), (9), (10), (11), (12), (13), (14), (15) ou (16) 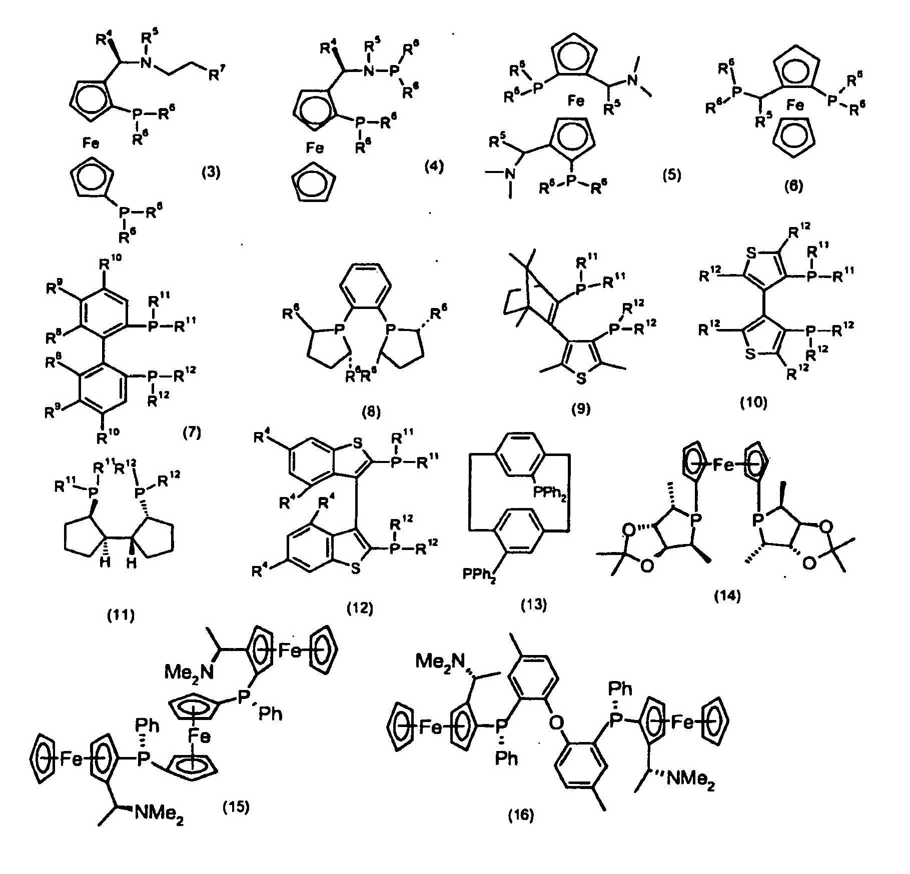 où
R⁴ est alkyle inférieur ;
R⁵ est alkyle inférieur ;
R⁶ est indépendamment aryle², hétéroaryle² cycloalkyle ou alkyle inférieur ;
R⁷ est N(alkyle inférieur)₂ ou pipéridinyle ;
R⁸ est alkyle inférieur, alcoxy inférieur, hydroxy ou alkyle inférieur-C(O)O- ;
R⁹ et R¹⁰ sont indépendamment l'hydrogène, alkyle inférieur, alcoxy inférieur ou di(alkyle inférieur)amino ; ou
R⁸ et R⁹ qui sont liés au même groupe phényle, ou R⁹ et R¹⁰ qui sont liés au même groupe phényle, ou les deux R⁸, combinés, sont -X-(CH₂)ₙ-Y-, où X est -O- ou -C(O)O-, Y est -O- ou -N(alkyle inférieur)- et n est un entier de 1 à 6, ou un groupe CF₂ ; ou
R⁸ et R⁹, ou R⁹ et R¹⁰, avec les atomes de carbone auxquels ils sont liés, forment un cycle naphtyle, tétrahydronaphtyle ou dibenzofurane ;
R¹¹ et R¹² sont indépendamment alkyle inférieur, cycloalkyle, phényle, naphtyle ou hétéroaryle, substitué avec 0 à 7 substituants choisis indépendamment dans le groupe consistant en alkyle inférieur, alcoxy inférieur, di(alkyle inférieur)amino, morpholino, phényle et tri(alkyle inférieur)silyle.

3. Procédé selon l'une quelconque des revendications 1 et 2 où le catalyseur est de formule
Ru(Z)₂D XVII
[Ru(Z)₂₋ₚ(D)(L¹)ₘ](B)ₚ XVIII
où Z représente un halogène ou le groupe A-COO, A représente alkyle inférieur, aryle² alkyle inférieur halogéné ou aryle² halogéné, B représente un anion non coordonnant comme défini ci-dessus et L¹ représente un ligand neutre comme défini ci-dessus, p représente les nombres 1 et 2, m représente les nombres 1, 2 ou 3, les ligands peuvent être identiques ou différents et D représente un ligand diphosphine chirale, où la diphosphine chirale est **caractérisée par** la formule (7), (9), (10) ou (12) comme défini dans la revendication 2.

4. Procédé selon la revendication 3 où Z est CH₃COO, CF₃COO ou un halogénure.

5. Procédé selon la revendication 3 où la diphosphine chirale est choisie dans le groupe consistant en les énantiomères (R) et (S) de MeOBIPHEP, BIPHEMP, TMBTP, 2-naphtyl)-MeOBIPHEP, (6-MeO-2-naphtyl)-MeOBIPHEP, 2-(thiényl)-MeOBIPHEP, 3,5-tBu-MeOBIPHEP, PHANEPHOS, BICP, TriMeOBIPHEP, (R,R,S,S)-Mandyphos, BnOBIPHEP, benzoylBIPHEP, pTol-BIPHEMP, tbutylCOOBIPHEP, iPrOBIPHEP, p-phényl-MeOBIPHEP, pAn-MeOBIPHEP, pTol-MeOBIPHEP, 3,5-Xyl-MeOBIPHEP, 3,5-Xyl-BIPHEMP, BINAP et 2-furyl-MeOBIPHEP, 3,5-Xyl-4-MeO-MeOBIPHEP, 2-furyl-MeOBIPHEP ou BITIANP.

6. Procédé selon la revendication 5 où la diphosphine chirale est (S)-(6-MeO-2-naphtyl)-MeOBIPHEP, 3,5-Xyl-4-MeO-MeOBIPHEP, (*S*)-2-furyl-MeOBIPHEP ou BITIANP.

7. Procédé selon l'une quelconque des revendications 1 à 6 où le catalyseur est choisi dans le groupe consistant en les énantiomères (R) et (S) de [Ru(CH₃COO⁻)₂(TMBTP)], [Ru(CF₃COO⁻)₂(TMBTP)], [Ru(CH₃COO⁻)₂(2-naphtyl)-MeOBIPHEP)], [Ru(CF₃COO⁻)₂(2-naphtyl)-MeOBIPHEP)], [Ru(CH₃COO⁻)₂(6-MeO-2-naphtyl)-MeOBIPHEP)] et [Ru(CF₃COO⁻)₂(6-MeO-2-naphtyl)-MeOBIPHEP)].

8. Procédé selon l'une quelconque des revendications 1 à 7 où l'hydrogénation catalytique est conduite à une pression de 1 à 150 bar.

9. Procédé selon la revendication 8 où l'hydrogénation catalytique est conduite à une pression de 10 à 100 bar.

10. Procédé selon l'une quelconque des revendications 1 à 9 où l'hydrogénation catalytique est conduite à une température de 10 à 100°C.

11. Procédé selon la revendication 10 où l'hydrogénation catalytique est conduite à une température de 20 à 80°C.

12. Procédé selon l'une quelconque des revendications 1 à 11 où l'hydrogénation catalytique est conduite en présence d'une base.

13. Procédé selon la revendication 12 où la base est choisie dans le groupe consistant en NEt₃, i-Pr₂NEt, i-Pr₂NH, C₆H₅CH₂NH₂, 1-phényl-benzylamine, (*R*) ou (*S*)), éthylènediamine, tétraméthyléthylènediamine, NaOAc, NaOEt, NaOH ou Bu₄NX, où X est F, Cl, Br ou I.

14. Procédé selon la revendication 13 où l'hydrogénation catalytique est conduite en présence de NEt₃ ou i-Pr₂NEt.

15. Procédé selon l'une quelconque des revendications 1 à 14 où l'hydrogénation catalytique est conduite dans un solvant.

16. Procédé selon la revendication 15 où l'hydrogénation catalytique avec un catalyseur à Ru est conduite avec des alcools, des hydrocarbures, des hydrocarbures chlorés, le THF ou l'eau, ou avec un mélange de ceux-ci.

17. Procédé selon la revendication 15 où l'hydrogénation catalytique avec un catalyseur à Rh est conduite avec des alcanols, le benzène, le toluène, le trifluorotoluène, le dichlorométhane, le dichloroéthane, l'éthylèneglycol, le DMF, DMA, la N-méthylpyrrolidinone, l'acétonitrile ou le DMSO ou avec un mélange de ceux-ci.

18. Procédé selon la revendication 16 où l'hydrogénation catalytique avec un catalyseur à Ru est conduite dans un solvant qui est le méthanol ou l'éthanol.

19. Procédé selon la revendication 15 où la concentration des solvants est 1-50 % en masse.

20. Procédé selon l'une quelconque des revendications 1 à 19 où le rapport substrat/catalyseur (s/C) est 5 : 30000.

21. Procédé selon l'une quelconque des revendications 1 à 20 où les composés de formule (I) sont
des acides 2-aryl/hétéroaryl-cyclopentanecarboxyliques,
des acides 4-aryl/hétéroaryl-2,5-dihydro-1H -pyrrolidine-3-carboxyliques,
des acides 4-aryl/hétéroaryl-tétrahydrofurane-3-carboxyliques,
des acides 4-aryl/hétéroaryl-tétrahydro-thiophène-3-carboxyliques,
des acides 1, 1-dioxo-4-aryl/hétéroaryl-tétrahydro-1λ⁶-thiophène-3-carboxyliques,
des acides 1-oxo-4-aryl/hétéroaryl-tétrahydro-1λ⁴-thiophène-3-carboxyliques,
un acide 2-aryl/hétéroaryl-cyclohexanecarboxylique,
des acides 4-aryl/hétéroaryl-pipéridine-3-carboxyliques,
des acides 5-aryl/hétéroaryl-pipéridine-4-carboxyliques,
des acides 4 -aryl/hétéroaryl-tétrahydro-pyrane-3-carboxyliques,
des acides 5-aryl/hétéroaryl-tétrahydro-pyrane-4-carboxyliques,
des acides 4-aryl/hétéroaryl-tétrahydro-thiopyrane-3-carboxyliques,
des acides 5-aryl/hétéroaryl-tétrahydro-thiopyrane-4-carboxyliques,
des acides 1,1-dioxo-4-aryl/hétéroaryl-hexahydro- 1λ⁶ -thiopyrane-3-carboxyliques,
des acides 1,1-dioxo-5-aryl/hétéroaryl-hexahydro-1λ⁶-thiopyrane-4-carboxyliques,
des acides 1-oxo-4-aryl/hétéroaryl-hexahydro-1λ⁴-thiopyrane-3-carboxyliques,
l'acide 2-phényl-cycloheptanecarboxylique,
l'acide 2-phényl-cyclooctanecarboxylique et leurs sels correspondants.

22. Composés de formule I selon la revendication 1, lesquels composés sont
le 1-*tert*-butylester d'acide (+)-(3*R*,4*R*)-4-(4-fluoro-phényl)-pipéridine-1,3-dicarboxylique et le 1-*tert*-butylester d'acide (-)-(3*S*,4*S*)-4-(4-fluoro-phényl)-pipéridine-1,3-dicarboxylique,
le 1-*tert*-butylester d'acide (-)-4-(1*H*-indol-3-yl)-pipéridine-1,3-dicarboxylique et
le 1-*tert*-butylester d'acide (+)-4-(1*H*-indol-3-yl)-pipéridine-1,3-dicarboxylique,
le 1-*tert*-butylester d'acide (-)-4-o-tolyl-pipéridine-1,3-dicarboxylique et le 1-*tert-*butylester d'acide (+)-4-o-tolyl-pipéridine-1,3-dicarboxylique,
le 1-*tert*-butylester d'acide (+)-4-(3-méthoxy-phényl)-pipéridine-1,3-dicarboxylique,
le 1-*tert*-butylester d'acide (+)-4-phényl-pipéridine-1,3-dicarboxylique et le 1-*tert-*butylester d'acide (-)-4-phényl-pipéridine-1,3-dicarboxylique,
le 1-*tert*-butylester d'acide (+)-3-phényl-pipéridine-1,4-dicarboxylique et le 1-*tert-*butylester d'acide (-)-3-phényl-pipéridine-1,4-dicarboxylique,
l'acide (-)-2-phényl-cyclopentanecarboxylique et
l'acide (+)-2-phényl-cyclopentanecarboxylique,
le 1-*tert*-butylester d'acide (+)-(3*R*,4*R*)-4-(phényl)-pyrrolidine-1,3-dicarboxylique et
le 1-*tert*-butylester d'acide (-)-(3*S*,4*S*)-4-(phényl)-pyrrolidine-1,3-dicarboxylique,
le 1-*tert*-butylester d'acide (-)-4-(4-chloro-phényl)-pyrrolidine-1,3-dicarboxylique et
le 1-*tert*-butylester d'acide (+)-4-(4-chloro-phényl)-pyrrolidine-1,3-dicarboxylique,
le 1-*tert*-butylester d'acide (+)-4-(3-fluoro-phényl)-pyrrolidine-1,3-dicarboxylique et
le 1-*tert*-butylester d'acide (-)-4-(3-fluoro-phényl)-pyrrolidine-1,3-dicarboxylique,
l'acide (3*R*,4*R*)-1-benzyl-4-phényl-pyrrolidine-3-carboxylique et
l'acide (3*RS*,4*RS*)-1-benzyl-4-phényl-pyrrolidine-3-carboxylique,
l'acide (-)-2-phényl-cyclooctanecarboxylique,
l'acide (+)-4-phényl-tétrahydro-thiophène-3-carboxylique et
l'acide (-)-4-phényl-tétrahydro-thiophène-3-carboxylique.

23. Procédé selon l'une quelconque des revendications 1 à 20 où les composés de départ de formule (II) sont 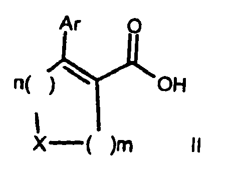 des acides 2-aryl/hétéroaryl-cyclopent-1-ènecarboxyliques,
des acides 4-aryl/hétéroaryl-2,5-dihydro-1H-pyrrole-3-carboxyliques,
des acides 4-aryl/hétéroaryl-2,5-dihydro-furane-3-carboxyliques,
des acides 4-aryl/hétéroaryl-2,5-dihydro-thiophène-3-carboxyliques,
des acides 1,1-dioxo-4-aryl-2,5-dihydro-1H-1λ⁶-thiophène-3-carboxyliques,
un acide 2-aryl/hétéroaryl-cyclohexyl-1-ènecarboxylique,
des acides 4-aryl/hétéroaryl-1,2,5,6-tétrahydro-pyridine-3-carboxyliques,
des acides 5-aryl/hétéroaryl-1,2,3,6-tétrahydro-pyridine-4-carboxyliques,
des acides 4-aryl/hétéroaryl-5,6-dihydro-2H-pyrane-3-carboxyliques,
des acides 5-aryl/hétéroaryl-3,6-dihydro-2H-pyrane-4-carboxyliques,
des acides 4-aryl/hétéroaryl-5,6-dihydro-2H-thiopyrane-3-carboxyliques,
des acides 5-aryl/hétéroaryl-3,6-dihydro-2H-thiopyrane-4-carboxyliques,
des acides 1,1-dioxo-4-aryl/hétéroaryl-1,2,5,6-tétrahydro-1λ⁶-thiopyrane-3-carboxyliques,
des acides 1,1-dioxo-5-aryl/hétéroaryl-1,2,3,6-tétrahydro-1λ⁶-thiopyrane-4-carboxyliques,
des acides 1-oxo-4-aryl/hétéroaryl-1,2,5,6-tétrahydro-1λ⁴-thiopyrane-3-carboxyliques,
des acides 1-oxo-4-aryl/hétéroaryl-2,5-dihydro-1H-1λ⁴-thiophène-3-carboxyliques, l'acide 2-phényl-cyclohept-1-ènecarboxylique,
l'acide 2-phényl-cyclooct-1-ènecarboxylique et leurs sels correspondants.
